(19) Europäisches Patentamt
European Patent Office
Office européen des brevets

(11) Numéro de publication: **0 524 041 B1**

(12) # FASCICULE DE BREVET EUROPEEN

(45) Date de publication du fascicule du brevet: **20.09.95**

(21) Numéro de dépôt: **92401763.5**

(22) Date de dépôt: **24.06.92**

(51) Int. Cl.⁶: **C07C 233/11**, C07C 233/15, C07C 235/28, C07C 327/44, A01N 37/18, A01N 37/34, A01N 57/30, A01N 37/22, A01N 43/42, A01N 49/00, A01N 55/00, C07C 323/41, C07C 323/63, C07C 309/88, C07D 213/30, C07D 333/16, C07C 231/02, C07C 231/12

(54) **Pesticides.**

(30) Priorité: **25.06.91 GB 9113624**

(43) Date de publication de la demande:
**20.01.93 Bulletin 93/03**

(45) Mention de la délivrance du brevet:
**20.09.95 Bulletin 95/38**

(84) Etats contractants désignés:
**AT BE CH DE DK ES FR GB GR IT LI LU NL PT SE**

(56) Documents cités:
**EP-A- 0 194 764**
**EP-A- 0 228 222**
**EP-A- 0 369 762**

**PATENT ABSTRACTS OF JAPAN vol. 6, no. 158 (C-120)19 Août 1982**

(73) Titulaire: **ROUSSEL UCLAF**
**102 Route de Noisy**
**F-93230 Romainville (FR)**

(72) Inventeur: **Robinson, John Edward**
**Ravens Lane**
**Berkhamsted,**
**Hertfodshire HP4 2DY (GB)**
Inventeur: **Cockerill, George Stuart**
**Ravens Lane**
**Berkhamsted,**
**Hertfodshire HP4 2DY (GB)**

(74) Mandataire: **Vieillefosse, Jean-Claude et al**
**ROUSSEL UCLAF**
**Département des Brevets**
**102, Route de Noisy**
**F-93235 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

PESTICIDE SCIENCE vol. 18, no. 4, 1987, BARKING GB pages 229 - 238 MICHAEL ELLIOT ET AL. 'Synthesis and Insecticidal Activity of Lipophilic Amides.'

JOURNAL OF PESTICIDE SCIENCE vol. 10, 1985, pages 11 - 17 MASAKAZU MIYAKADO ET AL. 'Chemistry and Insecticidal Activities of Piperaceae Amides and their Synthetic Analogues'

## Description

La présente invention concerne des composés pesticides, les procédés pour les préparer, les compositions les contenant et leur utilisation dans le traitement d'organismes nuisibles.

Une description a déjà été donnée d'amides insaturés insecticides ayant une chaîne méthylénique de 1 à au moins 10 atomes de carbone contenant facultativement un atome d'oxygène ou radical méthylène supplémentaire, ne portant pas de radical de terminaison (JP-A-57075961) ou portant différents radicaux de terminaison, notamment phényle facultativement substitué EP-A-228 222, EP-A-194 764 et EP-A-225 011, JP-A-57-212 150, Meisters et Wailes : Aust. J. Chem. 1966, 19, 1215, Vig et al. : J. Ind. Chem. Soc., 1974 51(9), 817, Miyakado et al : J. Pesticide Science, 1985, 10, 11 - 17], pyridyle (EP-A-269 457), bicycliques condensés EP-A-143 593 et EP-A-228 853), dihalovinyle ou éthynyle facultativement substitués EP-A-228 222). Elliot et al. : Pesticid. Sci. 1987, 18, 229 décrivent les relations entre structure et activité d'amides lipidiques du type ci-dessus et ont observé que lorsque l'extrémité N-alcoyle du radical amide est remplacée par une extrémité N-phényle, l'activité est détruite.

Le document EP-A-0 369 762 indique qu'un radical cyclopropyle interposé entre l'unité diénique et le radical de terminaison a un effet favorable sur les propriétés insecticides de ces amides insaturés.

Il a été découvert à présent avec surprise que lorsque le radical amide des composés ci-dessus est un radical anilide facultativement substitué, les composés obtenus ont de l'utilité comme pesticides et l'emportent, par l'amélioration de leurs propriétés, sur les composés dont le radical amide n'est pas un anilide.

La présente invention a donc pour objet un composé de formule (I) :

$$Q(CH_2)_a(O)_bQ^1CR^2=CR^3CR^4=CR^5CXNR^1R^X$$

ou un sel ou propesticide de celui-ci;
où

soit Q est un système cyclique aromatique monocyclique ou bicyclique condensé facultativement substitué dans lequel au moins un cycle est aromatique, soit Q est un radical dihalovinyle ou un radical $R^6$-C≡C-, où $R^6$ est $C_{1-4}$-alcoyle, tri-$C_{1-4}$-alcoylsilyle, halo ou hydrogène;

$Q^1$ est un cycle 1,2-cyclopropyle facultativement substitué par un ou plusieurs radicaux choisis parmi $C_{1-3}$-alcoyle, halo, $C_{1-3}$-haloalcoyle, $C_{2-3}$-alcynyle et cyano;

a est 0 ou 1;

b est 0 ou 1;

$R^2$, $R^3$, $R^4$ et $R^5$ sont identiques ou différents, au moins l'un étant hydrogène et les autres étant choisis indépendamment parmi hydrogène, halo, $C_{1-4}$-alcoyle et $C_{1-4}$-haloalcoyle;

X est oxygène ou soufre;

$R^1$ est phényle facultativement substitué par 1 à 5 substituants choisis parmi :

a) $C_{1-4}$-alcoyle, $C_{1-4}$-alcoxy, phénoxy et méthylènedioxy, chacun facultativement substitué par 1 à 5 halo;

b) halo, cyano, nitro, formyle, $C_{1-5}$-acyle;

c) $C_{2-3}$-alcynyle, $C_{2-3}$alcényle, chacun facultativement substitué par 1 à 5 halo;

d) un radical $S(O)_nR^7$, où n est 0, 1 ou 2 et $R^7$ est $C_{1-4}$-alcoyle, halo ou $NR^8R^9$, où $R^8$ et $R^9$ sont choisis indépendamment parmi hydrogène, $C_{1-4}$-alcoyle et $C_{1-4}$-acyle, et

e) un radical $NR^8R^9$, où $R^8$ et $R^9$ sont tels que définis ci-dessus:

$R^X$ est hydrogène ou un radical $C_{1-8}$-alcoyle ou benzyle facultativement substitué.

Un composé propesticide de formule (I) où $R^X$ est remplacé par $R^{XI}$, et $R^{XI}$ est choisi parmi :

(A)
$$\overset{|}{(D)_a\text{-}Y} = A$$

où

A est O ou S, Y est phosphore ou carbone, D est hydrogène, $C_{1-4}$-alcoyle, $C_{1-4}$-alcoxy, $C_{1-5}$-acyle ou aryle ou bien $CO_2D^1$, où $D^1$ est $C_{1-4}$-alcoyle ou aryle et a est 1 ou 2;

(B) $-S(O)_bD^2$

où

b est 0, 1 ou 2 et soit (i) $D^2$ est $C_{1-4}$-alcoyle, aryle, aryloxy ou $C_{1-4}$-alcoxy, dans lequel un cycle aryle peut être substitué par un ou plusieurs radicaux halo, nitro, cyano, $C_{1-4}$-alcoyle et $C_{1-4}$-alcoxy dont chacun à son tour est facultativement substitué par un ou plusieurs halogènes, soit (ii) $D^2$ est un radical $ND^3D^4$, où soit $D^3$ est $-COD^5$, où $D^5$ est hydrogène, fluor ou $C_{1-4}$-alcoyle, soit $D^3$ est $C_{1-4}$-alcoyle substitué par $C_{1-5}$-acyle ou aryle, carboalcoxy ou cyano, soit $D^3$ est un radical $CO_2D^6$, où $D^6$ est $C_{1-4}$-alcoyle ou aryle et $D^4$ est hydrogène ou $C_{1-4}$-alcoyle.

De préférence, $R^X$ est hydrogène ou $C_{1-4}$-alcoyle, comme méthyle ou isopropyle, ou benzyle ou un radical (B), où b est 0 et $D^2$ est phényle; ou un radical (A), où A est oxygène et D est hydrogène ou $C_{1-4}$-alcoxy.

Avantageusement, $R^1$ est phényle facultativement substitué par 1 à 3 radicaux choisis parmi alcoyle, $C_{1-4}$-alcoyle substitué par 1 à 5 halo, halo et $C_{1-4}$-alcoxy facultativement substitué par 1 à 5 halo.

Avantageusement, les substituants sur le cycle phényle de $R^1$ sont $C_{1-4}$-alcoyle, halo ou $C_{1-4}$-alcoyle substitué par 1 à 5 halo. De préférence, les substituants sont méthyle, éthyle, chloro, bromo, fluoro, trifluorométhyle, fluorométhyle ou difluorométhyle.

Lorsque Q est un système cyclique, des substituants appropriés sont notamment :

soit a) $C_{1-6}$-hydrocarbyle, $C_{1-6}$-alcoxy ou méthylènedioxy, chacun facultativement substitué par 1 à 5 halo;

soit b) halo, cyano, nitro, formyle ou $C_{1-5}$-acyle;

soit c) $C_{2-3}$-alcynyle ou $C_{2-3}$-alcényle, chacun facultativement substitué par 1 à 5 halo;

soit d) un radical $S(O)_nR^7$, où n est 0, 1 ou 2 et $R^7$ est $C_{1-4}$-alcoyle facultativement substitué par un ou plusieurs halo, halo ou $NR^8R^9$, où $R^8$ et $R^9$ sont choisis indépendamment entre hydrogène et $C_{1-4}$-alcoyle;

soit e) un radical $NR^8R^9$, où $R^8$ et $R^9$ sont choisis indépendamment parmi hydrogène, $C_{1-4}$-alcoyle et un radical $COR^{10}$, où $R^{10}$ est $C_{1-6}$-alcoyle ou $C_{1-6}$-alcoxy.

Avantageusement, lorsque Q est un système cyclique, il compte 5 à 10 atomes contenant un maximum de 3 hétéroatomes choisis parmi N, O et S et pour le reste des atomes de carbone facultativement substitués comme défini ci-dessus.

Avec avantage, Q est phényle, pyridyle, naphtyle ou un système cyclique bicyclique condensé contenant un maximum de 3 hétéroatomes choisis parmi N, O ou S, chacun facultativement substitué comme défini ci-dessus.

De préférence, Q est un cycle aromatique monocyclique ou un système cyclique bicyclique condensé dont au moins un cycle est aromatique et contenant 0 ou 1 atome d'azote et 0 ou 1 atome de soufre.

Lorsque Q est un cycle aromatique monocyclique, celui-ci est avantageusement phényle, pyridyle ou thiényle, mais de préférence phényle. Lorsque Q est un système cyclique bicyclique, celui-ci est de préférence naphtyle, quinoléinyle, tétrahydronaphtyle ou indanyle.

Lorsque Q contient un système aromatique, des substituants appropriés sont notamment :

soit a) $C_{1-6}$-hydrocarbyle, $C_{1-6}$-alcoxy ou méthylènedioxy, chacun facultativement substitué par 1 à 5 halo;

soit b) halo, cyano, nitro, formyle ou $C_{1-5}$-acyle;

soit c) $C_{2-3}$-alcynyle ou $C_{2-3}$-alcényle, chacun facultativement substitué par 1 à 5 halo;

soit d) un radical $S(O)_nR^7$, où n est 0, 1 ou 2 et $R^7$ est $C_{1-4}$-alcoyle facultativement substitué par un ou plusieurs halo, halo ou $NR^8R^9$, où $R^8$ et $R^9$ sont choisis indépendamment entre hydrogène et $C_{1-4}$-alcoyle;

soit e) un radical $NR^8R^9$ où $R^8$ et $R^9$ sont choisis indépendamment parmi hydrogène, $C_{1-4}$-alcoyle et un radical $COR^{10}$, où $R^{10}$ est $C_{1-6}$-alcoyle ou $C_{1-6}$-alcoxy.

Le système cyclique Q compte normalement jusqu'à trois substituants et est avantageusement non substitué ou substitué par un, deux ou trois substituants tels que halo ou $C_{1-5}$-haloalcoyle, comme trifluorométhyle.

La substitution du système cyclique Q dépend de la nature de ce système cyclique, mais est de préférence faite aux positions 3, 4 ou 5 lorsque Q est un cycle de six chaînons. Avantageusement, $R^2$, $R^3$, $R^4$ et $R^5$ sont choisis parmi hydrogène, méthyle et fluoro. Avec avantage, la stéréochimie des doubles liaisons est (E). Avantageusement, lorsque $R^3$ ou $R^5$ est fluoro, la stéréochimie de la double liaison à laquelle $R^3$ ou $R^5$ est uni est alors (Z).

De preférence, $R^2$ est hydrogène, $R^3$ est hydrogène ou fluoro, $R^5$ est hydrogène ou fluoro et $R^4$ est hydrogène ou $C_{1-4}$-alcoyle, plus avantageusement méthyle.

De préférence, b est 0 et a est 0.

De préférence, la configuration stéréochimique du radical cyclopropyle dans la chaîne est telle que les radicaux Q et la chaîne carbonée latérale se trouvent unis au cycle aux positions 2 et 1, respectivement, pour réaliser la géométrie trans. De préférence, la position 3 du cycle cyclopropyle est non substituée. Des substituants appropriés aux positions 1 et 2 du cycle cyclopropyle sont notamment hydrogène, fluoro, chloro, méthyle et trifluorométhyle. De préférence, la position 2 est non substituée et la position 1 est non substituée ou substituée par fluoro ou chloro.

De préférence, $R^1$ est phényle facultativement substitué par un ou plusieurs $C_{1-4}$-alcoyle, halo ou $C_{1-4}$-alcoyle substitué par un ou plusieurs halogènes.

Une classe appropriée de composés de formule (I) comprend ceux de formule (II) :

$$Q^aQ^{1a}CR^{2a}=CR^{3a}CR^{4a}=CR^{5a}C(=X^a)NHR^{1a} \quad (II)$$

ou un sel correspondant,
où

$Q^a$ est un radical phényle ou pyridyle facultativement substitué ou un système cyclique bicyclique condensé facultativement substitué dont au moins un cycle est aromatique et contenant 0 ou 1 atome d'azote ou 0 et 1 atome de soufre;

$Q^{1a}$ est un cycle cyclopropyle 1,2-disubstitué facultativement substitué par un ou plusieurs radicaux choisis parmi $C_{1-3}$-alcoyle, halo et $C_{1-3}$-haloalcoyle;

$R^{2a}$, $R^{3a}$, $R^{4a}$ et $R^{5a}$ sont identiques ou différents, au moins l'un étant un atome d'hydrogène et les autres étant choisis indépendamment parmi hydrogène, halo, $C_{1-4}$-alcoyle et $C_{1-4}$-haloalcoyle;

$X^a$ est oxygène ou soufre;

$R^{1a}$ est choisi parmi phényle facultativement substitué par halo et $C_{1-4}$-alcoyle facultativement substitué.

Des substituants appropriés pour $Q^a$ sont notamment un ou plusieurs radicaux choisis parmi halo, cyano, nitro, $C_{1-6}$-alcoyle, $C_{1-6}$-alcoxy et méthylènedioxy, chacun facultativement substitué par un ou plusieurs halo, ou bien le substituant est un radical $S(O)_nR^{7a}$, où n est 0, 1 ou 2 et $R^{7a}$ est $C_{1-6}$-alcoyle facultativement substitué par halo.

De préférence, $Q^a$ est phényle ou naphtyle ou pyridyle substitué.

Avantageusement, $R^{2a}$, $R^{3a}$, $R^{4a}$ et $R^{5a}$ sont choisis parmi hydrogène, méthyle et fluoro. De préférence, au maximum deux sont autres qu'hydrogène.

Avantageusement, $R^{1a}$ est choisi parmi phényle facultativement substitué par un ou plusieurs $C_{1-4}$-alcoyle tels que méthyle, $C_{1-4}$-alcoyle substitué par un ou plusieurs halogènes tels que trifluorométhyle, difluorométhyle ou fluorométhyle, ou halogène tel que chloro, fluoro ou bromo.

Des composés préférés de formule (II) sont notamment ceux de formule (III) :

$$Q^aQ^{1a}CH=CHCR^{4a}=CHCONHR^{1a} \quad (III)$$

où $Q^a$, $Q^{1a}$, $R^{4a}$ et $R^{1a}$ sont tels que définis ci-dessus.

Une classe préférée de composés de la présente invention comprend ceux de formule (IV) :

$$QQ^1CH=CR^3CR^4=CR^5CONHR^1 \quad (IV)$$

où Q, $Q^1$, $R^4$, $R^3$, $R^5$ et $R^1$ sont tels que définis ci-dessus.

Des composés préférés de formule (IV) sont notamment ceux où Q est phényle substitué, $Q^1$ est un radical cyclopropyle 1,2-disubstitué en trans, où la position 1 du radical cyclopropyle est non substituée ou substituée par fluoro ou chloro, $R^4$ est méthyle ou hydrogène, $R^3$ et $R^5$ sont hydrogène ou fluoro et $R^1$ est phényle facultativement substitué par un ou plusieurs $C_{1-4}$-alcoyle et difluorométhyle, par exemple méthyle, trifluorométhyle, difluorométhyle, fluorométhyle, chloro, fluoro, bromo.

Par le terme "halo", on entend fluoro, chloro, bromo et iodo.

Par le terme "radical hydrocarbyle" on entend des radicaux alcoyle, alcényle, alcynyle, aralcoyle incluant un radical alcoyle ou alcényle cyclique, facultativement substitué par alcoyle, alcényle ou alcynyle; et alcoyle et alcényle substitué par alcoyle et alcényle cyclique, et phényle.

Le terme "acyle" tel qu'il est utilisé ici a la signification COR, où R est $C_{1-5}$-alcoyle.

Les sels des composés de la présente invention sont normalement des sels d'addition d'acides. Ces sels peuvent être formés à partir d'acides minéraux, organiques ou cycloalcoyliques. Les sels préférés sont notamment ceux formés par l'acide chlorhydrique, l'acide bromhydrique, l'acide sulfurique, l'acide citrique, l'acide nitrique, l'acide tartrique, l'acide phosphorique, l'acide lactique, l'acide benzoïque, l'acide glutamique, l'acide aspartique, l'acide pyruvique, l'acide acétique, l'acide succinique, l'acide fumarique, l'acide maléique, l'acide oxalacétique, l'acide hydroxynaphtoïque, l'acide iséthionique, l'acide stéarique, l'acide méthanesulfonique, l'acide éthanesulfonique, l'acide benzènesulfonique, l'acide p-toluène sulfonique, l'acide lactobionique, l'acide glucuronique, l'acide thiocyannique, l'acide propionique, l'acide embonique, l'acide naphténoïque et l'acide perchlorique.

Les composés de formule (I) peuvent exister sous un certain nombre de formes stéréoisomères. La présente invention a pour objet les isomères géométriques et stéréoisomères individuels et leurs mélanges. L'invention a aussi pour objet les composés de formule (I) contenant des radioisotopes, en particulier ceux dans lesquels 1 à 3 atomes d'hydrogène sont remplacés par du tritium ou un ou plusieurs atomes de carbone sont remplacés par $^{14}C$.

Suivant un autre aspect, la présente invention a pour objet un procédé de préparation d'un composé de formule (I) tel que défini ci-dessus qui comprend (schéma 1) :

a) lorsque X est oxygène, la réaction de l'acide ou dérivé d'acide correspondant :

$$Q(CH_2)_a(O)_bQ^1CR^2=CR^3CR^4=CR^5C(=X)Z^1$$

avec une amine $H_2NR^1$, où Q, a, b, $Q^1$, $R^2$, $R^3$, $R^4$, $R^5$ et $R^1$ sont tels que définis ci-dessus, X est oxygène et $Z^1$ est hydroxyle, $C_{1-6}$-alcoxy, halo ou un ester phosphoroimidique [-P(O)(O-aryl) NH-aryle, où aryle est $C_{6-10}$-aryle];

b) la formation de la partie :

$$CR^2=CR^3CR^4=CR^5C(=X^1)NHR^1$$

par une réaction de type Wittig;

et facultativement, ensuite, la conversion d'un composé de formule (I) en un autre composé de formule (I) suivant des procédés connus de l'homme de métier.

Le procédé a est normalement exécuté à une température non extrême, par exemple entre -25 et 150 °C, dans un solvant aprotique anhydre, comme l'éther, le dichlorométhane, le toluène et le benzène. Les conditions précises dépendent de la nature du radical $Z^1$, par exemple lorsque $Z^1$ est alcoxy, la réaction est exécutée avec avantage à une température élevée, à savoir de 50 à 125 °C, et avantageusement au reflux, de préférence en présence d'un trialcoylaluminium, comme le triméthylaluminium, qui forme un complexe avec l'amine $H_2NR^1$. Lorsque $Z^1$ est halo ou phosphoroimidate, la réaction est exécutée avec avantage de -20 °C à 30 °C, et de préférence en présence d'une amine tertiaire, comme la triéthylamine ou la pyridine.

Si le dérivé d'acide est un halogénure d'acide, par exemple le chlorure d'acide, il peut être formé à partir de l'acide correspondant par réaction avec un réactant approprié, comme le chlorure d'oxalyle ou le chlorure de thionyle. Lorsque $Z^1$ est un radical phosphoroimidate, celui-ci est avantageusement formé à partir de (PhO)P(→O)NHPhCl, où Ph est phényle. L'acide ou la fonction acide dans le composé :

$$Q(CH_2)_a(O)_bQ^1CR^2=CR^3CR^4=CR^5COZ^1$$

peut s'obtenir par hydrolyse de l'ester correspondant.

Les esters peuvent être préparés suivant un certain nombre de voies constituant des variantes, par exemple (schéma 2) :

(i) une réaction de Wittig ou de Wadsworth-Emmons classique, par exemple à l'aide d'un aldéhyde et d'éthoxycarbonylméthylène triphénylphosphorane ou d'un anion de phosphonocrotonate de triéthyle ou de 3-méthylphosphonocrotonate de triéthyle. Cette dernière réaction peut conduire à un mélange d'isomères, par exemple un mélange de diénoates (Z) et (E) substitués; un tel mélange peut être mis à réagir comme ci-dessus, après quoi le mélange résultant des amides peut être séparé par chromatographie ou d'autres techniques appropriées. Le réactant de type Wittig peut être obtenu, par exemple, par la voie suivante ou une variante de celle-ci :

$$(CH_3)_2C=CHCO_2Et \xrightarrow[(2)]{(1)} Z^2CH_2C(CH_3)=CHCO_2Et \xrightarrow{(3)} W/W\text{-}E$$

W/W-E = réactant de Wittig/Wadsworth-Emmons;

(1) N-bromosuccinimide;

(2) par exemple $(EtO)_3P$ ou $(Ph)_3P$;

(3) une base telle que le diisopropylamidure de lithium, le butyllithium, le carbonate de potassium, un alcoolate de sodium ou l'hydrure de sodium;

où $Z^2$ = $(aryl)_3P$, $(aryl)_2P(O)$ ou $(C_{1-4}$-alcoxy$)_2P(O)$ où aryle est de préférence phényle et alcoxy est de préférence éthoxy;

(ii) par transposition et élimination de $HS(\rightarrow O)Z^3$ d'un composé de formule :

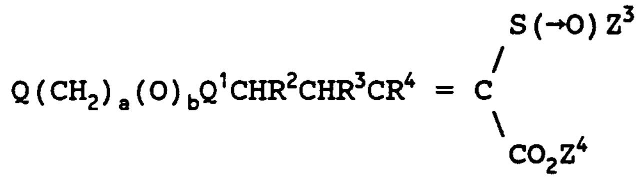

où Q, $Q^1$, $R^2$, $R^3$, a, b et $R^4$ sont tels que définis ci-dessus, $Z^3$ est tout radical approprié, par exemple phényle, phényle substitué tel que 4-chlorophényle, ou $C_{1-4}$-alcoyle, par exemple méthyle, $Z^4$ est $C_{1-4}$-alcoyle, par exemple méthyle ou éthyle.

Le composé ci-dessus peut être obtenu par la réaction d'un composé :

$Q(CH_2)_a(O)_bQ^1CHR^2CHR^3CR^4O$

avec un composé $Z^3S(O)CH_2CO_2Z^4$;

(iii) par élimination d'un composé :

$Q(CH_2)_a(O)_bQ^1CHR^2CR^3(OZ^5)CR^4=CR^5CO_2Z^4$

où Q, a, b, $Q^1$, $R^2$, $R^3$, $R^4$, $R^5$ et $Z^4$ sont tels que définis ci-dessus et $Z^5$ est hydrogène ou $C_{1-4}$-acyle tel qu'acétyle. Lorsque $Z^5$ est acétyle, la réaction est exécutée de préférence dans un solvant aromatique, avec avantage en présence d'un catalyseur au molybdène et d'une base, comme le bis-triméthylsilylacétamide.

Le composé ci-dessus peut être obtenu ci-dessus par réaction d'un aldéhyde approprié avec un composé sulfénylé approprié, puis par acylation.

(iv) par réaction d'un composé de formule :

$Q(CH_2)_a(O)_bQ^1CR^2=CR^3C(=O)R^4$

avec un composé de formule :

$Me_3SiCHR^5CO_2Z^4$

où Q, a, b, $R^2$ à $R^5$, $Q^1$ et $Z^4$ sont tels que définis ci-dessus.

Ce procédé peut être exécuté dans un solvant anhydre, par exemple le tétrahydrofuranne en l'absence d'oxygène, et en présence d'une base, par exemple le cyclohexylisopropylamidure de lithium.

(v) par réaction d'un composé de formule :

$Q(CH_2)_a(O)_bQ^1CR^2=CR^3C(OZ^6)=CR^5CO_2Z^4$

avec un composé de formule $R^4M^1$, où Q, a, b, $Q^1$, $R^2$, $R^3$, $R^4$, $R^5$ et $Z^4$ sont tels que définis ci-dessus, $Z^6$ est un radical approprié tel que phosphate de dialcoyle ou trifluorométhanesulfonate et $M^1$ est un métal tel que le cuivre (I) ou le cuivre (I) associé au lithium ou au magnésium.

Ce procédé peut être exécuté à basse température dans un solvant éthéré anhydre tel que l'éther diéthylique, le sulfure de diméthyle ou le tétrahydrofuranne en l'absence d'oxygène.

(vi) par réaction d'un composé de formule :

$Q(CH_2)_a(O)_bQ^1CR^2=CR^3M^2$

avec un composé de formule :

$Y\text{-}CR^4 = CR^5CO_2Z^4$

où Q, a, b, $Q^1$, $R^2$, $R^3$, $R^4$, $R^5$ et $Z^4$ sont tels que définis ci-dessus,

Y est halo ou étain et $M^2$ est un radical silyle ou contenant un métal, comme triméthylsilyle ou un radical contenant du zirconium, de l'étain, de l'aluminium ou du zinc, par exemple un radical chlorure de bis-(cyclopentadiényl)zirconium. Ce procédé est normalement exécuté à une température non extrême, à savoir entre 0 et 100°C, et avantageusement à la température ambiante dans un solvant éthéré non aqueux tel que le tétrahydrofuranne, en présence d'un catalyseur au palladium (0) [comme le bis-(triphénylphosphine)palladium] et sous atmosphère inerte d'azote ou d'argon.

(vii) par élimination de $Z^3S(\rightarrow O)H$ d'un composé de formule :

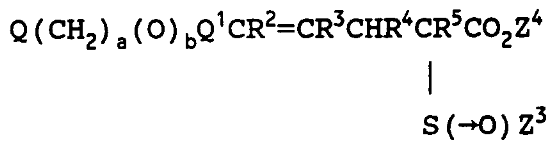

où Q, a, b, $Q^1$, $R^2$, $R^3$, $R^4$, $R^5$, $Z^3$ et $Z^4$ sont tels que définis ci-dessus.

Le composé ci-dessus peut être obtenu par réaction d'un composé :

$QQ^1CHR^2CR^3 = CHR^4$

avec

$Z^3S(O)CHR^5CO_2Z^4$

Le procédé (b) peut être exécuté en ayant un radical aldéhyde ou cétone uni soit à l'extrémité amide/thioamide, soit au fragment $QQ^1$ de formule (I), puis en faisant réagir celui-ci avec l'ylure de phosphore approprié, à savoir

$Q(CH_2)_a(O)_bQ^1(CR^2 = CR^3)COR^4 + Z^2CHR^5.C(=X)NHR^1$ ou
$Q(CH_2)_a(O)_bQ^1COR^2 + Z^2CHR^3.CR^4 = CR^5.C(=X)NHR^1$ ou
$Q(CH_2)_a(O)_bQ^1(CR^2 = CR^3)CHR^5Z^2 + R^5CO.C(=X)NH.R^1$

où Q, a, b, $Q^1$, $R^2$, $R^3$, $R^4$, $R^5$, $R^1$, X et $Z^2$ sont tels que définis ci-dessus.

Le <u>procédé (b)</u> est exécuté dans un solvant inerte anhydre, par exemple un éther tel que le tétrahydrofuranne ou un alcool tel que le méthanol, facultativement en présence d'une base pour la préparation de l'ylure de phosphore, de préférence en l'absence d'oxygène, par exemple sous atmosphère d'azote, à basse température (-60 à 20°C). L'ylure de phosphore peut être obtenu à partir de son précurseur comme décrit ci-dessus par réaction avec une base telle que le diisopropylamidure de lithium, le butyllithium, un alcoolate de sodium, le carbonate de sodium ou de potassium ou l'hydrure de sodium. Les composés de formule (I) où X est le soufre sont de préférence préparés par le procédé (b) si $Z^2$ est un radical $(C_{1-4}\text{-alcoxy})_2P = O$.

Les précurseurs du type $Z^2CHR^3CR^4 = CR^5(C = X^1)NHR$ sont préparés par la voie suivante ou une variante de celle-ci lorsque $X^1$ est oxygène :

$$CH_3CR^4=CR^5CO_2H \xrightarrow[2)]{1)} BrCH_2CR^4=CR^5COCl$$

$$\xrightarrow{3)} BrCH_2CR^4=CR^5CONHR^1 \xrightarrow{4)} Z^2CH_2CR^4=CR^5CONHR^1$$

1) N-bromosuccinimide,
2) chlorure de thionyle,
3) $NH_2R^1$,
4) $(EtO)_3P$ si $Z^2$ = $-(OEt)_2P(O)-$,

lorsque $X^1$ est soufre, les précurseurs du type $Z^2CHR^5(C=S)NHR^1$ sont préparés par réaction de l'anion dérivant de $Z^2CH_2R^5$ avec $R^1NCS$,

lorsque $X^1$ est oxygène, les précurseurs du type $Z^2CHR^5(C=O)NHR^1$ sont préparés par réaction de $Ph_3P$ ou $P(OEt)_3$ avec $ClCHR^5(CO)NHR^1$.

Les aldéhydes intermédiaires $Q(CH_2)_a(O)_bQ^1CR^2=O$ peuvent être préparés par hydrolyse acide d'un précurseur protégé approprié comme un cétal, un éther énolique ou un acétal, dans un solvant tel que l'acétone-eau, ou par oxydation des alcools appropriés au moyen, par exemple, de chlorochromate de pyridinium, de dichromate de pyridinium, de chlorure d'oxalyle-diméthylsulfoxyde ou de N-acétylamido-2,2,6,6-tétraméthylpipéridine-N-oxyde et d'acide p-toluènesulfonique dans un solvant tel que le dichlorométhane. Les aldéhydes peuvent être préparés aussi par réduction des nitriles appropriés avec un réactant tel que l'hydrure de diisobutylaluminium dans l'hexane.

Lorsque Q est un cycle cyclopropyle 1,2-disubstitué, les alcools (schéma 3) peuvent être préparés par :

a) réaction de $Q(CH_2)_a(O)_bCH=CX^2OH$ avec $(Z^7)_2M^2$ et $CH_2X^3_2$ , où $X^2$ est un radical tel qu'hydrogène, fluoro, chloro ou méthyle, $X^3$ est un halogène tel qu'iode, $Z^7$ est un radical $C_{1-4}$-alcoyle tel qu'éthyle et $M^2$ est un métal tel que le zinc, dans un solvant inerte, comme l'hexane ou le dichlorométhane, à une température modérée (-20 °C à 20 °C). Ainsi, $CH_2$ et $CH=CX^2$ se combinent pour former $Q^1$,

b) réaction de $Q(CH_2)_a(O)_bCH=CX^2CH_2OH$ avec $CX^4_2X^5CO_2M^3$, où $X^4$ et $X^5$ sont des halogènes tels que fluor et chlore et $M^3$ est un métal alcalin tel que le sodium, dans un solvant inerte, comme le diglyme, à une température modérée-élevée (150 °C à 200 °C). Ainsi, $CX^4_2$ et $CH=CX^2$ se combinent pour former $Q^1$.

Les alcools intermédiaires peuvent être préparés par réduction de l'ester $Q(CH_2)_a(O)_bCH=CX^2CO_2Z^4$ avec, par exemple, l'hydrure de diisobutylaluminium dans un solvant inerte, comme le dichlorométhane ou le tétrahydrofuranne, à une température modérée (-20 °C à 25 °C).

c) réduction d'un ester $Q(CH_2)_a(O)_bQ^1CO_2Z^4$ ou de l'acide carboxylique approprié avec, par exemple, l'hydrure de diisobutylaluminium, l'hydrure de lithium-aluminium, le borohydrure de sodium ou le diborane dans un solvant inerte, comme le dichlorométhane, l'éthanol ou le tétrahydrofuranne, à une température modérée (-20 °C à 25 °C). Les esters peuvent être préparés par réaction d'un diazoacétate $N_2CH.CO_2Z^4$ avec un composé $Q(CH_2)_a(O)_bCH=CH_2$ en présence d'un catalyseur contenant du cuivre, comme le sulfate de cuivre, auquel cas CH et $CH=CH_2$ se combinent pour former $Q^1$. Les esters peuvent être préparés aussi par réaction de $Q(CH_2)_a(O)_bCH=CHCO_2Z^4$ avec un anion issu de $Me_2S(O)_mC(Z^7)_2$, où $Z^7$ est hydrogène ou $C_{1-6}$-alcoyle et m est 1 ou 2.

Les schémas de réaction ci-après aident à illustrer la préparation des intermédiaires et leur conversion en les composés de formule (I).

Les intermédiaires de la présente invention constituent un autre aspect de celle-ci et peuvent être préparés, lorsqu'il y a lieu, suivant des procédés connus autres que ceux décrits.

Les composés de formule (I) peuvent être utilisés pour lutter contre des organismes nuisibles tels que les arthropodes, par exemple les insectes et acariens, et les helminthes, par exemple les nématodes, ou les mollusques, par exemple les limaces. La présente invention a donc pour objet un procédé pour la lutte contre les arthropodes et/ou helminthes et/ou mollusques, qui comprend l'administration aux arthropodes et/ou helminthes et/ou mollusques ou à leur environnement d'une quantité d'un composé de formule (I) suffisante pour détruire l'organisme nuisible. La présente invention a aussi pour objet un procédé pour lutter contre et/ou éradiquer les infestations par les arthropodes et/ou helminthes et/ou mollusques chez les

animaux (y compris l'être humain) et/ou les plantes (y compris les arbres) et/ou les produits stockés, qui comprend l'administration à l'animal ou au site d'une quantité efficace d'un composé de formule (I). L'invention a aussi pour objet les composés de formule (I) à utiliser en médecine humaine et vétérinaire, en santé publique et/ou en agriculture pour la lutte contre les arthropodes et/ou helminthes nuisibles.

Les composés de formule (I) sont d'une valeur particulière pour la protection des récoltes sur pied, du fourrage, des plantations, des cultures en serres, des vergers et des vignobles, des plantes ornementales et des arbres en plantation et en forêt, par exemple des céréales (comme le maïs, le froment, le riz et le sorgho), du coton, du tabac, des légumes et salades (comme les haricots, les choux, les curcubitacées, les laitues, les oignons, les tomates et les poivrons), des cultures vivrières (par exemple la pomme de terre, la betterave sucrière, l'arachide, le soya et le colza), de la canne à sucre, les prairies et cultures de fourrage (comme le maïs, le sorgho et la luzerne), des plantations (comme celles produisant le thé, le café, le cacao, la banane, l'huile de palme, la noix de coco, le caoutchouc et les épices), des vergers et bois plantés (comme ceux produisant les fruits à noyaux et pépins, les agrumes, les kiwis, les avocats, les mangues, les olives et les noix), des vignobles, des plantes ornementales, des fleurs et buissons en serre et dans les jardins et parcs, des arbres forestiers (à feuillage tant caduc que persistant) dans les forêts, plantations et pépinières.

Ils sont précieux aussi pour la protection du bois d'oeuvre (sur pied, abattu, transformé, stocké ou dans les bâtiments) contre l'attaque par les sirex (par exemple Urocerus), les coléoptères (par exemple les scolytidés, les platypodidés, les lyctidés, la bostrychidés, les cérambucidés et les anobiidés) et les termites.

Ils trouvent leur application dans la protection des produits stockés, comme les grains, les fruits, les noix, les épices et le tabac, à l'état entier, moulu ou transformé en produits, contre l'attaque par les mites, coléoptères et charançons. Ils protègent également les produits animaux stockés, comme les peaux, les poils, la laine et les plumes, sous forme naturelle ou convertie (par exemple en tapis ou matières textiles), contre l'attaque par les mites et coléoptères, de même que la viande et le poisson contre l'attaque par les coléoptères et les mouches.

Les composés de formule générale (I) sont d'une utilité particulière pour la lutte contre les arthropodes, les helminthes ou les mollusques qui sont nuisibles pour l'homme et les animaux domestiques ou sont les propagateurs ou vecteurs de maladies frappant ces derniers, par exemple celles décrites ci-dessus, plus spécialement dans le domaine de la lutte contre les tiques, la gale, les poux, les puces, les moucherons et les mouches causes des morsures, de nuisance et de myase.

Les composés de formule (I) peuvent être utilisés à de telles fins par application des composés tels quels ou sous forme diluée de façon connue à l'état de bain, de spray, de brouillard, de vernis, de mousse, de poudre, de poudre à poudrer, de suspension aqueuse, de pâte, de gel, de shampooing, d'onguent, de solide combustible, de mat de vaporisation, de spirale combustible, d'appât, de supplément alimentaire, de poudre mouillable, de granules, d'aérosol, de concentrés émulsionnables, de suspensions huileuses, de solutions huileuses, de bombe sous pression, d'article imprégné, de lotion ou d'autres compositions standard bien connues de l'homme de métier. Les concentrés pour bains ne sont pas appliqués tels quels, mais dilués avec de l'eau et les animaux sont immergés dans une cuve contenant le bain. Les sprays peuvent être appliqués à la main ou à l'aide d'une lance ou d'un arceau de pulvérisation. L'animal, la terre, la plante ou la surface peut être saturé du spray par application en grand volume ou être revêtu superficiellement du spray par application en petit ou ultrapetit volume. Les suspensions aqueuses peuvent être appliquées sur l'animal de la même façon que les sprays et les bains. Les poudres à poudrer peuvent être distribuées au moyen d'un applicateur de poudre ou, dans le cas des animaux, être incorporées à des sacs perforés attachés à des arbres ou à des poteaux. Les pâtes, shampooings et onguents peuvent être appliqués à la main ou répartis sur la surface d'une matière inerte contre laquelle les animaux se frottent et transfèrent ainsi la matière à leur peau. Les lotions sont distribuées sous la forme de dose de liquide de petit volume sur le dos des animaux, de façon que la totalité ou la majeure partie du liquide reste sur les animaux.

Les composés de formule (I) peuvent être présentés à l'état de compositions prêtes à l'usage sur les plantes, les animaux et les surfaces ou à l'état de compositions qui doivent être diluées avant l'application, mais les compositions des deux types comprennent un composé de formule (I) en mélange intime avec un ou plusieurs excipients ou diluants. Les excipients peuvent être liquides, solides ou gazeux ou peuvent comprendre des mélanges de telles substances et le composé de formule (I) peut être présent en une concentration de 99 à 0,025% p/v, suivant le fait que la composition doit ou non être davantage diluée.

Les poudres à poudrer, poudres et granules comprennent le composé de formule (I) en mélange intime avec un excipient inerte solide pulvérulent, par exemple des argiles appropriées, le kaolin, la bentonite, l'attapulgite, le noir de carbone adsorbant, le talc, le mica, la craie, le gypse, le phosphate tricalcique, le liège en poudre, le silicate de magnésium, les excipients végétaux, l'amidon et les terres de diatomées.

Ces compositions solides sont généralement préparées en imprégnant les diluants solides avec des solutions du composé de formule (I) dans des solvants volatils, en évaporant les solvants et, si la chose est souhaitée, en broyant les produits pour obtenir des poudres et, si la chose est souhaitée, en granulant, en pressant ou en encapsulant les produits.

Les sprays d'un composé de formule (I) peuvent comprendre une solution dans un solvant organique (par exemple ceux énumérés ci-dessous) ou une émulsion dans de l'eau (bain ou douche) préparée sur le terrain à partir d'un concentré émulsionnable (appelé aussi huile miscible à l'eau), qui peut servir aussi pour l'immersion. Le concentré comprend de préférence un mélange du constituant actif, avec ou sans solvant organique et un ou plusieurs émulsionnants. Les solvants peuvent être présents entre de larges limites, mais de préférence en une quantité de 0 à 90% p/v de la composition et peuvent être choisis parmi le kérosène, les cétones, les alcools, le xylène, le naphta aromatique et d'autres solvants connus pour la mise en composition. La concentration des émulsionnants peut varier entre de larges limites, mais se situe de préférence dans l'intervalle de 5 à 25% p/v et les émulsionnants sont avantageusement des tensioactifs non ioniques, notamment des esters polyoxyalcoyléniques d'alcoylphénols et dérivés polyoxyéthyléniques d'anhydrides d'hexitols, ou bien des tensioactifs anioniques, notamment le laurylsulfate de sodium, des sulfates d'éthers d'alcools gras, des sels de sodium et calcium d'alcoylarylsulfonates et d'alcoylsulfosuccinates. Des émulsionnants cationiques sont notamment le chlorure de benzalkonium et les éthosulfates d'ammonium quaternaire.

Des émulsionnants amphotères sont notamment l'imidazoline oléique carboxyméthylée et les alcoyldiméthylbétaïnes.

Les mats de vaporisation comprennent normalement un mélange de coton et de cellulose pressé en une plaque d'environ 32 mm sur 22 mm sur 3 mm traitée au moyen d'une quantité atteignant 0,3 ml d'un concentré qui contient le constituant actif dans un solvant organique et facultativement un antioxydant, un colorant et un parfum. L'insecticide est vaporisé par une source de chaleur, comme un appareil de chauffage électrique pour mats.

Les solides combustibles comprennent normalement de la sciure de bois et un liant en mélange avec le constituant actif et à l'état façonné en rubans (habituellement en hélice). Un colorant et un fongicide peuvent être ajoutés aussi.

Les poudres mouillables comprennent un excipient solide inerte, un ou plusieurs tensioactifs et facultativement des stabilisants et/ou antioxydants.

Les concentrés émulsionnables comprennent des émulsionnants et souvent un solvant organique tel que kérosène, des cétones, des alcools, des xylènes, du naphta aromatique et d'autres solvants connus.

Les poudres mouillables et concentrés émulsionnables contiennent normalement 5 à 95% en poids du constituant actif et sont dilués avant usage, par exemple avec de l'eau.

Les vernis comprennent une solution du constituant actif dans un solvant organique, conjointement avec une résine et facultativement un plastifiant.

Les bains peuvent être préparés non seulement à partir de concentrés émulsionnables, mais aussi à partir de poudres mouillables, de bains à base de savon et de suspensions aqueuses comprenant un composé de formule (I) en mélange intime avec un dispersant et un ou plusieurs tensioactifs.

Les suspensions aqueuses d'un composé de formule (I) peuvent comprendre une suspension dans de l'eau conjointement avec un agent de mise en suspension, de stabilisation ou autre. Les suspensions ou solutions peuvent être appliquées telles quelles ou sous une forme diluée de façon connue.

Les onguents (ou graisses) peuvent être préparés à partir d'huiles végétales, d'esters synthétiques d'acides gras ou de lanoline, conjointement avec un base inerte telle que la paraffine molle. Un composé de formule (I) est de préférence réparti uniformément dans le mélange en solution ou suspension. Les onguents peuvent aussi être obtenus à partir de concentrés émulsionnables par dilution de ces derniers dans une base d'onguent.

Les pâtes et shampooings sont aussi des compositions semi-solides dans lesquelles un composé de formule (I) peut être présent à l'état de dispersion uniforme dans une base appropriée, comme de la paraffine liquide ou molle, ou bien dans une base non grasse avec du glycérol, de la colle ou un savon convenable. Du fait que les onguents, shampooings et pâtes sont habituellement appliqués sans autre dilution, ils doivent contenir le pourcentage approprié du composé de formule (I) que requiert le traitement.

Les sprays en aérosol peuvent être préparés à l'état de simple solution du constituant actif dans le propulseur d'aérosol et un cosolvant tel qu'un alcane halogéné et les solvants mentionnés ci-dessus, respectivement. Les compositions en lotions peuvent être présentées à l'état de solution ou suspension d'un composé de formule (I) dans un milieu liquide. Un oiseau ou mammifère hôte peut aussi être protégé contre l'infestation par les acariens ectoparasites en portant un produit manufacturé façonné en matière plastique convenablement moulée qui est imprégné d'un composé de formule (I). Ces produits manufactu-

rés comprennent les colliers, marques d'oreilles, bandes, toiles et rubans convenablement attachés sur la partie appropriée du corps. La matière plastique est avantageusement un poly(chlorure de vinyle).

Les composés de formule (I) sont à utiliser dans la protection et le traitement des espèces végétales, auquel cas une quantité insecticide, acaricide, molluscide nématocide efficace du constituant actif est appliquée. La dose d'application varie avec le composé choisi, la nature de la composition, le mode d'application, l'espèce végétale, la densité de plantation, l'infestation probable et différents autres facteurs, mais en général une dose convenant pour l'agriculture se situe dans l'intervalle de 0,001 à 3 kg par hectare et de préférence entre 0,01 et 1 kg par hectare. Les compositions typiques à usage agricole contiennent entre 0,0001% et 50% d'un composé de formule (I) et avantageusement entre 0,1 et 15% en poids d'un composé de formule (I).

La concentration du composé de formule (I) pour une application sur un animal, des locaux ou des endroits extérieurs varie avec le composé choisi, l'intervalle entre les traitements, la nature de la composition et l'infestation probable, mais en général le composé doit être contenu dans la composition appliquée en quantité de 0,001 à 20,0% p/v, de préférence 0,01 à 10% p/v. La quantité du composé déposé sur un animal varie avec le mode d'application, la taille de l'animal, la concentration du composé dans la composition appliquée, le facteur de dilution de la composition et la nature de la composition, mais se situe généralement dans l'intervalle de 0,0001% à 0,5% p/p, sauf pour les compositions non diluées, comme les compositions en lotion qui sont généralement déposées à une concentration de l'intervalle de 0,1 à 20,0% et de préférence de 0,1 à 10%. La quantité du composé à appliquer sur les produits stockés se situe généralement dans l'intervalle de 0,1 à 20 ppm. Les pulvérisations dans les espaces peuvent être réalisées pour arriver à une concentration initiale moyenne de 0,001 à 1 mg du composé de formule (I) par $m^3$ d'espace traité.

Les onguents, les graisses, les pâtes et les aérosols sont habituellement appliqués au hasard comme décrit ci-dessus et des concentrations de 0,001 à 20% p/v d'un composé de formule (I) dans la composition appliquée peuvent être utilisées.

Les composés de formule (I) se sont révélés avoir de l'activité contre la mouche ordinaire (Musca domestica). De surcroît, certains composés de formule (I) ont de l'activité contre d'autres arthropodes nuisibles, notamment Myzus persicae, Tetranychus urticae, Spodoptera littoralis, Heliotuis virescens, Plutella xylostella, Culex spp., Tribolium castaneum, Sitophilus granarius, Periplaneta americana et Blattella germanica. Les composés de formule (I) sont donc utiles pour la lutte contre les arthropodes, par exemple les insectes et acariens, dans tout milieu où ils constituent une nuisance, par exemple en agriculture, en élevage, en santé publique et en milieu domestique.

Des insectes nuisibles sont notamment des membres des ordres de coléoptères (par exemple Anobium, Ceuthorrhynchus, Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus ou Anthrenus spp.), des lépidoptères (par exemple Ephestia, Mamestra, Earias, Pectinophora, Ostrinia, Trichloplusia, Pieris, Laphygma, Agrotis, Amathes, Wiseana, Tryporyza, Diatraea, Sparganothis, Cydia, Archips, Plutella, Chilo, Heliothis, Spodoptera littoralis, Helrotuis virescens, Spodoptera ou Tineola spp.), des diptères (par exemple Musca, Aedes, Anopheles, Culex, Glossina, Simulium, Stomoxys, Haematobia, Tabanus, Hydrotaea, Lucilia, Chrysomyia, Callitroga, Dermatobia, Gasterophilus, Hypoderma, Hylemyia, Atherigona, Chlorops, Phytomyza, Ceratitis, Liriomyza et Melophagus spp.), des phtiraptères (Mallophaga, par exemple Damalina spp., et Anoplura, par exemple Linognathus et Haematopinus spp.), des hémiptères (par exemple Aphis, Bemisia, Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleyrodes, Triatoma, Psylla, Myzus, Megoura, Phylloxera, Adelges, Nilaparvata, Nephotettix ou Cimex spp.), des orthoptères (par exemple Locusta, Gryllus, Schistocerca ou Acheta spp.), des dictyoptères (par exemple Blattella, Periplaneta ou Blatta spp.), des hyménoptères (par exemple Athalia, Cephus, Atta, Solenopsis ou Monomorium spp.), des isoptères (par exemple Odontotermes et Reticulitermes spp.), des siphonaptères (par exemple Ctenocephalides ou Pulex spp.), des thysanures (par exemple Lepisma spp.), des dermaptères (par exemple Forficula spp.) et des psocoptères (par exemple Peripsocus spp.) et des thysanoptères (par exemple Thrips tabaci).

Des acariens nuisibles sont notamment les tiques, par exemple les membres des genres Boophilus, Ornithodorus, Rhipicephalus, Amblyomma, Hyalomma, Ixodes, Haemaphysalis, Dermacentor et Anocentor, et les mites et les gales telles que Acarus, Tetranychus, Psoroptes, Notoednes, Sarcoptes, Psorergates, Chorioptes, Eutrombicula, Demodex, Panonychus, Bryobia, Eriophyes, Blaniulus, Polyphagotarsonemus, Scutigerella et Oniscus spp.

Les nématodes qui attaquent les plantes et les arbres importants en agriculture, sylviculture et horticulture, tant directement qu'en propageant des maladies bactériennes, virales, mycoplasmiques ou

fongiques des plantes, sont notamment les nématodes des gales des racines, comme Meloidogyne spp. (par exemple M. incognita); les nématodes des racines, comme Globodera spp. (par exemple G. rostochiensis); Heterodera spp. (par exemple H. avenae); Radopholus spp. (par exemple R. similis); les nématodes des prairies, comme Pratylenchus spp. (par exemple P. pratensis); Belonolaimus spp (par exemple B. gracilis); Tylenchulus spp. (par exemple T. semipenetrans); Rotylenchulus spp. (par exemple R. reniformis); Rotylenchus spp. (par exemple R. robustus); Helicotylenchus spp. (par exemple H. multicinctus); Hemicycliophora spp. (par exemple H. gracilis); Criconemoides spp. (par exemple C. similis); Trichodorus spp. (par exemple T. primitivus); les nématodes xiphophores, comme Xiphinema spp. (par exemple X. diversicaudatum), Longidorus spp. (par exemple L. elongatus); Hoplolaimus spp. (par exemple H. coronatus); Aphelenchoides spp. (par exemple A. ritzema-bosi, A. besseyi); et les nématodes des bulbes, comme Ditylenchus spp. (par exemple D dipsaci).

Les composés de l'invention peuvent être combinés avec un ou plusieurs autres constituants pesticides actifs (par exemple des pyréthroïdes, des carbamates et des organophosphates) et/ou avec des attractifs, des répulsifs, des bactéricides, des fongicides, des nématocides, des anthelminthiques et ainsi de suite. De surcroît, il a été observé que l'activité des composés de l'invention peut être accrue par l'addition d'un agent de synergie ou de potentialisation, par exemple un agent de synergie de la classe des inhibiteurs de l'oxydase, comme le pipéronylbutoxyde ou le 2-propynylphénylphosphonate de propyle; un deuxième composé de l'invention ou un pyréthroïde pesticide. Lorsqu'un agent de synergie inhibiteur de l'oxydase est présent dans une composition de l'invention, le rapport de l'agent de synergie au composé de formule (I) se situe dans l'intervalle de 25:1 à 1:25, par exemple à environ 10:1.

Des stabilisants pour empêcher toute dégradation chimique que peuvent subir les composés de l'invention sont notamment, par exemple, des antioxydants (comme les tocophérols, le butylhydroxyanisole et le butylhydroxytoluène) et des capteurs (comme l'épichlorhydrine), de même que des bases organiques et inorganiques, par exemple des trialcoylamines telles que la triéthylamine, qui peuvent agir comme stabilisants basiques et capteurs.

Possibilités d'application industrielle.

Les composés de la présente invention ont des propriétés pesticides et/ou une photostabilité accrues et/ou une toxicité réduite pour les mammifères.

Les exemples suivants illustrent de façon non limitative des aspects préférés de l'invention.

Compositions.

1. Concentré émulsionnable.

| | |
|---|---|
| Composé de formule (I) | 10,00 |
| Alcoylphénol éthoxylé * | 7,50 |
| Alcoylarylsulfonate * | 2,50 |
| Solvant aromatique en $C_{8-13}$ | 80,00 |
| | 100,00 |

* Tensioactif.

2. Concentré émulsionnable.

| | |
|---|---|
| Composé de formule (I) | 10,00 |
| Alcoylphénol éthoxylé * | 2,50 |
| Alcoylarylsulfonate * | 2,50 |
| Solvant cétonique | 64,00 |
| Solvant aromatique en $C_{8-13}$ | 18,00 |
| Antioxydant | 3,00 |
| | 100,00 |

* Tensioactif.

3. Poudre mouillable.

| | |
|---|---|
| Composé de formule (I) | 5,00 |
| Solvant aromatique en $C_{8-13}$ | 7,00 |
| Solvant aromatique en $C_{18}$ | 28,00 |
| Kaolin | 10,00 |
| Alcoylarylsulfonate * | 1,00 |
| Acide naphtalènesulfonique * | 3,00 |
| Terre de diatomées | 46,00 |
| | 100,00 |

* Tensioactif.

4. Poudre à poudrer.

| | |
|---|---|
| Composé de formule (I) | 0,50 |
| Talc | 99,50 |
| | 100,00 |

5. Appât.

| | |
|---|---|
| Composé de formule (I) | 0,5 |
| Sucre | 79,5 |
| Cire de paraffine | 20,0 |
| | 100,00 |

6. Concentré en émulsion.

| | |
|---|---|
| Composé de formule (I) | 5,00 |
| Solvant aromatique en $C_{8-13}$ | 32,00 |
| Alcool cétylique | 3,00 |
| Monooléate de polyoxyéthylèneglycérol * | 0,75 |
| Esters de polyoxyéthylènesorbitan * | 0,25 |
| Solution de silicone | 0,1 |
| Eau | 58,9 |
| | 100,00 |

* Tensioactif.

7. Concentré en suspension.

| | |
|---|---|
| Composé de formule (I) | 10,00 |
| Alcoylphénol éthoxylé * | 3,00 |
| Solution de silicone | 0,1 |
| Alcanediol | 5,0 |
| Fumée de silice | 0,50 |
| Gomme de xanthane | 0,20 |
| Eau | 80,0 |
| Agent tampon | 1,2 |
| | 100,00 |

* Tensioactif.

8. Microémulsion.

| | |
|---|---|
| Composé de formule (I) | 10,00 |
| Monooléate de polyoxyéthylèneglycérol * | 10,00 |
| Alcanediol | 4,00 |
| Eau | 76,00 |
| | 100,00 |

* Tensioactif.

9. Granules dispersables dans l'eau.

| | |
|---|---|
| Composé de formule (I) | 70,00 |
| Polyvinylpyrrolidine | 2,50 |
| Alcoylphénol éthoxylé | 1,25 |
| Alcoylarylsulfonate | 1,25 |
| Kaolin | 25,00 |
| | 100,00 |

10. Granules.

| | |
|---|---|
| Composé de formule (I) | 2,00 |
| Alcoylphénol éthoxylé * | 5,00 |
| Alcoylarylsulfonate * | 3,00 |
| Solvant aromatique $C_{8-13}$ | 20,00 |
| Granules de kieselguhr | 70,00 |
| | 100,00 |

* Tensioactif.

11. Aérosol (bombe).

| | |
|---|---|
| Composé de formule (I) | 0,3 |
| Pipéronylbutoxyde | 1,5 |
| Solvant hydrocarboné saturé en $C_{8-13}$ | 58,2 |
| Butane | 40,0 |
| | 100,00 |

12. Aérosol (bombe).

| | |
|---|---|
| Composé de formule (I) | 0,3 |
| Solvant hydrocarboné saturé en $C_{8-13}$ | 10,0 |
| Monooléate de sorbitan * | 1,0 |
| Eau | 40,0 |
| Butane | 48,7 |
| | 100,00 |

* Tensioactif.

13. Aérosol (bombe).

| | |
|---|---|
| Composé de formule (I) | 1,00 |
| $CO_2$ | 3,00 |
| Monooléate de polyoxyéthylèneglycérol * | 1,40 |
| Propanone | 38,00 |
| Eau | 56,60 |
| | 100,00 |

* Tensioactif.

14. Vernis.

| | |
|---|---|
| Composé de formule (I) | 2,50 |
| Résine | 5,00 |
| Antioxydant | 0,50 |
| White spirit très aromatique | 92,0 |
| | 100,00 |

15. Spray (prêt à l'usage).

| | |
|---|---|
| Composé de formule (I) | 0,10 |
| Antioxydant | 0,10 |
| Kérosène inodore | 99,8 |
| | 100,00 |

16. Spray potentialisé (prêt à l'usage).

| | |
|---|---|
| Composé de formule (I) | 0,10 |
| Pipéronylbutoxyde | 0,50 |
| Antioxydant | 0,10 |
| Kérosène inodore | 99,30 |
| | 100,00 |

17. Microcapsules.

| | |
|---|---|
| Composé de formule (I) | 10,0 |
| Solvant aromatique en $C_{8-13}$ | 10,0 |
| Diisocyanate aromatique # | 4,5 |
| Alcoylphénol éthoxylé * | 6,0 |
| Alcoyldiamine # | 1,0 |
| Diéthylènetriamine | 1,0 |
| Acide chlorhydrique concentré | 2,2 |
| Gomme de xanthane | 0,2 |
| Fumée de silice | 0,5 |
| Eau | 64,6 |
| | 100,00 |

* Tensioactif.
# Réagit en formant les parois de polyurée des microcapsules.

18. Concentré dispersable.

| | |
|---|---|
| Composé de formule (I) | 5,0 |
| N-méthylpyrrolidinone | 15,00 |
| N-alcoylpyrrolidinone | 53,00 |
| Solvant aromatique en $C_{8-13}$ Phosphate d'éther polyoxyéthylénique de nonylphénol | 16,00<br>6,00 |
| Alcoylphénol éthoxylé | 3,50 |
| Alcoylarylsulfonate | 1,30 |
| Ether polyalcoylèneglycolique | 0,20 |
| | 100,00 |

L'antioxydant peut être l'un quelconque des composés suivants pris isolément ou en combinaison : hydroxytoluène butylé, hydroxyanisole butylé et vitamine C (acide ascorbique).

Les exemples suivants illustrent de façon non limitative certains aspects préférés de l'invention.

## SECTION EXPERIMENTALE.

### Procédés et modes opératoires généraux de synthèse.

Différents composés ont été synthétisés et caractérisés suivant les techniques expérimentales ci-après.

Les spectres $^1H$ RMN ont été relevés sur un spectromètre Bruker AM-250 sur des solutions dans du deutérochloroforme contenant du tétraméthylsilane comme étalon interne et sont exprimés en ppm par rapport à TMS, nombre de protons, nombre de pics et constante de couplage J Hz.

L'avancement des réactions a pu être surveillé convenablement sur des feuilles d'aluminium (40 mm sur 80 mm) préalablement recouvertes d'une couche de 0,25 mm de gel de silice avec un indicateur fluorescent et développées dans un solvant ou mélange de solvants qui convient. Les températures sont toujours en degrés Celsius.

L'éther diéthylique, l'hexane, l'éthanol, le méthanol, la triéthylamine, la pyridine, le sulfate de magnésium et l'hydroxyde de sodium sont acquis chez BDH. Le dichlorométhane est acquis chez Romil Chemicals et le diméthylformamide chez Rathburn Chemicals Ltd. L'origine des autres composés chimiques est indiquée dans le texte.

### EXEMPLE A.-

(±)-(2E,4E)-N-Phényl-5-[c-2-(3,4-dibromophényl)-r-1-fluorocyclopropyl]penta-2,4-diénamide (1).

(i) On prépare le (Z)-3-(3,4-dibromophényl)-2-fluoroprop-2-énoate d'éthyle par un procédé analogue à celui de l'exemple 24 (EP 0 369 762 Al) à partir de 3,4-dibromobenzaldéhyde (exemple 14, EP 0 369 762 Al).

$^1H$ RMN : 1,2 (3H, t); 4,3 (24, q); 6,6 (1H, d); 7,4 (3H, m).

(ii) On prépare le (±)-5-[c-2-(3,4-dibromophényl)-r-1-fluorocyclopropyl]penta-2,4-diénoate d'éthyle à partir du composé ci-dessus par un procédé analogue à l'exemple 1 (EP 0 369 762 Al) au moyen de 4-phosphonocrotonate de triéthyle (acquis chez Lancaster).

$^1H$ RMN : 1,3 (3H, t); 1,6 (1H, m); 2,3 (1H, m); 4,2 (2H, q); 5,8 (1H, d); 5,9 (1H, dd); 6,4 (1H, dd); 7,0 (1H, m); 7,3 (1H, dd); 7,5 (2H, m).

(iii) On agite et on chauffe à 50°C du (±)-5-[c-2-(3,4-dibromophényl)-r-1-fluorocyclopropyl]penta-2,4-diénoate d'éthyle (3,5 g) dans de l'éthanol (50 ml). On ajoute une solution d'hydroxyde de sodium (0,8 g) dans de l'eau (5 ml) et on poursuit le chauffage pendant encore 3 heures. On chasse le solvant sous vide et on ajoute de l'eau et de l'éther diéthylique. On sépare la solution aqueuse et on l'acidifie avec de l'acide chlorhydrique dilué. On extrait le précipité à l'éther diéthylique, on lave la couche organique à la saumure et on la sèche sur du sulfate de magnésium. Par élimination du solvant sous vide, on obtient l'acide (±)-5-[c-2-(3,4-dibromophényl)-r-1-fluorocyclopropyl]penta-2,4-diénoïque (2,5 g).

$^1H$ RMN : 1,5 (1H, m); 1,8 (1H, m): 2,3 (1H, m), 5,9 (1H, m); 6,4 (1H, dd); 7,0 (1H, m); 7,3 (1H, dd); 7,5 (2H, m).

(iv) On met l'acide ci-dessus (360 mg) en suspension dans du dichlorométhane (15 ml) et on agite le tout sous azote à la température ambiante tandis qu'on ajoute du chlorure d'oxalyle (acquis chez Aldrich)

(156 mg, 107 µl) et du diméthylformamide (1 goutte). On poursuit l'agitation pendant 2 heures et on chasse le solvant sous vide. On dissout le solide résiduel dans du dichlorométhane (20 ml) et on ajoute de la triéthylamine (123 mg, 170 µl) et de l'aniline (acquise chez Aldrich) (114 mg, 112 µl). Après agitation jusqu'au lendemain, on lave la solution organique successivement avec de l'acide chlorhydrique 2N, de l'hydrogénocarbonate de sodium saturé et de la saumure, puis on la sèche sur du sulfate de magnésium. Par élimination du solvant sous vide, on obtient, après cristallisation dans le méthanol, le composé annoncé au titre (75 mg). P.F. 174 à 175 °C.

$^1$H RMN (DMSO) : 1,7 (1H, m); 2,1 (1H, m); 2,6 (1H, m); 6,3 (2H, m); 6,5 (1H, dd); 7,1 (1H, dd); 7,5 (4H, m).

On prépare les composés 1 à 3 de manière analogue à l'aide du réactant de Wittig et de l'aniline qui sont spécifiés.

EXEMPLE B.-

(±)-(2E,4E)-N-Phényl-5-[c-2-(3,4-dibromophényl)-r-1-fluorocyclopropyl]3-méthylpenta-2,4-diénamide(2).

(i) On prépare le(±)-5-[c-2-(3,4-dibromophényl)-r-1-fluorocyclopropyl]-3-méthylpenta-2,4-diénoate d'éthyle au moyen de 3-méthyl-4-phosphonocrotonate de triéthyle (acquis chez Lancaster) comme décrit dans l'exemple A(ii) ci-dessus.

$^1$H RMN : 1,3 (3H, t); 1,6 (1H, m); 1,8 (1H, m); 2,0 et 2,3 (3H, s); 2,3 (1H, m); 4,2 (2H, q); 6,0 et 7,8 (3H, m); 7,0 (1H, m); 7,5 (2H, m).

(ii) On hydrolyse l'ester ci-dessus dans les conditions décrites dans l'exemple A(iii) pour obtenir l'acide (±)-5-[c-2-(3,4-dibromophényl)-r-1-fluorocyclopropyl]-3-méthylpenta-2,4-diénoïque.

$^1$H RMN : 1,6 (1H, m); 1,8 (1H, m); 2,1 et 2,3 (3H, s); 2,3 (1H, m); 5,7 et 5,8 (1H, s); 6,0 (2H, m); 6,4 et 7,8 (1H, m); 7,0 (1H, m), 7,5 (2H, m).

(iii) On met l'acide ci-dessus (3,0 g) en suspension dans du dichlorométhane sec (50 ml) et on agite le tout sous azote à 0 °C, tandis qu'on ajoute du chlorure d'oxalyle (840 µl) et du diméthylformamide (1 goutte). On poursuit l'agitation pendant 2 heures et on chasse le solvant sous vide. On ajoute du dichlorométhane (100 ml) et on agite la solution à -20 °C sous azote. On ajoute de la pyridine (790 µl) et de l'aniline (880 µl) et on poursuit l'agitation pendant encore 17 heures. On ajoute de l'eau et du dichlorométhane, on sépare la phase organique et on la lave successivement avec de l'acide chlorhydrique 2N, de l'hydrogénocarbonate de sodium saturé et de la saumure, après quoi on la sèche sur du sulfate de magnésium. Après filtration, on évapore le filtrat sous pression réduite pour recueillir un solide (3,0 g). Par purification par chromatographie (silice, éther-hexane), on obtient le composé annoncé au titre (1,1 g). P.F. 138 °C.

$^1$H RMN : 1,6 (1H, m); 1,8 (1H, m); 2,3 (1H, m); 2,4 (3H, s); 5,8 (2H, m); 6,4 (1H, d); 7,5 (8H, m).

(±)-(2E,4E)-N-2-Méthylphényl-5-[c-2-(3,4-dibromophényl)-r-1-fluorocyclopropyl]penta-2,4-diénamide(19).

On fait réagir de l'acide (±)-5-[c-2-(3,4-dibromophényl)-r-1-fluorocyclopropyl]penta-2,4-diénoïque (préparé comme dans l'exemple A) (132 g) dans du dichlorométhane (5 litres) avec du chlorure d'oxalyle (56 g) et du diméthylformamide (10 gouttes) (voir exemple A). Après avoir chassé les constituants volatils, on dissout le chlorure d'acide dans du dichlorométhane sec (5 litres) et on refroidit la solution à -10 °C. On ajoute de la pyridine (34 g) et 5 minutes plus tard, de l'o-toluidine (46 g). On laisse le mélange reposer à la température ambiante jusqu'au lendemain. On dilue le mélange de réaction à l'hexane et on recueille le produit par filtration, on le lave à l'hexane et on le sèche sous vide. On triture le produit brut à chaud dans de l'isopropanol, on le collecte par filtration, on le lave à l'hexane et on le sèche pour obtenir le composé annoncé au titre (258 g).

(±)-(2E,4E)-N-2-Méthylphényl-5-[c-2-(3,4-dichlorophényl)-r-1-fluorocyclopropyl]penta-2,4-diénamide (68).

On prépare l'acide (±)-5-[c-2-(3,4-dichlorophényl)-r-1-fluorocyclopropyl]penta-2,4-diénoïque par analogue avec l'exemple A à partir de 3,4-dichlorobenzaldéhyde (acquis chez Aldrich). On fait réagir l'acide ci-dessus (1 g) dans du dichlorométhane avec du chlorure d'oxalyle (0,36 ml) et du diméthylformamide (2 gouttes). On fait réagir le chlorure d'acide dans le dichlorométhane avec de la pyridine (0,34 ml) et de l'o-toluidine (0,44 g) à -10 °C. Au terme de la réaction, on traite le mélange de réaction comme décrit précédemment et on lave le produit brut avec de l'éther-hexane, puis on le sèche sous vide pour obtenir le composé annoncé au titre (0,64 g).

On prépare les composés 1 et 4 à 85 de manière analogue à l'aide du réactant de Wittig et de l'aniline qui sont spécifiés (tableau I).

EXEMPLE C.-

(±)-(2E,4E)-N-2-Méthylphényl-5-[c-2-(3,4-dibromophényl)-r-1-fluorocyclopropyl]penta-2,4-diénamide(19).

N-2-Méthylphényldiéthyl-4-phosphonocrotonamide.

(i) On mélange du N-bromosuccinimide (136 g) avec du peroxyde de benzoyle et on les ajoute en quatre fractions à de l'acide crotonique (60 g) dans du tétrachlorométhane (400 ml) chauffé au reflux. Après 2 heures de chauffage, on ajoute de l'acide chlorhydrique dilué au mélange refroidi et on sépare la phase organique qu'on sèche et qu'on concentre pour recueillir l'acide 4-bromocrotonique (35 g).

(ii) On agite de l'acide 4-bromocrotonique (98 g) et du chlorure de thionyle (250 ml) pendant 18 heures à la température ambiante, puis on chasse les constituants volatils pour recueillir le chlorure de 4-bromocrotonyle (107 g).

(iii) On ajoute du chlorure de 4-bromocrotonyle (30 g) dans de l'éther sec à une solution de pyridine (14,3 g) et d'o-toluidine (22,8 g) dans de l'éther à 0 °C, sous vive agitation.

(iv) On chauffe du N-2-méthylphényl-4-bromocrotonamide (20 g) et du phosphite de triéthyle (16,7 g) ensemble à 140 °C en chassant par distillation le bromoéthane qui se forme. On purifie le produit brut par chromatographie sur de la silice (5% d'éthanol dans de l'acétate d'éthyle) pour recueillir le phosphonocrotonamide sous la forme d'un solide incolore (P.F. 84 °C).

$^1$H RMN : 7,83 (1H, s); 7,71 (1H); 7,16, 7,09 (3H, 2m); 6,82 (1H, s); 6,07 (1H, d de d); 2,22 (3H, s); 1,36 (6H, t).

On ajoute du 2-(3,4-dibromophényl)-1-fluorocyclopropylméthanal (préparé à partir de 3,4-dibromobenzaldéhyde par analogie avec les exemples cités dans le document EP 0 369 762) (2,5 g) à un mélange du phosphonocrotonamide précité (3,63 g) et de carbonate de potassium (1,38 g) dans du méthanol (100 ml). On agite le mélange de réaction à la température ambiante pendant 2 jours et on le dilue à l'eau. On recueille par filtration le produit solide qu'on sèche, qu'on lave à l'hexane et qu'on sèche sous vide pour recueillir le composé annoncé au titre (1,88 g).

Soit

On prépare une solution de diisopropylamidure de lithium dans du tétrahydrofuranne sec à partir de n-butyllithium dans l'hexane (2,58 ml à 1,6 mole par litre) et de diisopropylamine (0,63 ml). On fait réagir ce composé comme le phosphonocrotonamide précité (1,24 g) dans du tétrahydrofuranne à -78 °C pendant 2 heures. On ajoute du 2-(3,4-dibromophényl)-1-fluorocyclopropylméthanal (1,21 g). Après 18 heures à la température ambiante, on ajoute de l'acide chlorhydrique aqueux dilué et on recueille le produit brut par filtration. On triture ce dernier dans de l'isopropanol chaud, on le recueille par filtration et on le sèche sous vide pour obtenir le composé annoncé au titre (1,73 g).

EXEMPLE D.-

(±)-(2E,4E)-N-(2-Fluorométhylphényl)-5-[c-2-(3,4-dibromophényl)-r-1-fluorocyclopropyl]penta-2,4-diénamide (86).

On ajoute goutte à goutte une solution du composé 64 (100 mg, 0,2 millimole) dans du dichlorométhane (10 ml) à une solution agitée de trifluorure de diéthylamino-soufre (0,05 ml, 0,2 millimole) dans du dichlorométhane (5 ml) à -78 °C sous azote. On agite le mélange de réaction pendant 4,5 heures en le laissant se réchauffer jusqu'à la température ambiante. On verse le mélange de réaction dans de l'eau (50 ml) et on sèche la couche organique (sulfate de magnésium). Par évaporation du solvant, on obtient un solide brun foncé qu'on triture dans l'éther pour recueillir, sous la forme d'un solide brun pâle, le composé annoncé au titre (12 mg, 12%). P.F. 163 à 165 °C, $M^{+1}$ = 566.

$^1$H RMN DMSO : 1,6 (1H, m); 2,1 (1H, m); 2,5 (1H, m); 5,4 (2H, d); 6,4 (3H, m), 7,4 (8H, m); 9,7 (1H, s).

EXEMPLE E.-

(±)-(2E,4E)-N-(2-Hydroxyphényl)-5-[c-2-(3,4-dibromophényl)-r-1-fluorocyclopropyl]penta-2,4-diénamide(87).

(i) On ajoute de l'imidazole (3,73 g) en une fois à une solution de 2-aminophénol (2,0 g) dans du diméthylformamide sec (10 ml) agité à 25 °C sous azote. On ajoute goutte à goutte du chlorotriméthylsi-

lane (2,98 g) en 5 minutes et on poursuit l'agitation pendant encore 18 heures. On verse le mélange dans de l'eau (30 ml) et on extrait le tout à l'éther diéthylique (2 x 60 ml). On sépare la solution organique et on la lave avec de l'hydroxyde de sodium 2N (2 x 40 ml) et de la saumure, puis on la sèche sur du sulfate de magnésium. Par évaporation du filtrat sous pression réduite, on obtient une huile. Par chromatographie sur une colonne de gel de silice éluée avec 10% d'éther dans l'hexane, on obtient la 2-triméthylsilyloxyaniline (0,85 g).

$^1$H RMN : 0,2 (9H, s); 3,5 (2H, s); 6,4 (4H, m).

(ii) On prépare l'amide correspondant de l'acide (±)-(2E,4E)-5-[c-2-(3,4-dibromophényl)-r-1-fluorocyclopropyl]penta-2,4-diénoïque suivant la technique décrite dans l'exemple B(iii).

(iii) On ajoute du fluorure de tétrabutylammonium (solution 1M dans du tétrahydrofuranne; 1,5 ml) en 5 minutes à une solution agitée du composé ci-dessus (550 mg) dans du tétrahydrofuranne (10 ml) maintenue sous azote à la température ambiante. On poursuit l'agitation pendant encore 18 heures au terme desquelles on ajoute de l'éther diéthylique (50 ml) et de l'eau (40 ml). On sépare la solution organique, on la lave à la saumure et on la sèche ($MgSO_4$). Par évaporation du filtrat sous pression réduite, on obtient le produit brut. Par chromatographie sur de la silice éluée avec de l'éther diéthylique-hexane, on obtient le composé annoncé au titre (87) (92 mg).

EXEMPLE F.-

(i) Séparation des énantiomères du (±)-(2E,4E)-5-[c-2-(3,4-dibromophényl)-r-1-fluorocyclopropyl]penta-2,4-diénoate d'éthyle.

On sépare les énantiomères du composé ci-dessus par hplc préparatoire sur une colonne chirale à cellule OD (25 mm sur 200 mm). On procède à des injections multiples (5 ml) de l'ester ci-dessus (800 mg) dans de l'isopropanol chaud (solution à 2%). La phase mobile est formée de 6% d'isopropanol dans de l'hexane pour une élution au débit de 9 ml par minute. On combine les fractions et on les collecte à 27 minutes. [α] = +304,4° (300 mg) (Fraction A) et à 46 minutes, [α] = -272,9° (300 mg) (Fraction B). On peut réaliser la séparation des énantiomères du composé ci-dessus comme décrit dans la demande de brevet USA n° 07/811 439.

(ii) (±)-(2E,4E)-N-2-Méthylphényl-5-[(1S,2R)-2-(3,4-dibromophényl)-1-fluorocyclopropyl]penta-2,4-diénamide (88).

On hydrolyse la fraction A ci-dessus dans les conditions décrites dans l'exemple A(iii) pour recueillir l'acide correspondant [α] = +280,5°. Par traitement avec de l'o-toluidine dans les conditions décrites dans l'exemple B(iii), on obtient le composé annoncé au titre (68 mg), P.F. 185 à 186°C, [α] = +263,2°.

$^1$H RMN : 1,5 (1H, m); 1,8 (1H, m); 2,4 (1H, m); 2,4 (3H, s); 5,9 (2H, m); 6,5 (1H, dd); 7,0 à 8,0 (9H, m).

(iii) (-)-(2E,4E)-N-2-Méthylphényl-5-[(1R,2S)-2-(3,4-dibromophényl)-r-1-fluorocyclopropyl]penta-2,4-diénamide 89.

On hydrolyse la fraction B dans les conditions décrites dans l'exemple A(iii) pour obtenir l'acide correspondant, [α] = -276,8°. Par traitement avec de l'o-toluidine dans les conditions décrites dans l'exemple B(iii), on obtient le composé annoncé au titre (250 mg), P.F. 174 à 176°C.

$^1$H RMN : 1,5 (1H, m), 1,8 (1H, m), 2,4 (1H, m), 2,4 (3H, s); 5,9 (2H, m); 6,5 (1H, dd), 7,0 à 8,0 (9H, m).

EXEMPLE G.-

(±)-(2E,4E)-N-2-Méthylphényl-5-[r-1-chloro-c-(3,4-dichlorophényl)cyclopropyl]-3-méthylpenta-2,4-diénamide (90).

On prépare le (±)-(2E,4E)-3-méthyl-[r-1-chloro-c-(3,4-dichlorophényl)cyclopropyl]penta-2,4-diénoate d'éthyle comme décrit dans l'exemple 25 du document EP 0 369 762 Al. On convertit l'ester ci-dessus, par analogie avec l'exemple B(ii) et B(iii), en le composé annoncé au titre. P.F. = 170,6°C.

$^1$H RMN : 1,8 (2H, m); 2,3 (3H, s); 2,4 (3H, s); 2,5 (1H, m); 5,9 (2H, m); 6,5 (1H, d); 6,9 à 8,0 (8H, m).

On prépare les composés 91 à 95 d'une manière analogue à partir de l'aldéhyde approprié.

EXEMPLE H.-

(±)-(2Z,4E)-N-2-Méthylphényl-5-[c-2-(3,4-dibromophényl)-r-1-fluorocyclopropyl]-2-fluoro-3-méthylpenta-2,4-diénamide (96).

On traite du (±)-c-2-(3,4-dibromophényl)-r-1-fluorocyclopropylméthanal (exemple A) comme décrit dans l'exemple 23 du document EP 0 369 762 Al pour obtenir le (±)-(2Z,4E)-5-[c-2-(3,4-dibromophényl)-r-1-fluorocyclopropyl]-2-fluoro-3-méthylpenta-2,4-diénoate d'éthyle. On convertit l'ester ci-dessus, par analogie avec l'exemple B(ii) et B(iii), en le composé annoncé au titre. P.F. 104 à 105 °C, $M^{+1}$ = 510.

$^1$H RMN : 1,6 (2H, m); 1,8 (1H, m); 2,3 (3H, s); 2,4 (3H, d); 5,9 (1H, dd); 6,9 (1H, d); 7,0 à 8,0 (8H, m).

On prépare de manière analogue le (±)-(2Z,4E)-N-(2-chlorophényl)-5-[c-2-(3,4-dibromophényl)-r-1-fluorocyclopropyl]-2-fluoro-3-méthylpenta-2,4-diénamide (97).P.F. 128 à 129 °C, $M^{+1}$ = 530.

$^1$H RMN : 1,6 (1H, m); 1,9 (1H, m); 2,3 (1H, m); 2,4 (3H, s), 5,9 (1H, dd); 6,9 (1H, d); 7,0 à 7,6 (7H, m); 8,6 (1H, m).

EXEMPLE I.-

(±)-(2Z,4E)-N-(2-Méthylphényl)-5-[c-2-(3,4-dichlorophényl)-r-1-fluorocyclopropyl]-2-fluoropenta-2,4-diénamide (98).

On dissout du 2-fluorocrotonate d'éthyle (6,0 g) (préparé suivant E.D. Bergmann et I. Shahak, J. Chem. Soc. 4033 1961), du N-bromosuccinimide (8,1 g) et du peroxyde de benzoyle (0,15 g) dans du tétrachlorure de carbone (120 ml) et on les chauffe au reflux pendant 3 à 5 heures. On refroidit le mélange et on le filtre, puis on évapore le filtrat à siccité sous pression réduite. Par chromatographie sur de la silice éluée avec 10% d'éther diéthylique dans l'hexane, on obtient le 4-bromo-2-fluorocrotonate d'éthyle sous la forme d'une huile jaune (7,6 g).

$^1$H RMN : 1,5 (3H, t); 4,1 (2H, dd); 4,4 (2H, q) ; 6,4 (1H, dt).

On chauffe l'ester ci-dessus (7,6 g) avec du phosphite de triéthyle (6,6 g, 7,0 ml) dans les conditions de la distillation de 140 à 145 °C jusqu'à fin de distillation du bromure d'éthyle. On poursuit le chauffage pendant 1 heure à 150 °C et on refroidit la solution. Par chromatographie sur du gel de silice élué à l'éther diéthylique, on obtient, après évaporation, le 2-fluoro-4-phosphonocrotonatede triéthyle (4,65 g), $M^{+1}$ = 167.

$^1$H RMN : 1,5 (9H, t); 2,9 (2H, ddd); 4,4 (6H, m); 6,4 (1H, ddt).

On ajoute une solution de n-butyllithium (3,0 ml) (1,6 M dans l'hexane) à une solution agitée de diisopropylamine (0,66 ml) dans du tétrahydrofuranne sec (40 ml) maintenue à -10 °C sous azote. Après 30 minutes, on refroidit la solution à -40 °C et on y ajoute une solution de 2-fluoro-4-phosphonocrotonate de triéthyle (1,26 g) dans du tétrahydrofuranne sec (10 ml). On laisse la solution se réchauffer jusqu'à la température ambiante en 1,5 heure et on y ajoute une solution de (±)-c-2-(3,4-dichlorophényl)-r-1-fluorocyclopropylméthanol (0,89 g) (exemple B) dans du tétrahydrofuranne sec (10 ml). Après agitation pendant 18 heures, on ajoute de l'eau et on traite le mélange de façon normale. Par chromatographie sur de la silice éluée avec 30% d'éther diéthylique dans l'hexane, on obtient le (±)-(2Z,4E)-5-[c-2-(3,4-dichlorophényl)-r-1-fluorocyclopropyl]-2-fluoropenta-2,4-diénoate d'éthyle sous la forme d'une huile incolore (1,23 g).

$^1$H RMN : 1,4 (3H, t); 1,5 à 2,5 (3H, m), 4,4 (2H, q), 5,8 à 7,6 (3H, m).

On convertit l'ester ci-dessus, par analogie avec l'exemple B(ii) et B(iii), en le composé annoncé au titre. $M^{+1}$ = 408.

$^1$H RMN : 1,7 (1H, m); 2,0 (1H, m); 2,2 (3H, s); 2,6 (1H, m); 6,3 (1H, dd); 6,5 (1H, dd); 6,8 (1H, dd); 7,1 à 7,6 (7H, m); 9,8 (1H, s).

On prépare les composés 99 à 101 de manière analogue à l'aide du benzaldéhyde substitué et de l'aniline.

EXEMPLE J.-

(±)-(2E,4E)-N-(2-Méthylphényl)-5-[trans-2-(3,4-dibromophényl)-cyclopropyl]-3-méthylpenta-2,4-diénamide (102).

La préparation du (±)-(2E,4E)-5-[trans-2-(3,4-dibromophényl)cyclopropyl]-3-méthylpenta-2,4-diénoate d'éthyle est décrite dans l'exemple 14 du document EP 0 369 762 Al. Par analogie avec l'exemple B(ii) et B(iii), on convertit l'ester ci-dessus en le composé annoncé au titre, $M^{+1}$ = 474.

$^{1}H$ RMN : 1,3 (2H, m); 1,8 (1H, m); 2,0 (1H, m); 2,3 (3H, s); 2,4 (3H, s); 5,7 (2H, m); 6,2 (1H, d); 6,9 à 8,0 (8H, m).

On prépare de même le (±)-(2E,4E)-N-(2-méthylphényl)-5-[trans-2-(3,4-dibromophényl)-cyclopropyl]-penta-2,4-diénamide (composé 103). P.F. 165 °C, $M^{+1}$ = 460.

$^{1}H$ RMN : 1,3 (2H, m); 1,8 (1H, m); 2,0 (1H, m); 2,4 (3H, s); 5,7 (1H, dd); 6,0 (1H, d); 6,3 (1H, dd); 6,9 (1H, d); 7,0 à 8,0 (8H, m).

EXEMPLE K.-

(-)-(2E,4E)-N-(2-Méthylphényl)-5-[(1R,2S)-trans-2-(3,4-dibromophényl)cyclopropyl]-3-méthylpenta-2,4-diénamide (104).

On prépare le (-)5-[trans-2-(3,4-dibromophényl)cyclopropyl]-3-méthylpenta-2,4-diénoate d'éthyle par un procédé analogue à celui décrit dans l'exemple 12 du document EP 0 369 762 Al. Par analogie avec l'exemple B(ii) et B(iii), on convertit l'ester ci-dessus en le composé annoncé au titre $[\alpha]_D^{20}$ = -124 °.

$^{1}H$ RMN : 1,3 (2H, m); 1,8 (1H, m); 2,0 (1H, m); 2,3 (3H, s); 2,4 (3H, s); 5,7 (2H, m); 6,2 (1H, d); 6,9 à 8,0 (8H, m).

On prépare de manière analogue (exemple 12 du document EP 0 369 762 Al) le (+)-(2E,4E)-N-(2-méthylphényl)-5-[trans-2-(3,4-dibromophényl)cyclopropyl]-3-méthylpenta-2,4-diénamide (composé 105). $[\alpha]_D^{20}$ = +85 °.

$^{1}H$ RMN : 1,3 (2H, m); 1,8 (1H, m); 2,0 (1H, m); 2,3 (3H, s); 2,4 (3H, s); 5,7 (2H, m); 6,2 (1H, d); 6,9 à 8,0 (8H, m).

EXEMPLE L.-

(±)-(2E,4E)-N-Phényl-5-[c-2-(3,4-dibromophényl)-r-1-fluorocyclopropyl]penta-2,4-diènethioamide (106).

On prépare le composé annoncé au titre à partir du c-2-(3,4-dibromophényl)-r-1-fluorocyclopropylméthanol (exemple B) d'une manière analogue à celle décrite dans le document EP-A-0 369 762 (exemple 26), mais en utilisant de l'isothiocyanate de phényle au lieu d'isothiocyanate d'isobutyle. P.F. = 68 °C, $M^{+1}$ = 480.

$^{1}H$ RMN : 1,5 (1H, m); 1,8 (1H, m); 2,3 (1H, m); 6,0 (1H, dd); 6,4 (2H, m); 7,0 à 7,9 (10H, m).

EXEMPLE Q.-

(±)-(2E,4E)-N-Méthyl-N-phényl-5-[c-2-(3,4-dibromonhényl)-r-1-fluorocyclopropyl]penta-2,4-diénamide(111).

On prépare l'amide ci-dessus à partir de N-méthylaniline et d'acide (±)-(2E,4E)-5-[c-2-(3,4-dibromophényl)-r-1-fluorocyclopropyl]penta-2,4-diénoïque suivant la technique décrite dans l'exemple B(iii). Le semi-solide donne $M^{+1}$ = 478.

$^{1}H$ RMN : 1,4 (1H, m); 1,7 (1H, m); 2,2 (1H, m); 3,4 (3H, s); 5,8 (2H, m); 6,2 (1H, m); 6,9 à 7,5 (9H, m).

On prépare les composés 112 à 119 d'une manière analogue à partir des acides et amines correspondants.

La N-méthylaniline est acquise chez Aldrich Chemical Company. La N-méthyl-o-toluidine est préparée suivant les procédés décrits dans la littérature (J. Chem. Soc. 1930, 992 à 994, Hickinbottom). On prépare de façon semblable les composés suivants : N-isopropyl-o-toluidine, N-isobutyl-o-toluidine, N-isopropylaniline et N-méthyl-o-chloroaniline. On prépare la N-benzyl-o-toluidine suivant Org. Syn. Col. Vol. 1, 102.

EXEMPLE R.-

(±)-(2E,4E)-N-(2-Méthylphényl)-N-(carboéthoxy-5-[c-2-(3,4-dibromophényl)-r-1-fluorocyclopropyl]penta-2,4-diénamide (120).

On prépare le chlorure d'acide à partir de l'acide diénoïque (523 mg) en solution dans du THF anhydre (4 ml) suivant l'exemple B. On fait réagir de la N-carboéthoxytoluidine (240 mg) (préparée à partir d'o-toluidine et de chloroformiate d'éthyle) dans du THF (4 ml) avec du n-butyllithium dans l'hexane (0,838 ml à 1,6 M) à -60 °C. Après 1 heure à -60 °C, on ajoute le chlorure d'acide précité et, après encore 1 heure à -50 °C, on traite le mélange de réaction de manière classique. Par purification chromatographique, on

obtient, sous la forme d'un solide vitreux, le composé annoncé au titre (300 mg). P.F. 40 à 53°C, $M^{+1}$ = 552.

$^1$H RMN : 1,24 (3H, t); 1,67, 1,80 (2H, 2m); 2,22 (3H, s); 2,33 (1H, m); 4,25 (2H, m); 6,01 (1H, d de d); 6,68 (1H, d de d); 6,96 (1H, d); 7,1 à 7,7 (8H, m).

| Composé n° | Nom du composé |
|---|---|

1. le (±)-(2E,4E)-N-phényl-5-[c-2-(3,4-dibromophényl)-r-1-fluorocyclopropyl]penta-2,4-diénamide,
2. le (±)-(2E,4E)-N-phényl-5-[c-2-(3,4-dibromophényl)-r-1-fluorocyclopropyl]-3-méthylpenta-2,4-diénamide,
3. le (±)-(2E,4E)-N-(2-chlorophényl)-5-[c-2-(3,4-dibromophényl)-r-1-fluorocyclopropyl]penta-2,4-diénamide,
4. le (±)-(2E/Z,4E)-N-(2-chlorophényl)-5-[c-2-(3,4-dibromophényl)-r-1-fluorocyclopropyl]-3-méthylpenta-2,4-diénamide (2E:2Z = 7:3),

5. le (±)-(2E,4E)-N-(4-chlorophényl)-5-[c-2-(3,4-dibromophényl)-r-1-fluorocyclopropyl]-3-méthylpenta-2,4-diénamide,

6. le (±)-(2E,4E)-N-(4-fluorophényl)-5-[c-2-(3,4-dibromophényl)-r-1-fluorocyclopropyl]-3-méthylpenta-2,4-diénamide,

7. le (±)-(2E/Z,4E)-N-(4-fluorophényl)-5-[c-2-(3,4-dibromophényl)-r-1-fluorocyclopropyl]-3-méthylpenta-2,4-diénamide (2E:2Z = 1:21),

8. le (±)-(2E/Z,4E)-N-(2-fluorophényl)-5-[c-2-(3,4-dibromophényl)-r-1-fluorocyclopropyl]-3-méthylpenta-2,4-diénamide (2E:2Z = 6:4),

9. le (±)-(2E,4E)-N-(3-chlorophényl)-5-[c-2-(3,4-dibromophényl)-r-1-fluorocyclopropyl]-3-méthylpenta-2,4-diénamide,

10. le (±)-(2E,4E)-N-(3-méthoxyphényl)-5-[c-2-(3,4-dibromophényl)-r-1-fluorocyclopropyl]-3-méthylpenta-2,4-diénamide,

11. le (±)-(2E,4E)-N-(2-trifluorométhylphényl)-5-[c-2-(3,4-dibromophényl)-r-1-fluorocyclopropyl]-3-méthylpenta-2,4-diénamide,

12. le (±)-(2E,4E)-N-(2-fluorophényl)-5-[c-2-(3,4-dibromophényl)-r-1-fluorocyclopropyl]penta-2,4-diénamide,

13. le (±)-(2E/Z,4E)-N-(2-méthylphényl)-5-[c-2-(3,4-dibromophényl)-r-1-fluorocyclopropyl]-3-méthylpenta-2,4-diénamide (2E:2Z = 3:1),

14. le (±)-(2E,4E)-N-(2-méthylphényl)-5-[c-2-(3,4-dibromophényl)-r-1-fluorocyclopropyl]-3-méthylpenta-2,4-diénamide,

15. le (±)-(2E,4E)-N-(2-dichlorophényl)-5-[c-2-(3,4-dibromophényl)-r-1-fluorocyclopropyl]penta-2,4-diénamide,

16. le (±)-(2E,4E)-N-(2,6-difluorophényl)-5-[c-2-(3,4-dibromophényl)-r-1-fluorocyclopropyl]penta-2,4-diénamide,

17. le (±)-(2E,4E)-N-(2-bromophényl)-5-[c-2-(3,4-dibromophényl)-r-1-fluorocyclopropyl]penta-2,4-diénamide,

18. le (±)-(2E,4E)-N-(2-éthylphényl)-5-[c-2-(3,4-dibromophényl)-r-1-fluorocyclopropyl]penta-2,4-diénamide,

19. le (±)-(2E,4E)-N-(2-méthylphényl)-5-[c-2-(3,4-dibromophényl)-r-1-fluorocyclopropyl]penta-2,4-diénamide,

20. le (±)-(2E,4E)-N-(2-trifluorométhylphényl)-5-[c-2-(3,4-dibromophényl)-r-1-fluorocyclopropyl]penta-2,4-diénamide,

21. le (±)-(2E,4E)-N-(4-fluorophényl)-5-[c-2-(3,4-dibromophényl)-r-1-fluorocyclopropyl]penta-2,4-diénamide,

22. le (±)-(2E,4E)-N-(3-fluorophényl)-5-[c-2-(3,4-dibromophényl)-r-1-fluorocyclopropyl]penta-2,4-diénamide,

23. le (±)-(2E,4E)-N-(4-méthylphényl)-5-[c-2-(3,4-dibromophényl)-r-1-fluorocyclopropyl]-3-méthylpenta-2,4-diénamide,

24. le (±)-(2E,4E)-N-(3-méthylphényl)-5-[c-2-(3,4-dibromophényl)-r-1-fluorocyclopropyl]-3-méthylpenta-2,4-diénamide,

25. le (±)-(2E,4E)-N-(4-méthylphényl)-5-[c-2-(3,4-dibromophényl)-r-1-fluorocyclopropyl]penta-2,4-diénamide,

26. le (±)-(2E,4E)-N-(3-méthylphényl)-5-[c-2-(3,4-dibromophényl)-r-1-fluorocyclopropyl]penta-2,4-diénamide,

27. le (±)-(2E,4E)-N-(2-fluoro-6-méthylphényl)-5-[c-2-(3,4-dibromophényl)-r-1-fluorocyclopropyl]penta-2,4-diénamide,

28. le (±)-(2E,4E)-N-(2,6-diméthylphényl)-5-[c-2-(3,4-dibromophényl)-r-1-fluorocyclopropyl]penta-2,4-diénamide,

29. le (±)-(2E,4E)-N-(4-méthoxyphényl)-5-[c-2-(3,4-dibromophényl)-r-1-fluorocyclopropyl]penta-2,4-diénamide,

30. le (±)-(2E,4E)-N-(2-chloro-5-méthylphényl)-5-[c-2-(3,4-dibromophényl)-r-1-fluorocyclopropyl]penta-2,4-diénamide,

31. le (±)-(2E,4E)-N-(4-fluoro-2-méthylphényl)-5-[c-2-(3,4-dibromophényl)-r-1-fluorocyclopropyl]penta-2,4-

diénamide,

32. le (±)-(2E,4E)-N-(3-fluoro-2-méthylphényl)-5-[c-2-(3,4-dibromophényl)-r-1-fluorocyclopropyl]penta-2,4-diénamide,

33. le (±)-(2E,4E)-N-(3-chloro-2-méthylphényl)-5-[c-2-(3,4-dibromophényl)-r-1-fluorocyclopropyl]penta-2,4-diénamide,

34. le (±)-(2E,4E)-N-(2-chloro-6-méthylphényl)-5-[c-2-(3,4-dibromophényl)-r-1-fluorocyclopropyl]penta-2,4-diénamide,

35. le (±)-(2E,4E)-N-(2,3-dichlorophényl)-5-[c-2-(3,4-dibromophényl)-r-1-fluorocyclopropyl]penta-2,4-diénamide,

36. le (±)-(2E,4E)-N-(4-trifluorométhylphényl)-5-[c-2-(3,4-dibromophényl)-r-1-fluorocyclopropyl]penta-2,4-diénamide,

37. le (±)-(2E,4E)-N-(3-méthoxyphényl)-5-[c-2-(3,4-dibromophényl)-r-1-fluorocyclopropyl]penta-2,4-diénamide,

38. le (±)-(2E,4E)-N-(2,5-diméthylphényl)-5-[c-2-(3,4-dibromophényl)-r-1-fluorocyclopropyl]penta-2,4-diénamide,

39. le (±)-(2E,4E)-N-(2-méthylthiophényl)-5-[c-2-(3,4-dibromophényl)-r-1-fluorocyclopropyl]penta-2,4-diénamide,

40. le (±)-(2E,4E)-N-(2-cyanophényl)-5-[c-2-(3,4-dibromophényl)-r-1-fluorocyclopropyl]penta-2,4-diénamide,

41. le (±)-(2E,4E)-N-(2-isopropylphényl)-5-[c-2-(3,4-dibromophényl)-r-1-fluorocyclopropyl]penta-2,4-diénamide,

42. le (±)-(2E,4E)-N-(5-chloro-2-méthylphényl)-5-[c-2-(3,4-dibromophényl)-r-1-fluorocyclopropyl]penta-2,4-diénamide,

43. le (±)-(2E,4E)-N-(4-fluoro-2-trifluorométhyl-phényl)-5-[c-2-(3,4-dibromophényl)-r-1-fluorocyclopropyl]-penta-2,4-diénamide,

44. le (±)-(2E,4E)-N-(2,3-diméthylphényl)-5-[c-2-(3,4-dibromophényl)-r-1-fluorocyclopropyl]penta-2,4-

diénamide,

45. le (±)-(2E,4E)-N-(2-nitrophényl)-5-[c-2-(3,4-dibromophényl)-r-1-fluorocyclopropyl]penta-2,4-diénamide,

46. le (±)-(2E,4E)-N-(5-fluoro-2-méthylphényl)-5-[c-2-(3,4-dibromophényl)-r-1-fluorocyclopropyl]penta-2,4-diénamide,

47. le (±)-(2E,4E)-N-(3-bromo-2-méthylphényl)-5-[c-2-(3,4-dibromophényl)-r-1-fluorocyclopropyl]penta-2,4-diénamide,

48. le (±)-(2E,4E)-N-(5-iodo-2-méthylphényl)-5-[c-2-(3,4-dibromophényl)-r-1-fluorocyclopropyl]penta-2,4-diénamide,

49. le (±)-(2E,4E)-N-(2-phénoxyphényl)-5-[c-2-(3,4-dibromophényl)-r-1-fluorocyclopropyl]penta-2,4-diénamide,

50. le (±)-(2E,4E)-N-(2-diméthylaminophényl)-5-[c-2-(3,4-dibromophényl)-r-1-fluorocyclopropyl]penta-2,4-diénamide,

51. le (±)-(2E,4E)-N-(2-éthoxycarbonylphényl)-5-[c-2-(3,4-dibromophényl)-r-1-fluorocyclopropyl]penta-2,4-diénamide,

52. le (±)-(2E,4E)-N-(2,4-difluorophényl)-5-[c-2-(3,4-dibromophényl)-r-1-fluorocyclopropyl]penta-2,4-diénamide,

53. le (±)-(2E,4E)-N-(2-acétylphényl)-5-[c-2-(3,4-dibromophényl)-r-1-fluorocyclopropyl]penta-2,4-diénamide,

54. le (±)-(2E,4E)-N-(2-chloro-4-fluorophényl)-5-[c-2-(3,4-dibromophényl)-r-1-fluorocyclopropyl]penta-2,4-diénamide,

55. le (±)-(2E,4E)-N-[2-(1-méthylvinyl)phényl]-5-[c-2-(3,4-dibromophényl)-r-1-fluorocyclopropyl]penta-2,4-diénamide,

56. le (±)-(2E,4E)-N-(2-iodophényl)-5-[c-2-(3,4-dibromophényl)-r-1-fluorocyclopropyl]penta-2,4-diénamide,

57. le (±)-(2E,4E)-N-(2-éthynylphényl)-5-[c-2-(3,4-dibromophényl)-r-1-fluorocyclopropyl]penta-2,4-diénamide,

58. le (±)-(2E,4E)-N-(2-formylphényl)-5-[c-2-(3,4-dibromophényl)-r-1-fluorocyclopropyl]penta-2,4-diénamide,

59. le (±)-(2E,4E)-N-(2-trifluorométhoxyphényl)-5-[c-2-(3,4-dibromophényl)-r-1-fluorocyclopropyl]penta-2,4-diénamide,

60. le (±)-(2E,4E)-N-(2-méthoxycarbonylphényl)-5-[c-2-(3,4-dibromophényl)-r-1-fluorocyclopropyl]penta-2,4-diénamide,

61. le (±)-(2E,4E)-N-(2-propylphényl)-5-[c-2-(3,4-dibromophényl)-r-1-fluorocyclopropyl]penta-2,4-diénamide,

62. le (±)-(2E,4E)-N-(2-fluorosulfonylphényl)-5-[c-2-(3,4-dibromophényl)-r-1-fluorocyclopropyl]penta-2,4-diénamide,

63. le (±)-(2E,4E)-N-(2-chlorophényl)-5-[c-2-(3,4-dibromophényl)-r-1-fluorocyclopropyl]-3-méthylpenta-2,4-diénamide,

64. le (±)-(2E,4E)-N-(2-hydroxyméthylphényl)-5-[c-2-(3,4-dibromophényl)-r-1-fluorocyclopropyl]penta-2,4-diénamide,

65. le (±)-(2E,4E)-N-(2-aminophényl)-5-[c-2-(3,4-dibromophényl)-r-1-fluorocyclopropyl]penta-2,4-diénamide,

66. le (±)-(2E,4E)-N-(2-chlorophényl)-5-[c-2-(3,4-dichlorophényl)-r-1-fluorocyclopropyl]penta-2,4-diénamide,

67. le (±)-(2E/Z,4E)-N-(2-chlorophényl)-5-[c-2-(3,4-dichlorophényl)-r-1-fluorocyclopropyl]-3-méthylpenta-2,4-diénamide (2E:2Z = 5:4),

68. le (±)-(2E,4E)-N-(2-méthylphényl)-5-[c-2-(3,4-dichlorophényl)-r-1-fluorocyclopropyl]penta-2,4-diénamide,

69. le (±)-(2E,4E)-N-(2-chlorophényl)-5-[r-1-fluoro-c-2-(3,4,5-trichlorophényl)cyclopropyl]penta-2,4-diénamide,

70. le (±)-(2E,4E)-N-(2-méthylphényl)-5-[r-1-fluoro-c-2-(3,4,5-trichlorophényl)cyclopropyl]penta-2,4-diénamide,

71. le (±)-(2E/Z,4E)-N-(2-méthylphényl)-5-[c-2-(3,4-dichlorophényl)-r-1-fluorocyclopropyl]-3-méthylpenta-2,4-diénamide (2E:2Z = 1:1),

72. le (±)-(2E,4E)-N-(2-méthylphényl)-5-[r-1-fluoro-c-2-(4-iodophényl)cyclopropyl]penta-2,4-diénamide,

73. le (±)-(2E,4E)-N-(2-méthylphényl)-5-[c-2-(3-chloro-4-iodophényl)-r-1-fluorocyclopropyl]penta-2,4-diénamide,

74. le (±)-(2E,4E)-N-(2-méthylphényl)-5-[r-1-fluoro-c-2-(4-trifluorométhylphényl)cyclopropyl]penta-2,4-diénamide,

75. le (±)-(2E,4E)-N-(2-chlorophényl)-5-[r-1-fluoro-c-2-(4-trifluorométhylphényl)cyclopropyl]penta-2,4-diénamide,

76. le (±)-(2E,4E)-N-(2-méthylphényl)-5-[r-1-fluoro-c-2-(4-trifluorométhylphényl)cyclopropyl]-3-méthylpenta-2,4-diénamide,

77. le (±)-(2E,4E)-N-(2-méthylphényl)-5-[r-1-fluoro-c-(4-iodophényl)cyclopropyl]-3-méthylpenta-2,4-diénamide,

78. le (±)-(2E,4E)-N-(5-fluoro-2-méthylphényl)-5-[c-2-(3,4-dichlorophényl)-r-1-fluorocyclopropyl]penta-2,4-diénamide,

79. le (±)-(2E,4E)-N-(5-fluoro-2-méthylphényl)-5-[r-1-fluoro-c-2-(3,4,5-trichlorophényl)cyclopropyl]penta-2,4-diénamide,

80. le (±)-(2E,4E)-N-(2-chloro-4-fluorophényl)-5-[r-1-fluoro-c-2-(3,4,5-trichlorophényl)cyclopropyl]penta-2,4-diénamide,

81. le (±)-(2E,4E)-N-(4-fluoro-2-méthylphényl)-5-[r-1-fluoro-c-2-(3,4,5-trichlorophényl)cyclopropyl]penta-2,4-diénamide,

82. le (±)-(2E,4E)-N-(4-fluoro-2-méthylphényl)-5-[c-2-(3,4-dichlorophényl)-r-1-fluorocyclopropyl]penta-2,4-diénamide,

83. le (±)-(2E,4E)-N-(2-chloro-4-fluorophényl)-5-[c-2-(3,4-dichlorophényl)-r-1-fluorocyclopropyl]penta-2,4-diénamide,

84. le (±)-(2E,4E)-N-(2-méthylphényl)-5-[c-2-(4-bromophényl)-r-1-fluorocyclopropyl]penta-2,4-diénamide,

85. le (±)-(2E,4E)-N-(2-chlorophényl)-5-[c-2-(4-bromophényl)-r-1-fluorocyclopropyl]penta-2,4-diénamide,

86. le (±)-(2E,4E)-N-(2-fluorométhylphényl)-5-[c-2-(3,4-dibromophényl)-r-1-fluorocyclopropyl]penta-2,4-diénamide,

87. le (±)-(2E,4E)-N-(2-hydroxyphényl)-5-[c-2-(3,4-dibromophényl)-r-1-fluorocyclopropyl]penta-2,4-diénamide,

88. le (+)-(2E,4E)-N-(2-méthylphényl)-5-[(1S,2R)-2-(3,4-dibromophényl)-1-fluorocyclopropyl]penta-2,4-diénamide,

89. le (-)-(2E,4E)-N-(2-méthylphényl)-5-[(1R,2S)-2-(3,4-dibromophényl)-1-fluorocyclopropyl]penta-2,4-diénamide,

90. le (±)-(2E,4E)-N-(2-méthylphényl)-5-[r-1-chloro-c-2-(3,4-dichlorophényl)cyclopropyl]-3-méthylpenta-2,4-diénamide,

91. le (±)-(2E,4E)-N-(2-chlorophényl)-5-[r-1-chloro-c-2-(3,4-dibromophényl)cyclopropyl]penta-2,4-diénamide,

92. le (±)-(2E,4E)-N-(2-méthylphényl)-5-[r-1-chloro-c-2-(3,4-dibromophényl)cyclopropyl]penta-2,4-diénamide,

93. le (±)-(2E,4E)-N-(2-méthylphényl)-5-[r-1-chloro-c-2-(3,4-dibromophényl)cyclopropyl]-3-méthylpenta-2,4-diénamide,

94. le (±)-(2E/Z,4E)-N-(2-méthylphényl)-5-[r-1-chloro-c-2-(3,4-dibromophényl)cyclopropyl]-3-méthylpenta-2,4-diénamide ,

95. le (±)-(2E/Z,4E)-N-(2-chlorophényl)-5-[r-1-chloro-c-(3,4-dibromophényl)cyclopropyl]-3-méthylpenta-2,4-diénamide (2E:2Z = 2:1),

96. le (±)-(2Z,4E)-N-(2-méthylphényl)-5-[c-2-(3,4-dibromophényl)-r-1-fluorocyclopropyl]-2-fluoro-3-méthylpenta-2,4-diénamide,

97. le (±)-(2Z,4E)-N-(2-chlorophényl)-5-[c-2-(3,4-dibromophényl)-r-1-fluorocyclopropyl]-2-fluoro-3-méthylpenta-2,4-diénamide,

98. le (±)-(2Z,4E)-N-(2-méthylphényl)-5-[c-2-(3,4-dichlorophényl)-r-1-fluorocyclopropyl]-2-fluoropenta-2,4-diénamide,

99. le (±)-(2Z,4E)-N-(2-chlorophényl)-5-[c-2-(3,4-dichlorophényl)-r-1-fluorocyclopropyl]-2-fluoropenta-2,4-

diénamide,

100. le (±)-(2Z,4E)-N-(2-chlorophényl)-5-[c-2-(3,4-dibromophényl)-r-1-fluorocyclopropyl]-2-fluoropenta-2,4-diénamide,

101. le (±)-(2Z,4E)-N-(2-méthylphényl)-5-[c-2-(3,4-dibromophényl)-r-1-fluorocyclopropyl]-2-fluoropenta-2,4-diénamide,

102. le (±)-(2E,4E)-N-(2-méthylphényl)-5-[trans-2-(3,4-dibromophényl)cyclopropyl]-3-méthylpenta-2,4-diénamide,

103. le (±)-(2E,4E)-N-(2-méthylphényl)-5-[trans-2-(3,4-dibromophényl)cyclopropyl]penta-2,4-diénamide,

104. le (-)-(2E,4E)-N-(2-méthylphényl)-5-[(1R,2S)-trans-2-(3,4-dibromophényl)cyclopropyl]-3-méthylpenta-2,4-diénamide,

105. le (+)-(2E,4E)-N-(2-méthylphényl)-5-[(1S,2R)-trans-2-(3,4-dibromophényl)cyclopropyl]-3-méthylpenta-2,4-diénamide,

106. le (±)-(2E,4E)-N-phényl-5-[c-2-(3,4-dibromophényl)-r-1-fluorocyclopropyl]penta-2,4-diènethioamide,

111. le (±)-(2E,4E)-N-méthyl-N-phényl-5-[c-2-(3,4-dibromophényl)-r-1-fluorocyclopropyl]penta-2,4-diénamide,

112. le (±)-(2E,4E)-N-isopropyl-N-(2-méthylphényl)-5-[c-2-(3,4-dibromophényl)-r-1-fluorocyclopropyl]penta-2,4-diénamide,

113. le (±)-(2E,4E)-N-isobutyl-N-(2-méthylphényl)-5-[c-2-(3,4-dibromophényl)-r-1-fluorocyclopropyl]penta-2,4-diénamide,

114. le (±)-(2E,4E)-N-isopropyl-N-phényl-5-[c-2-(3,4-dibromophényl)-r-1-fluorocyclopropyl]penta-2,4-diénamide,

115. le (±)-(2E,4E)-N-méthyl-N-(2-méthylphényl)-5-[c-2-(3,4-dibromophényl)-r-1-fluorocyclopropyl]-3-méthylpenta-2,4-diénamide,

116. le (±)-(2E,4E)-N-méthyl-N-(2-méthylphényl)-5-[c-2-(3,4-dibromophényl)-r-1-fluorocyclopropyl]penta-2,4-diénamide,

117. le (±)-(2E,4E)-N-benzyl-N-(2-méthylphényl)-5-[c-2-(3,4-dibromophényl)-r-1-fluorocyclopropyl]penta-2,4-diénamide,

118. le (±)-(2E,4E)-N-(2-chlorophényl)-N-méthyl-5-[c-2-(3,4-dibromophényl)-r-1-fluorocyclopropyl]penta-2,4-diénamide,

119. le (±)-(2E,4E)-N-(2-chlorophényl)-N-méthyl-5-[c-2-(3,4-dibromophényl)-r-1-fluorocyclopropyl]-3-méthylpenta-2,4-diénamide,

120. le (±)-(2E,4E)-N-(2-méthylphényl)-N-carboéthoxy-5-[c-2-(3,4-dibromophényl)-r-1-fluorocyclopropyl]penta-2,4-diénamide.

TABLEAU I

| Composé n° | Réactant de Wittig | Aniline | E,E:E,Z a:b |
|---|---|---|---|
| 1 | 1 | Aniline | a |
| 2 | 2 | Aniline | a |
| 3 | 1 | 2-Chloroaniline | a |
| 4 | 2 | 2-Chloroaniline | 7:3 |
| 5 | 2 | 4-Chloroaniline | a |
| 6 | 2 | 4-Fluoroaniline | a |
| 7 | 2 | 4-Fluoroaniline | 95:5 |

| | | | |
|---|---|---|---|
| 8 | 2 | 2-Fluoroaniline | 6:4 |
| 9 | 2 | 3-Chloroaniline | a |
| 10 | 2 | 2-Méthoxyaniline | a |
| 11 | 2 | 2-Trifluorométhylaniline | a |
| 12 | 1 | 2-Fluoroaniline | a |
| 13 | 2 | 2-Méthylaniline | 3:1 |
| 14 | 2 | 2-Méthylaniline | a |
| 15 | 1 | 2,6-Dichloroaniline | a |
| 16 | 1 | 2,6-Difluoroaniline | a |
| 17 | 1 | 2-Bromoaniline | a |
| 18 | 1 | 2-Ethylaniline | a |
| 19 | 1 | 2-Méthylaniline | a |
| 20 | 1 | 2-Trifluorométhylaniline | a |
| 21 | 1 | 4-Fluoroaniline | a |
| 22 | 1 | 3-Fluoroaniline | a |
| 23 | 2 | 4-Méthylaniline | a |
| 24 | 2 | 3-Méthylaniline | a |
| 25 | 1 | 4-Méthylaniline | a |
| 26 | 1 | 3-Méthylaniline | a |
| 27 | 1 | 2-Fluoro-6-méthylaniline | a |
| 28 | 1 | 2,6-Diméthylaniline | a |
| 29 | 1 | 4-Méthoxyaniline | a |
| 30 | 1 | 2-Chloro-5-méthylaniline | a |

| | | | |
|---|---|---|---|
| 31 | 1 | 4-Fluoro-2-méthylaniline | a |
| 32 | 1 | 3-Fluoro-2-méthylaniline | a |
| 33 | 1 | 3-Chloro-2-méthylaniline | a |
| 34 | 1 | 2-Chloro-6-méthylaniline | a |
| 35 | 1 | 2,3-Dichloroaniline | a |
| 36 | 1 | 4-Trifluorométhylaniline | a |
| 37 | 1 | 3-Méthoxyaniline | a |
| 38 | 1 | 2,5-Diméthylaniline | a |
| 39 | 1 | 2-Méthylthioaniline | a |
| 40 | 1 | 2-Cyanoaniline | a |
| 41 | 1 | 2-Isopropylaniline | a |
| 42 | 1 | 5-Chloro-2-méthylaniline | a |
| 43 | 1 | 4-Fluoro-2-trifluorométhylaniline | a |
| 44 | 1 | 2,3-Diméthylaniline | a |
| 45 | 1 | 2-Nitroaniline | a |
| 46 | 1 | 3-Fluoro-6-méthylaniline | a |
| 47 | 1 | 3-Bromo-2-méthylaniline | a |
| 48 | 1 | 3-Iodo-6-méthylaniline | a |
| 49 | 1 | 2-Phénoxyaniline | a |
| 50 | 1 | 2-N,N-Diméthylaminoaniline | a |
| 51 | 1 | 2-Ethoxycarbonylaniline | a |
| 52 | 1 | 2,4-Difluoroaniline | a |
| 53 | 1 | 2-Acétylaniline | a |

| | | | |
|---|---|---|---|
| 54 | 1 | 2-Chloro-4-fluoroaniline | a |
| 55 | 1 | 2-Isopropénylaniline | a |
| 56 | 1 | 2-Iodoaniline | a |
| 57 | 1 | 2-Ethynylaniline * | a |
| 58 | 1 | 2-Formylaniline | a |
| 59 | 1 | 2-Trifluorométhoxyaniline | a |
| 60 | 1 | 2-Méthoxycarbonylaniline | a |
| 61 | 1 | 2-n-Propylaniline | a |
| 62 | 1 | 2-Fluorosulfonylaniline | a |
| 63 | 2 | 2-Chloroaniline | a |
| 64 | 1 | 2-Hydroxyméthylaniline | a |
| 65 | 1 | 1,2-Phénylènediamine | a |

* La 2-éthynylaniline est préparée suivant Arcadi et al., <u>Tet. Letts</u>, 30(19), 2581, 1989.

Réactant de Wittig 1 = 4-Phosphonocrotonate de triéthyle
2 = 3-Méthyl-4-phosphonocrotonate de triéthyle.

TABLEAU II

| Composé n° | Réactant de Wittig | Benzaldéhyde où R est | Aniline | E,E:E,Z a:b |
|---|---|---|---|---|
| 66 | 1 | 3,4-Dichloro | 2-Chloroaniline | a |
| 67 | 2 | 3,4-Dichloro | 2-Chloroaniline | 5:4 |
| 68 | 1 | 3,4-Dichloro | 2-Méthylaniline | a |
| 69 | 1 | 3,4,5-Trichloro | 2-Chloroaniline | a |

| | | | | |
|---|---|---|---|---|
| 70 | 1 | 3,4,5-Trichloro | 2-Méthylaniline | a |
| 71 | 2 | 3,4-Dichloro | 2-Méthylaniline | 1:1 |
| 72 | 1 | 4-Iodo | 2-Méthylaniline | a |
| 73 | 1 | 3-Chloro-4-iodo | 2-Méthylaniline | a |
| 74 | 1 | 4-Trifluoro-méthyle | 2-Méthylaniline | a |
| 75 | 1 | 4-Trifluoro-méthyle | 2-Chloroaniline | a |
| 76 | 2 | 4-Trifluoro-méthyle | 2-Méthylaniline | a |
| 77 | 2 | 4-Iodo | 2-Méthylaniline | a |
| 78 | 1 | 3,4-Dichloro | 4-Fluoro-2-méthylaniline | a |
| 79 | 1 | 3,4,5-Trichloro | 4-Fluoro-2-méthylaniline | a |
| 80 | 1 | 3,4,5-Trichloro | 2-Chloro-4-fluoroaniline | a |
| 81 | 1 | 3,4,5-Trichloro | 4-Fluoro-2-méthylaniline | a |
| 82 | 1 | 3,4-Dichloro | 4-Fluoro-2-méthylaniline | a |
| 83 | 1 | 3,4-Dichloro | 2-Chloro-4-fluoroaniline | a |
| 84 | 1 | 4-Bromo | 2-Méthylaniline | a |
| 85 | 1 | 4-Bromo | 2-Chloroaniline | a |
| 86-90 | Voir section expérimentale | | | |
| 91 | 1 | 3,4-Dibromo | 2-Chloroaniline | a |
| 92 | 1 | 3,4-Dibromo | 2-Méthylaniline | a |

| | | | | |
|---|---|---|---|---|
| 93 | 2 | 3,4-Dibromo | 2-Méthylaniline | a |
| 94 | 2 | 3,4-Dibromo | 2-Méthylaniline | 2:1 |
| 95 | 2 | 3,4-Dibromo | 2-Chloroaniline | 2:1 |
| 96-98 | Voir section expérimentale | | | |
| 99 | 3 | 3,4-Dichloro | 2-Chloroaniline | a |
| 100 | 3 | 3,4-Dibromo | 2-Méthylaniline | a |
| 101 | 3 | 3,4-Dibromo | 2-Chloroaniline | a |
| 102-110 | Voir section expérimentale | | | |
| 111 | 1 | 3,4-Dibromo | N-Méthylaniline | a |
| 112 | 1 | 3,4-Dibromo | N-Isopropyl-o-toluidine | a |
| 113 | 1 | 3,4-Dibromo | N-Isobutyl-aniline | a |
| 114 | 1 | 3,4-Dibromo | N-Isopropyl-aniline | a |
| 115 | 2 | 3,4-Dibromo | N-méthyl-o-toluidine | a |
| 116 | 1 | 3,4-Dibromo | N-méthyl-o-toluidine | a |
| 117 | 1 | 3,4-Dibromo | N-benzyl-o-toluidine | a |
| 118 | 1 | 3,4-Dibromo | N-méthyl-o-chloroaniline | a |
| 119 | 2 | 3,4-Dibromo | N-méthyl-o-chloroaniline | a |

Réactant de Wittig
1 = 4-Phosphonocrotonate de triéthyle.
2 = 3-Méthyl-4-phosphonocrotonate de triéthyle.
3 = 2-Fluoro-4-phosphocrotonate de triéthyle.

RESULTATS BIOLOGIQUES.

Les exemples suivants illustrent non limitativement l'activité pesticide des composés de formule (I).

EXEMPLE A - EPREUVES PAR PULVERISATION.

On fait l'épreuve de l'activité des composés de l'invention en dissolvant ceux-ci dans de l'acétone (5%) et en les diluant avec de l'eau - "Synperonic" (94,5% - 0,5%) pour obtenir une émulsion aqueuse. On utilise l'émulsion ensuite pour traiter les insectes suivants sur lesquels on observe l'activité aux doses de pulvérisation suivantes.

Musca domestica.

On confine 20 femelles de Musca dans un cylindre en carton fermé par de la gaze aux deux extrémités. On pulvérise la solution contenant le composé sur les insectes ainsi enfermés et on évalue la mortalité après 48 heures à 25 °C.

Les composés suivants sont actifs à 1000 ppm sinon moins :
5, 7, 9.

Les composés suivants sont actifs à 200 ppm sinon moins :
1, 2, 3, 4, 6, 8.

Plutella xylostella.

On pulvérise la solution contenant le composé sur des disques taillés dans des feuilles de chou de Chine infestées par huit larves au deuxième stade du développement de Plutella. On évalue la mortalité après 2 jours à 25 °C.

Les composés suivants sont actifs à 1000 ppm sinon moins :
5, 7, 9, 10, 14, 17, 20, 21, 22, 98, 50, 55, 57, 81, 83, 100, 114.

Les composés suivants sont actifs à 200 ppm sinon moins :
1, 2, 3, 4, 6, 8, 11, 12, 13, 16, 19, 88, 89, 90, 93, 94, 95, 99, 101, 28, 66, 96, 97, 31, 52, 54, 58, 63, 65, 66, 68, 70, 74, 75, 76, 77, 78, 79, 80, 82, 84, 85, 111 à 119 sauf 114, 117.

Tetranychus urticae.

On pulvérise la solution contenant le composé sur des disques taillés dans des feuilles de haricot vert infestées. On évalue la mortalité après 2 jours à 25 °C.

Les composés suivants sont actifs à 1000 ppm sinon moins :
6, 14, 16, 20, 28, 71, 96.

Spodoptera littoralis.

On pulvérise la solution à essayer contenant le composé sur des feuilles non infestées qu'on laisse sécher. On infeste celles-ci ensuite avec dix larves nouvellement écloses. On évalue la mortalité après 3 jours.

Les composés suivants sont actifs à 1000 ppm sinon moins :
5, 7, 9, 10, 14, 22, 98, 32, 39, 45, 53, 55, 62, 75, 81, 83, 114.

Les composés suivants sont actifs à 200 ppm sinon moins :
1, 2, 3, 4, 6, 8, 11, 12, 13, 16, 17, 19, 20, 21, 28, 66, 96, 97, 31, 34, 44, 46, 50, 52, 54, 58, 63, 64, 66, 67, 68, 69, 70, 71, 72, 73, 74, 76, 77, 78, 79, 82, 84, 85, 86, 88, 89, 90, 91, 92, 93, 94, 95, 99, 100, 101, 111 à 119 sauf 114.

Myzus persicae.

On dépose dix adultes de Myzus sur un disque taillé dans une feuille de chou de Chine. Après 24 heures, on pulvérise sur le disque la solution contenant le composé. On évalue la mortalité après 2 jours à 25 °C.

Les composés suivants sont actifs à 1000 ppm sinon moins :
2, 4, 6, 8, 14, 13, 44, 68, 97, 115.

Les composés suivants sont actifs à 200 ppm sinon moins :
11, 71, 67, 96.

Diabrotica undecimpunctata.

On pulvérise la solution contenant le composé sur un papier filtre et un morceau d'aliment artificiel. On infeste ceux-ci ensuite avec des larves au deuxième stade du développement qu'on y retient pendant 48 heures au terme desquelles on évalue la mortalité.

Les composés suivants sont actifs à 1000 ppm sinon moins :
2, 3, 6, 9, 14, 16, 19, 20, 28, 98, 31, 44, 49, 54, 58, 61, 66, 67, 69, 74, 75, 79, 82, 85, 93, 94, 99, 101, 112, 115, 116.

Les composés suivants sont actifs à 200 ppm sinon moins :
4, 11, 13, 96, 97, 46, 68, 71, 72, 73, 77, 84, 89, 92.

EXEMPLE B - EPREUVES D'APPLICATION TOPIQUE.

Blattella germanica.

On applique topiquement 0,5 µl d'une solution du composé dans de la butanone (avec ou sans pipéronylbutoxyde) sur des mâles de B. germinaca. On évalue la mortalité après 6 jours.

Les composés suivants sont actifs à 10 µg sinon moins (+ pipéronylbutoxyde) :
2, 4, 6, 8, 11, 13, 14, 96, 97, 58, 63, 67, 71, 76, 77, 84, 89, 93, 94, 99, 100, 111, 115, 119.

Musca domestica.

On applique 0,3 µl d'une solution du composé et de pipéronylbutoxyde dans de la butanone sur des femelles de mouche domestique (par lots de 20). On évalue la mortalité après 2 jours.

Les composés suivants sont actifs à 1,5 µg sinon moins (+ pipéronylbutoxyde) :
98, 34, 39, 54, 55, 74, 75, 83, 88, 99, 100, 113, 118, 119.

Les composés suivants sont actifs à 0,3 µg sinon moins (+ pipéronylbutoxyde) :
1, 2, 3, 4, 6, 8, 9, 11, 12, 13, 14, 17, 18, 19, 21, 22, 23, 24, 28, 66, 96, 97, 31, 32, 38, 46, 50, 52, 63, 66, 67, 68, 69, 70, 71, 72, 73, 76, 77, 78, 79, 81, 82, 84, 85, 86, 89, 90, 91, 92, 93, 94, 95, 101, 111, 112, 115, 116, 117.

Les composés suivants sont actifs à 10 µg sinon moins (+ pipéronylbutoxyde) :
2, 4, 6, 8, 11, 13, 14.

Musca domestica.

On applique 0,3 µl de solutions du composé et de pipéronylbutoxyde dans de la butanone sur des femelles de mouche domestique (par lots de 20). On évalue la mortalité après 2 jours.
1, 2, 3, 4, 6, 8, 9, 11, 12, 13, 14, 17, 18, 19, 21, 22, 23, 24.

TABLEAU III

Propriétés physicochimiques.

| Composé n° | P.F. °C | Spectre de masse $M^{+1}$ | Composé n° | P.F. °C | Spectre de masse $M^{+1}$ |
|---|---|---|---|---|---|
| 1 | 174-5 | 464 | 51 | 131,6 | 536 |
| 2 | 138 | 478 | 52 | 179 | 500 |
| 3 | 168-9 | 498 | 53 | 149,7 | 506 |
| 4 | 60 | 512 | 54 | 176,6 | 518 |
| 5 | 147 | 512 | 55 | 226,3 | 504 |
| 6 | 127 | 498 | 56 | 192,5 | 590 |
| 7 | 64 | 498 | 57 | 178,1 | 488 |
| 8 | 60 | 496 | 58 | 141,5 | 492 |
| 9 | 148 | 512 | 59 | 182,7 | 548 |
| 10 | 104 | 508 | 60 | 156 | 522 |
| 11 | 49 | 546 | 61 | 183,2 | 506 |
| 12 | 171-6 | 482 | 62 | > 300 | 546 |
| 13 | 132 | 492 | 63 | 102,7 | 512 |

| | | | | | |
|---|---|---|---|---|---|
| 14 | 144 | 492 | 64 | 183,9 | 494 |
| 15 | 199 | 532 | 65 | 165 | 479 |
| 16 | 189 | 500 | 66 | 166-7 | 410 |
| 17 | 177 | 542 | 67 | 45-6 | 424 |
| 18 | 193 | 492 | 68 | 185-6 | 390 |
| 19 | 186 | 478 | 69 | 176-7 | 444 |
| 20 | 188 | 532 | 70 | 192-3 | 424 |
| 21 | 181 | 482 | 71 | 127-8 | 404 |
| 22 | 189 | 482 | 72 | 183 | 448 |
| 23 | 154 | 492 | 73 | 189 | 482 |
| 24 | 127 | 492 | 74 | 193 | - |
| 25 | 189 | - | 75 | 171 | - |
| 26 | 182 | 478 | 76 | 140 | - |
| 27 | 193,5 | 496 | 77 | 139 | - |
| 28 | 185,6 | 492 | 78 | 182,5 | 408 |
| 29 | 206,2 | 494 | 79 | 185,1 | 442 |
| 30 | 194,5 | 512 | 80 | 186,1 | 462 |
| 31 | 192,2 | 496 | 81 | 186,8 | 442 |
| 32 | 192,1 | 496 | 82 | 183,3 | 408 |
| 33 | 193,9 | 512 | 83 | 178,5 | 428 |
| 34 | 188 | 512 | 84 | 165,6 | 400 |
| 35 | 169,4 | 532 | 85 | 139-44 | 420 |
| 36 | 194,1 | 532 | 86 | 163-5 | 566 |

| | | | | | |
|---|---|---|---|---|---|
| 37 | 155,4 | 494 | 87 | 167,7 | 482 |
| 38 | 192,8 | 492 | 88 | 185-6 | - |
| 39 | 156,1 | 510 | 89 | 174,6 | - |
| 40 | 179,3 | 489 | 90 | 170,6 | - |
| 41 | 178 | 506 | 91 | 140-1 | 514 |
| 42 | 197,5 | 512 | 92 | 170-1 | 494 |
| 43 | 165,9 | 550 | 93 | 153-4 | 508 |
| 44 | 192,5 | 492 | 94 | 140-1 | 508 |
| 45 | 157,4 | 509 | 95 | 142-3 | 528 |
| 46 | 180,3 | 496 | 96 | 104-5 | 510 |
| 47 | 271,6 | 556 | 97 | 128-9 | 530 |
| 48 | 191,3 | 604 | 98 | 152-3 | 408 |
| 49 | 153 | 556 | 99 | 109-10 | 428 |
| 50 | 133 | 507 | 100 | 154-5 | 496 |
| | | | 101 | 111-2 | 516 |
| | | | 102-111 | Voir section expérimentale | |
| | | | 112 | 129-30 | 520 |
| | | | 113 | huile | 534 |
| | | | 114 | 135-7 | 506 |
| | | | 115 | 135 | 492 |
| | | | 116 | huile | 506 |
| | | | 117 | 51-3 | 568 |
| | | | 118 | 126-8 | 512 |

| | | | | | |
|---|---|---|---|---|---|
| | | | 119 | huile | 526 |

TABLEAU IV

| Composé n° | Résultats de $^1$H RMN |
|---|---|
| 1 | 1,7 (1H, m); 2,1 (1H, m); 2,6 (1H, m); 6,2 à 6,6 (3H, m); 7,1 (1H, m); 7,2 à 7,0 (8H, m); 10,1 (1H, s). |
| 2 | 1,5 (1H, m); 1,8 (1H, m); 2,3 (1H, m); 2,4 (3H, s); 5,9 (2H, m); 6,4 (1H, d); 7 à 7,9 (9H, m). |
| 3 | 1,6 (1H, m); 1,8 (1H, m); 2,3 (1H, m); 5,9 (1H, dd); 6,1 (1H, d); 6,5 (1H, dd); 7,4 (1H, dd); 7 à 8,5 (8H, m). |
| 4 | 1,7 (2H, m); 2,1 et 2,4 (3H, s); 2,3 (1H, m); 5,9 à 6,1 (3H, m); 6,5 et 7,9 (1H, d), 7 à 8,5 (7H, m). |
| 5 | 1,5 (1H, m); 1,8 (1H, m); 2,3 (1H, m); 2,4 (3H, s); 5,9 (2H, m); 6,4 (1H, d); 7,1 (1H, dd); 7,2 à 7,6 (7H, m). |
| 6 | 1,5 (1H, m); 1,8 (1H, m), 2,3 (1H, m); 2,4 (3H, s), 5,9 (2H, m); 6,4 (1H, d); 7 à 7,6 (8H, m). |
| 7 | 1,4 à 1,8 (2H, m); 1,9 (3H, s); 2,2 (1H, m); 5,7 (1H, s); 6,0 (1H, dd); 6,9 à 8 (9H, m). |
| 8 | 1,5 à 1,9 (2H, m); 2,1 et 2,4 (3H, s); 2,3 (1H, m); 5,8 à 6,2 (3H, m); 6,4 et 7,9 (1H, d); 7 à 8,5 (7H, m). |
| 9 | 1,5 (1H, m); 1,8 (1H, m); 2,3 (1H, m); 2,4 (3H, m); 5,9 (2H, m); 6,4 (1H, d); 7 à 7,8 (8H, m). |

| | |
|---|---|
| 10 | 1,5 (1H, m); 1,8 (1H, m); 2,3 (1H, m); 2,4 (3H, s); 3,9 (3H, s); 5,9 (2H, m); 6,4 (1H, d); 6,9 à 7,6 (6H, m); 7,9 (1H, s); 8,5 (1H, d). |
| 11 | 1,6 (1H, m); 1,8 (1H, m); 2,3 (1H, m); 2,4 (3H, s); 5,9 (2H, m); 6,4 (1H, d); 7 à 8,4 (8H, m). |
| 12 | 1,3 (1H, m); 1,5 (1H, m); 2,0 (1H, m); 5,6 (1H, dd); 6,1 (2H, m); 7,0 (1H, dd); 6,7 à 7,3 (6H, m); 7,9 (1H, m); 9,0 (1H, s). |
| 13 | 1,6 (1H, m); 1,8 (1H, m); 2,1 et 2,4 (3H, s); 2,25 (3H, s); 2,3 (1H, m); 5,8 à 6,5 (3H, m); 6,9 à 8 (8H, m). |
| 14 | 1,6 (1H, m); 1,8 (1H, m), 2,25 (3H, s); 2,3 (1H, m), 2,4 (3H, s); 5,9 (2H, m); 6,4 (1H, d); 6,9 à 8 (8H, m). |
| 15 | 1,75 (1H, m); 2,2 (1H, m); 2,7 (1H, m); 6,4 (2H, m); 6,6 (1H, m); 7,2 à 7,9 (7H, m); 10,1 (1H, s). |
| 16 | 1,1 (1H, m); 1,6 (1H, m); 1,8 (1H, m); 6,0 (1H, dd), 6,2 à 6,5 (2H, m); 6,9 à 7,9 (7H, m); 9,6 (1H, s). |
| 17 | 1,05 (1H, m); 1,25 (1H, m); 1,8 (1H, m); 5,3 à 5,5 (1H, m); 5,7 à 6 (2H, m), 6,4 à 7,4 (8H, m); 8,5 (1H, s). |
| 18 | 1,1 (3H, t); 1,6 (1H, m); 1,75 (1H, m); 2,4 à 2,7 (3H, m); 6,0 (1H, dd); 6,2 à 6,5 (2H, m); 7 à 9,3 (9H, m). |
| 19 | 1,8 (1H, m); 2,2 (1H, m); 2,3 (3H, s); 2,7 (1H, m); 6,2 à 6,6 (3H, m); 7,1 à 7,4 (5H, m); 7,6 (1H, d); 7,8 (2H, m); 9,5 (1H, s). |
| 20 | 1,8 (1H, m); 2,2 (1H, m); 2,7 (1H, m); 6,3 à 6,7 (3H, m); 7,3 (2H, dd); 7,5 à 7,9 (6H, m); 9,8 (1H, s). |
| 21 | 1,45 (1H, m); 1,85 (1H, m); 2,4 (1H, m); 5,9 à 6,4 (3H, m); 6,9 à 7,2 (4H, m); 7,4 à 7,6 (4H, m); 9,7 (1H, s). |

| | |
|---|---|
| 22 | 1,5 (1H, m); 1,9 (1H, m); 2,4 (1H, m); 6,0 à 6,4 (3H, m); 6,7 (1H, m); 7 à 7,6 (7H, m); 9,5 (1H, s). |
| 23 | 1,6 (1H, m); 1,8 (1H, m); 2,3 (1H, m), 2,35 (3H, s); 2,4 (3H, s); 5,9 (2H, m); 6,4 (1H, d); 7 à 7,6 (8H, m). |
| 24 | 1,5 (1H, m); 1,9 (1H, m); 2,1 (3H, s); 2,15 (3H, s); 2,4 (1H, m); 5,7 à 6,2 (3H, m); 6,65 (1H, d); 6,9 à 7,5 (6H, m); 9,75 (1H, s). |
| 25 | 1,45 (1H, m); 1,75 (1H, m); 2,05 (3H, s); 2,4 (1H, m); 5,9 à 6,3 (3H, m); 6,8 à 7,5 (8H, m), 9,85 (1H, s). |
| 26 | 1,65 (1H, m); 2,1 (1H, m); 2,25 (3H, s); 2,65 (1H, m); 6,2 à 6,4 (2H, m); 6,5 (1H, dd); 6,9 (1H, d); 7,1 à 7,8 (7H, m); 10,05 (1H, s). |
| 27 | 1,6 (1H, m); 2,05 (1H, m); 2,1 (3H, s); 2,6 (1H, m); 6,1 à 6,5 (3H, m); 7,0 (1H, m); 7,15 à 7,75 (6H, m); 9,6 (1H, s). |
| 28 | 1,65 (1H, m); 2,05 (1H, m); 2,1 (6H, s); 2,6 (1H, m); 6,1 à 6,55 (3H, m); 7 à 7,7 (7H, m); 9,4 (1H, s). |
| 29 | 1,45 (1H, m); 1,9 (1H, m); 2,4 (1H, m); 3,5 (3H, s); 5,9 à 6,3 (3H, m); 6,6 à 7,5 (8H, m); 9,8 (1H, s). |
| 30 | 1,4 (1H, m); 1,9 (1H, m); 2,1 (3H, s); 2,4 (1H, m); 6 à 6,3 (3H, m); 6,75 à 7,5 (7H, m); 9,4 (1H, s). |
| 31 | 1,55 (1H, m); 1,8 (1H, m); 2,15 (3H, s), 2,45 (1H, m); 5,9 à 6,4 (3H, m); 6,7 à 7,5 (7H, m); 9,2 (1H, s). |
| 32 | 1,55 (1H, m); 2,0 (1H, m); 2,1 (3H, s); 2,6 (1H, m); 6,1 à 6,55 (3H, m); 6,9 à 7,7 (7H, m); 9,55 (1H, m). |
| 33 | 1,65 (1H, m); 2,05 (1H, m); 2,2 (3H, s); 2,6 (1H, m); 6,1 à 6,5 (3H, m); 7,1 à 7,7 (7H, m); 9,6 (1H, s). |

| | |
|---|---|
| 34 | 1,6 (1H, m); 2,0 (1H, m); 2,15 (3H, s); 2,55 (1H, m); 6,1 à 6,55 (3H, m); 7,1 à 7,7 (7H, m); 9,6 (1H, s). |
| 35 | 1,65 (1H, m); 2,05 (1H, m); 2,6 (1H, m); 6,1 à 6,5 (3H, m); 7,1 à 7,8 (7H, m); 9,7 (1H, s). |
| 36 | 1,2 (1H, m); 1,8 (1H, m); 2,3 (1H, m), 5,8 à 6,1 (3H, m); 6,5 (1H, dd); 7 à 7,8 (8H, m). |
| 37 | 1,65 (1H, m); 2,05 (1H, m); 2,6 (1H, m); 3,2 (3H, s); 6,1 à 6,5 (3H, m); 6,6 (1H, dd); 7,1 à 7,7 (7H, m); 10 (1H, s). |
| 38 | 1,7 (1H, m); 2,05 (1H, m); 2,2 (3H, s); 2,3 (3H, s); 2,6 (1H, m); 6,1 à 6,5 (3H, m), 6,8 à 7,7 (7H, m); 9,3 (1H, s). |
| 39 | 1,65 (1H, m); 2,0 (1H, m); 2,4 (3H, s); 2,6 (1H, m); 6,1 à 6,5 (3H, m); 7,1 à 7,7 (8H, m); 9,4 (1H, s). |
| 40 | 1,7 (1H, m); 2,1 (1H, m); 2,6 (1H, m); 6,2 à 6,6 (3H, m); 7,2 à 7,9 (8H, m), 10,25 (1H, s). |
| 41 | 1,05 (6H, d); 1,6 (1H, m); 2,05 (1H, m); 2,6 (1H, m); 3,25 (1H, m); 6,1 à 6,5 (3H, m); 7,1 à 7,75 (8H, m); 9,4 (1H, s). |
| 42 | 1,65 (1H, m); 2,05 (1H, m); 2,2 (3H, s); 2,65 (1H, m); 6,15 à 6,5 (3H, m); 7,1 à 7,8 (7H, m); 9,4 (1H, s). |
| 43 | 1,6 (1H, m), 2,0 (1H, m); 2,6 (1H, m); 6,2 à 6,6 (3H, m); 7,1 à 9,0 (7H, m); 9,7 (1H, s). |
| 44 | 1,75 (1H, m); 2,1 (1H, m); 2,15 (3H, s); 2,35 (3H, s); 2,65 (1H, m); 6,2 à 6,6 (3H, m); 7,0 à 7,8 (7H, m); 9,5 (1H, s). |
| 45 | 1,65 (1H, m); 2,05 (1H, m); 2,55 (1H, m); 6,1 à 6,6 (3H, m); 7,1 à 8 (8H, m); 10,3 (1H, s). |
| 46 | 1,6 (1H, m); 2,0 (1H, m); 2,2 (3H, m); 2,6 (1H, m); 6,1 à 6,6 (3H, m); 6,8 à 7,8 (7H, m); 9,3 (1H, s). |

| | |
|---|---|
| 47 | 1,65 (1H, m); 2,05 (1H, m); 2,2 (3H, s); 2,55 (1H, m); 6,1 à 6,5 (3H, m); 7,1 à 7,7 (7H, m); 9,55 (1H, s). |
| 48 | 1,65 (1H, m); 2,05 (1H, m); 2,2 (3H, s); 2,6 (1H, m); 6,15 à 6,5 (3H, m); 6,95 à 8 (7H, m); 9,4 (1H, s). |
| 49 | 1,6 (1H, m); 2,0 (1H, m); 2,55 (1H, m); 6,1 à 6,5 (3H, m); 6,8 à 8,2 (13H, m); 9,5 (1H, s). |
| 50 | 1,7 (1H, m); 2,05 (1H, m); 2,55 (1H, m); 2,6 (6H, s); 6,1 à 6,6 (3H, m); 6,9 à 8,1 (8H, m); 9,15 (1H, s). |
| 51 | 1,3 (3H, t); 1,65 (1H, m); 2,05 (1H, m); 2,6 (1H, m); 4,3 (2H, q); 6,2 à 6,6 (3H, m); 7,1 à 8,4 (8H, m); 10,7 (1H, s). |
| 52 | 1,6 (1H, m); 2,1 (1H, m); 2,6 (1H, m); 6,15 à 6,6 (3H, m); 7 à 8 (7H, m); 9,85 (1H, s). |
| 53 | 1,7 (1H, m); 2,1 (1H, m); 2,55 (1H, m); 2,6 (3H, s); 6,2 à 6,6 (3H, m); 7,2 à 8,45 (8H, m); 11,4 (1H, s). |
| 54 | 1,5 (1H, m); 1,85 (1H, m); 2,35 (1H, m); 5,95 à 6,3 (3H, m); 6,9 à 7,6 (7H, m); 9,4 (1H, s). |
| 55 | 1,6 (1H, m); 1,95 (3H, s); 2,1 (1H, m); 2,6 (1H, m); 4,9 (1H, s); 5,2 (1H, s); 6,15 à 6,5 (3H, m); 7,1 à 7,7 (8H, m); 9,2 (1H, s). |
| 56 | 1,7 (1H, m); 2,05 (1H, m); 2,6 (1H, m); 6,2 à 6,55 (3H, m); 7 (1H, dd); 7,2 à 7,75 (6H, m); 7,9 (1H, dd); 9,45 (1H, s). |
| 57 | 1,65 (1H, m); 2,05 à 2,55 (1H, m); 4,5 (1H, s); 6,2 à 6,5 (3H, m); 7,05 à 7,9 (8H, m); 9,35 (1H). |
| 58 | 1,7 (1H, m); 2,1 (1H, m); 2,6 (1H, m); 6,2 à 6,6 (3H, m); 7,2 à 8,3 (8H, m); 10,0 (1H, s); 10,95 (1H, s). |

| | |
|---|---|
| 59 | 1,65 (1H, m); 2,05 (1H, m); 2,55 (1H, m); 6,2 à 6,5 (3H, m); 7,1 à 8 (8H, m); 9,8 (1H). |
| 60 | 1,7 (1H, m); 2,1 (1H, m); 2,6 (1H, m); 3,9 (3H); 6,15 à 6,6 (3H, m); 7,1 à 8,4 (8H, m); 10,8 (1H, s). |
| 61 | 0,9 (3H, t); 1,5 (2H, m); 1,65 (1H, m); 2,05 (1H); 2,55 (3H, m); 6,1 à 6,53 (3H, m); 7,1 à 7,75 (8H, m), 9,4 (1H, s). |
| 62 | 1,7 (1H, m); 2,05 (1H, m); 2,06 (1H, m); 6,2 à 6,6 (3H, m); 7,2 à 8,2 (8H, m); 9,9 (1H, s). |
| 63 | 1,55 (1H, m); 1,8 (1H, m); 2,3 (1H, m); 2,4 (3H, s); 5,8 à 6 (2H, m); 6,45 (1H, d); 7,0 à 7,7 (7H, m); 9,45 (1H, d). |
| 64 | 1,65 (1H, m), 2,1 (1H, m); 2,55 (1H, m); 4,5 (2H, s); 6,1 à 6,5 (3H, m); 7,1 à 7,7 (8H, m); 9,5 (1H, s). |
| 65 | 1,65 (1H, m); 2,0 (1H, m); 2,55 (1H, m); 6,1 à 6,5 (3H, m); 6,8 à 7,7 (8H, m); 9,9 (1H, m). |
| 66 | 1,55 (1H, m); 1,8 (1H, m); 2,35 (1H, m); 5,8 à 6,2 (2H, m); 6,5 (1H, dd); 7 à 7,75 (8H, m); 8,5 (1H, d). |
| 67 | 1,65 (1H, m); 2,05 (1H, m); 2 et 2,3 (3H, s); 2,6 (1H, m); 6 à 6,4 (3H, m); 7,1 à 7,95 (7H, m), 9,45 (1H, s). |
| 68 | 1,7 (1H, m); 2,05 (1H, m); 2,2 (3H, s); 2,6 (1H, m); 6,15 à 6,55 (3H, m); 7 à 7,7 (8H, m); 9,4 (1H, s). |
| 69 | 1,7 (1H, m); 2,15 (1H, m); 2,55 (1H, m); 6,15 à 6,55 (3H, m); 7,1 à 7,85 (7H, m), 9,6 (1H, s). |
| 70 | 1,8 (1H, m); 2,2 (1H, m); 2,25 (3H, s); 2,65 (1H, m); 6,1 à 6,55 (3H, m); 7 à 7,65 (7H, m); 9,4 (1H, s). |

| | |
|---|---|
| 71 | 1,65 (1H, m); 2,05 (1H, m); 2,0 et 2,3 (3H, s); 2,2 (3H, s); 2,6 (1H, m); 6 à 6,4 (3H, m); 7 à 8 (7H, m); 9,3 (1H, s). |
| 72 | 1,5 (1H, m); 1,8 (1H, m); 2,25 (3H, s); 2,35 (1H, m); 5,8 à 6,1 (2H, m); 6,4 à 6,6 (1H, t); 6,95 à 8 (10H, m). |
| 73 | 1,45 (1H, m); 1,85 (1H, m); 2,0 (3H, s); 2,35 (1H, m); 6,0 à 6,3 (3H, m); 6,75 à 7,7 (8H, m); 9,2 (1H, s). |
| 74 | 1,55 (1H, m); 1,85 (1H, m); 2,3 (3H, s): 2,4 (1H, m); 5,8 à 6,1 (2H, m); 6,5 (1H, t); 7 à 7,65 (9H, m); 7,95 (1H, s). |
| 75 | 1,6 (1H, m), 1,9 (1H, m); 2,45 (1H, m); 5,9 à 6,15 (2H, m); 6,5 (1H dd); 7 à 7,7 (9H); 8,5 (1H, d). |
| 76 | 1,6 (1H, m); 1,85 (1H, m); 2,25 (3H, s); 2,35 (3H, s); 2,4 (1H, m); 5,8 à 6 (2H, m); 6,45 (1H, d); 6,9 à 7,6 (8H, m); 7,95 (1H, s). |
| 77 | 1,5 (1H, m); 1,8 (1H, m); 2,25 (3H, s); 2,3 (1H, m); 2,35 (3H, s); 5,75 à 6 (2H, m); 6,4 (1H, d); 6,9 à 7,3 (6H, m); 7,65 (2H, d); 7,95 (1H, s). |
| 78 | 1,7 (1H, m); 2,1 (1H, m); 2,2 (3H, s); 2,6 (1H, m); 6,15 à 6,5 (3H, m); 6,8 à 7,6 (7H, m); 9,4 (1H, s). |
| 79 | 1,55 (1H, m); 2,0 (3H, s); 2,05 (1H, m); 2,45 (1H, m); 5,9 à 6,3 (3H, m); 6,7 (1H, td); 7 à 7,45 (5H, m), 9,2 (1H, s). |
| 80 | 1,7 (1H, m); 2,2 (1H, m); 2,65 (1H, m); 6,2 à 6,5 (3H, m); 7,15 à 7,8 (6H, m); 9,7 (1H, s). |
| 81 | 1,65 (1H, m); 2,1 (3H, s); 2,2 (1H, m); 2,6 (1H, m); 6 à 6,5 (3H, m); 6,9 à 7,1 (6H, m); 9,45 (1H, s). |
| 82 | 1,6 (1H, m); 2,1 (1H, m); 2,2 (3H, s); 2,55 (1H, m); 6,1 à 6,5 (3H, m); 6,9 à 7,6 (7H, m); 9,45 (1H, s). |

| | |
|---|---|
| 83 | 1,65 (1H, m); 2,1 (1H, m); 2,6 (1H, m); 6,1 à 6,5 (3H, m); 7,15 à 7,8 (7H, m); 9,7 (1H, s). |
| 84 | 1,65 (1H, m); 2,0 (1H, m); 2,2 (3H, s); 2,55 (1H, m); 6,1 à 6,5 (3H, m); 7,0 à 7,6 (9H, m); 9,4 (1H, s). |
| 85 | 1,65 (1H, m); 1,95 (1H, m); 2,55 (1H, m); 6,2 à 6,5 (3H, m); 7,1 à 7,6 (8H, m); 7,8 (1H, d); 9,65 (1H, s). |
| 86 | 1,6 (1H, m); 2,1 (1H, m); 2,5 (1H, m); 5,4 (2H, d); 6,4 (3H, m); 7,4 (8H, m); 9,7 (1H, s). |
| 87 | 1,4 (1H, m); 1,9 (1H, m); 2,4 (1H, m); 6,1 (3H, m); 6,6 (3H, m); 7,1 (2H, m), 7,6 (3H, m); 9,3 (1H, s); 9,6 (1H, s). |
| 88 | Voir section expérimentale |
| 89 | |
| 90 | |
| 91 | 1,85 (1H, m); 2,05 (1H, m); 2,75 (1H, m); 6,2 à 6,6 (3H, m); 7,1 à 7,75 (7H, m); 7,8 (1H, d); 9,6 (1H, s). |
| 92 | 1,85 (1H, m); 2,05 (1H, m); 2,2 (3H, s); 2,8 (1H, m); 6,2 à 6,45 (2H, m); 6,55 (1H, t); 7 à 7,7 (8H, m); 9,35 (1H, s). |
| 93 | 1,85 (1H, m); 2,05 (1H, m); 2,2 (3H, s); 2,3 (3H, s); 2,8 (1H, m); 6 à 6,2 (2H, m); 6,4 (1H, d); 7,0 à 7,7 (7H, m); 9,25 (1H, s). |
| 94 | 1,8 (1H, m); 2,0 et 2,3 (3H, s); 2,05 (1H, m); 2,2 (3H, s); 2,8 (1H, m); 6,0 à 6,5 (3H, m); 7 à 8,1 (7H, m); 9,2 et 9,3 (1H, s). |
| 95 | 1,9 (1H, m); 2,0 et 2,3 (3H, s); 2,05 (1H, m); 2,8 (1H, m); 6,05 à 6,5 (3H, m); 7,1 à 8,1 (7H, m); 9,4 et 9,45 (1H, s). |
| 96 | Voir section expérimentale |
| 97 | |

| | |
|---|---|
| 98 | 1,65 (1H, m); 2,05 (1H, m); 2,2 (3H, s); 2,6 (1H, m); 6,2 à 6,85 (3H, m); 7,1 à 7,6 (7H, m); 9,8 (1H, s). |
| 99 | 1,7 (1H, m); 2,1 (1H, m); 2,6 (1H, m); 6,25 à 6,6 (2H, m); 6,8 (1H, dd); 7,25 à 7,6 (7H, m); 9,95 (1H, s). |
| 100 | 1,7 (1H, m); 2,1 (1H, m); 2,2 (3H, s); 2,6 (1H, m); 6,2 à 6,85 (3H, m); 7,1 à 7,7 (7H, m); 9,8 (1H, s). |
| 101 | 1,7 (1H, m); 2,1 (1H, m); 2,7 (1H, m); 6,2 à 6,9 (3H, m); 7,2 à 7,7 (7H, m); 10,0 (1H, s). |
| 102-111 | Voir section expérimentale |
| 112 | 0,8 (3H, d); 1,2 (3H, d); 1,4 (1H, m); 1,7 (1H, m); 2,1 (3H, s); 2,1 (1H, m); 4,8 (1H, m); 5,5 (1H, d); 5,8 (1H, m); 6,2 (1H, m); 6,9 à 7,6 (8H, m). |
| 113 | 0,9 (6H, m); 1,3 (1H, m); 1,6 (1H, m); 1,8 (1H, m); 2,1 (3H, s); 2,1 (1H, m); 3,0 (1H, m); 4,0 (1H, m); 5,6 (1H, d); 5,8 (1H, m); 6,2 (1H, m); 6,9 à 7,5 (8H, m). |
| 114 | 1,0 (6H, d); 1,4 (1H, m); 1,7 (1H, m); 2,1 (1H, m); 5,0 (1H, m); 5,5 (1H, d); 5,7 (1H, m); 6,1 (1H, m); 6,9 à 7,5 (9H, m). |
| 115 | 1,4 (1H, m); 1,7 (1H, m); 2,1 (3H, s); 2,1 (1H, m); 3,2 (3H, s); 5,6 (1H, d); 5,8 (1H, m); 6,2 (1H, m); 6,9 à 7,5 (8H, m). |
| 116 | 1,4 (1H, m), 1,7 (1H, m); 2,1 (3H, s); 2,1 (1H, m); 2,2 (3H, s); 3,1 (3H, s); 5,4 (1H, s); 5,7 (1H, m); 6,0 (1H, d); 6,9 à 7,5 (7H, m). |
| 117 | 1,5 (1H, m); 1,8 (1H, m); 2,0 (3H, s); 2,2 (1H, m); 4,5 (1H, d); 5,3 (1H, d); 5,7 (1H, d); 5,8 (1H, m); 6,3 (1H, m); 6,8 à 7,6 (13H, m). |
| 118 | 1,5 (1H, m); 1,8 (1H, m); 2,2 (1H, m); 3,3 (3H, s); 5,6 (1H, d); 5,8 (1H, m); 6,3 (1H, m); 6,9 à 7,6 (8H, m). |

| | |
|---|---|
| 119 | 1,5 (1H, m); 1,7 (1H, m); 2,1 (1H, m); 2,2 (3H, s); 3,2 (3H, m); 5,5 (1H, s); 5,8 (1H, m); 6,1 (1H, d); 6,9 à 7,6 (7H, m). |

Schéma 1. Préparation de composés de formule I.

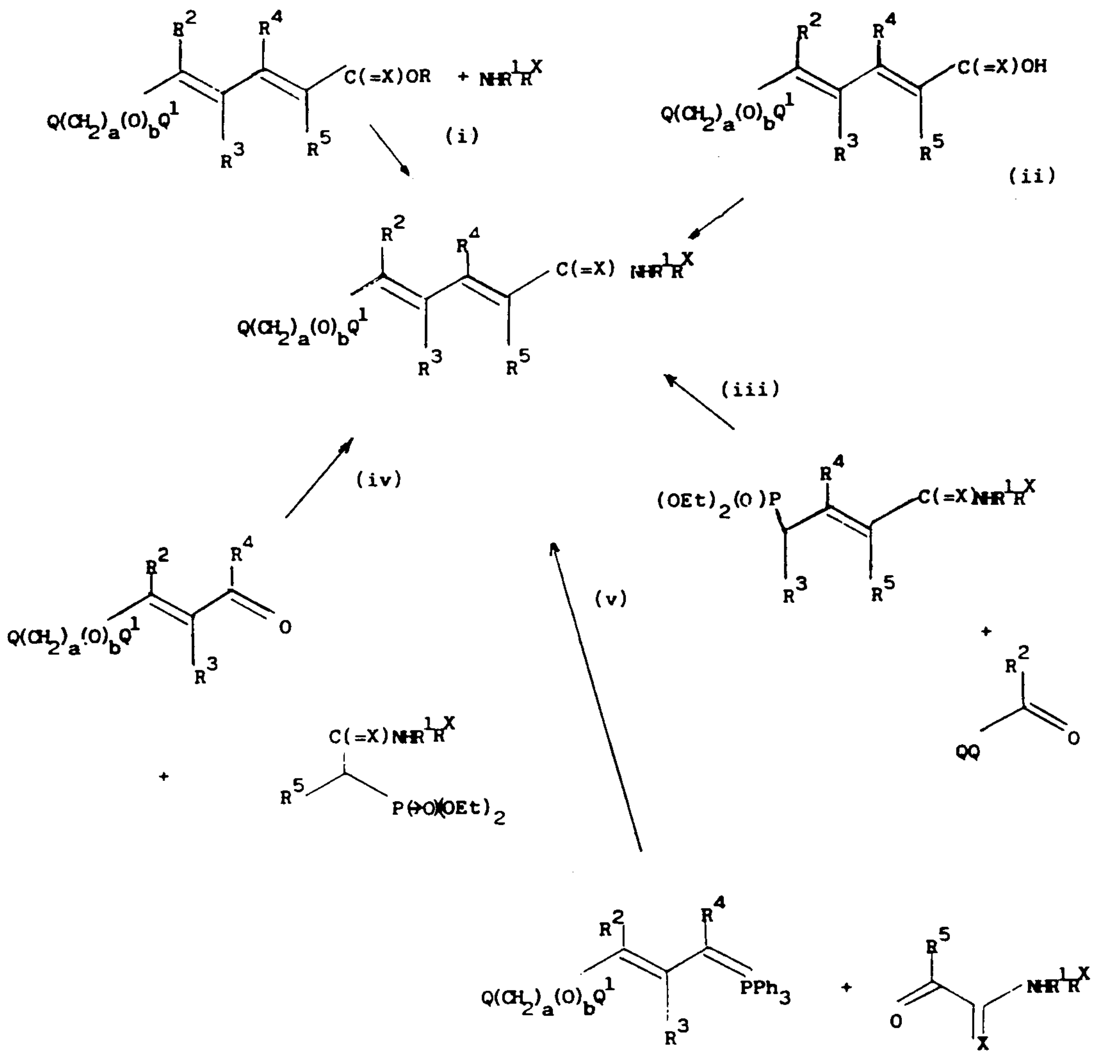


(i) Opération (a) $Z^1$ = OR, X = O
(ii) Opération (a) $Z^1$ = OH, X = O
(iii) Opération (b) X = O, S
(iv) Opération (b) X = O, S
(V) Opération (b) X = O, S

Schéma 2. Préparation d'esters intermédiaires.

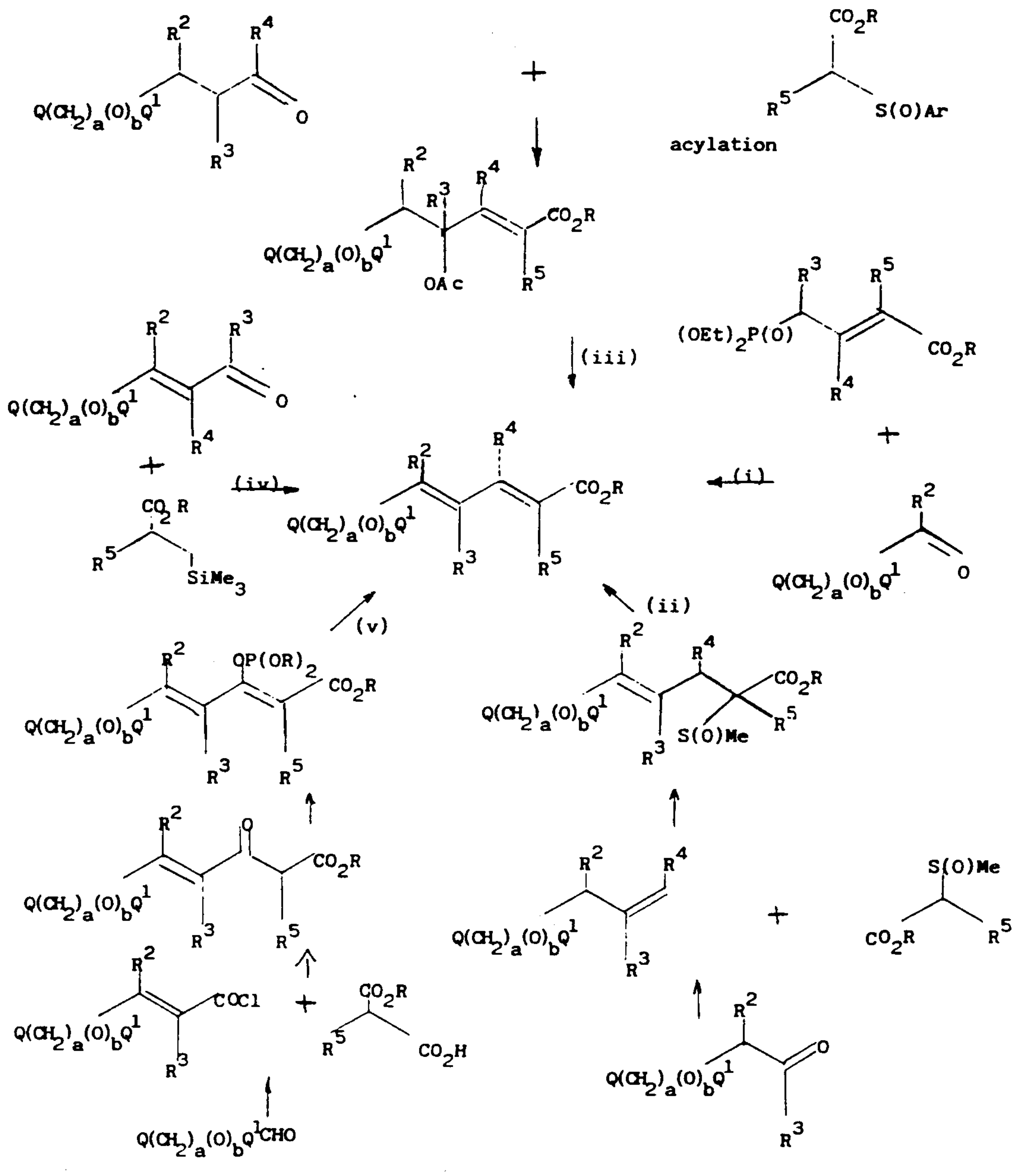


(i) à (v) désignent les opérations (i) à (v) décrites ci-dessus pour la préparation d'esters intermédiaires.

Schéma 3. Préparation d'alcools et aldéhydes intermédiaires.

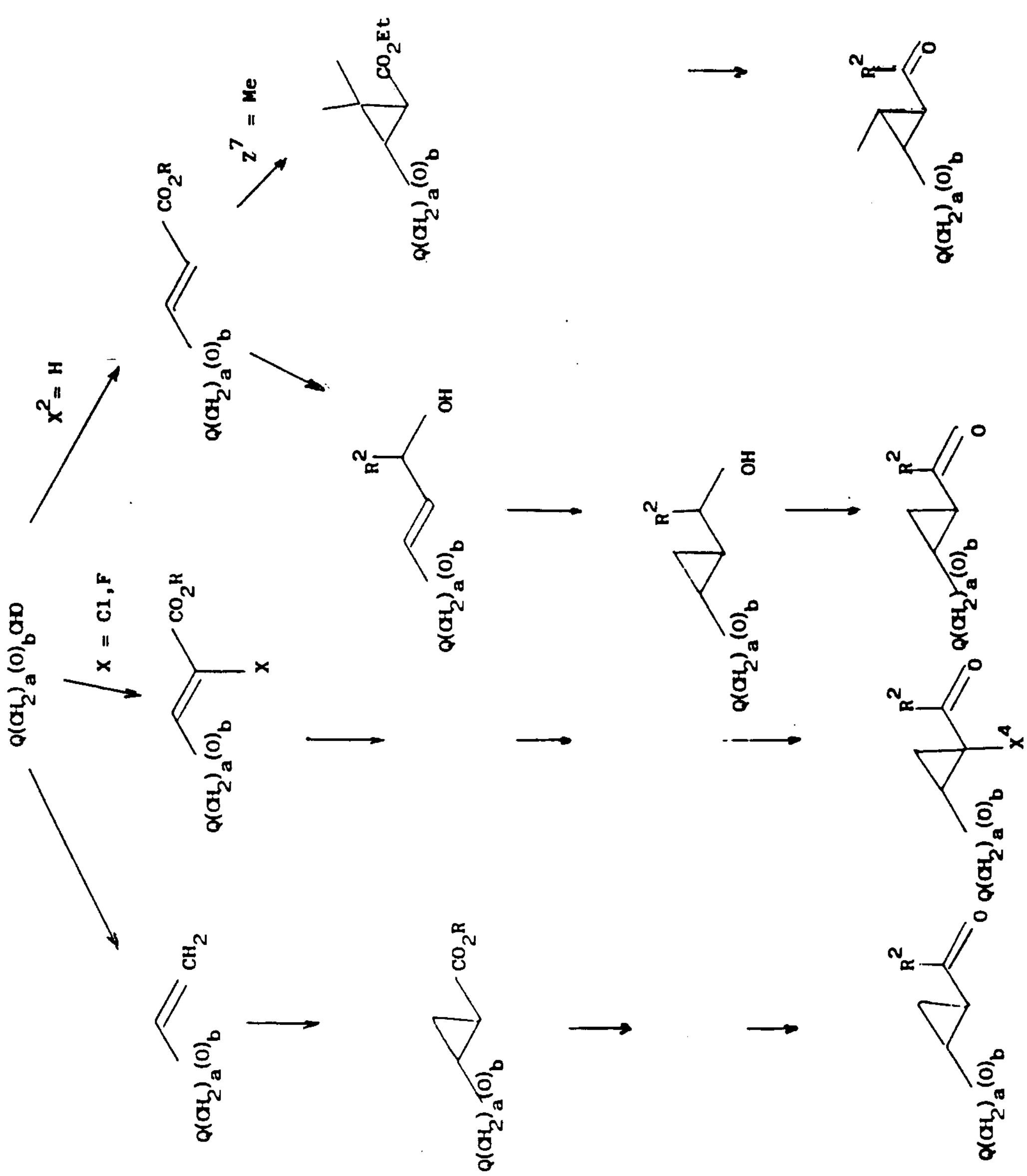


## Revendications

**Revendications pour les Etats contractants suivants : AT, BE, CH, DE, DK, FR, GB, IT, GR, LI, LU, NL, PT, SE**

**1.** Composé de formule (I) :

$$Q(CH_2)_a(O)_bQ^1CR^2=CR^3CR^4=CR^5CXNR^1R^X$$

ou un sel ou propesticide de celui-ci;
où

soit Q est un système cyclique aromatique monocyclique ou bicyclique condensé facultativement substitué dans lequel au moins un cycle est aromatique, soit Q est un radical dihalovinyle ou un radical $R^6$-C≡C-, où $R^6$ est $C_{1-4}$-alcoyle, tri-$C_{1-4}$-alcoylsilyle, halo ou hydrogène;

$Q^1$ est un cycle 1,2-cyclopropyle facultativement substitué par un ou plusieurs radicaux choisis parmi $C_{1-3}$-alcoyle, halo, $C_{1-3}$-haloalcoyle, $C_{2-3}$-alcynyle et cyano;

a est 0 ou 1;

b est 0 ou 1;

$R^2$, $R^3$, $R^4$ et $R^5$ sont identiques ou différents, au moins l'un étant hydrogène et les autres étant

choisis indépendamment parmi hydrogène, halo, $C_{1-4}$-alcoyle et $C_{1-4}$-haloalcoyle;

X est oxygène ou soufre;

$R^1$ est phényle facultativement substitué par 1 à 5 substituants choisis parmi :

a) $C_{1-4}$-alcoyle, $C_{1-4}$-alcoxy, phénoxy et méthylènedioxy, chacun facultativement substitué par 1 à 5 halo;

b) halo, cyano, nitro, formyle, $C_{1-5}$-acyle;

c) $C_{2-3}$-alcynyle, $C_{2-3}$alcényle, chacun facultativement substitué par 1 à 5 halo;

d) un radical $S(O)_nR^7$, où n est 0, 1 ou 2 et $R^7$ est $C_{1-4}$-alcoyle, halo ou $NR^8R^9$, où $R^8$ et $R^9$ sont choisis indépendamment parmi hydrogène, $C_{1-4}$-alcoyle et $C_{1-4}$-acyle, et

e) un radical $NR^8R^9$, où $R^8$ et $R^9$ sont tels que définis ci-dessus:

$R^X$ est hydrogène ou un radical $C_{1-8}$-alcoyle ou benzyle facultativement substitué.

**2.** Composé propesticide de formule (I) suivant la revendication 1, où $R^X$ est remplacé par $R^{XI}$, et $R^{XI}$ est choisi parmi :

(A) $$\overset{|}{}(D)_a-Y = A$$

où

A est O ou S, Y est phosphore ou carbone, D est hydrogène, $C_{1-4}$-alcoyle, $C_{1-4}$-alcoxy, $C_{1-5}$-acyle ou aryle ou bien $CO_2D^1$, où $D^1$ est $C_{1-4}$-alcoyle ou aryle et a est 1 ou 2;

(B) $-S(O)_bD^2$

où

b est 0, 1 ou 2 et soit (i) $D^2$ est $C_{1-4}$-alcoyle, aryle, aryloxy ou $C_{1-4}$-alcoxy, dans lequel un cycle aryle peut être substitué par un ou plusieurs radicaux halo, nitro, cyano, $C_{1-4}$-alcoyle et $C_{1-4}$-alcoxy dont chacun à son tour est facultativement substitué par un ou plusieurs halogènes, soit (ii) $D^2$ est un radical $ND^3D^4$, où soit $D^3$ est $-COD^5$, où $D^5$ est hydrogène, fluor ou $C_{1-4}$-alcoyle, soit $D^3$ est $C_{1-4}$-alcoyle substitué par $C_{1-5}$-acyle ou aryle, carboalcoxy ou cyano, soit $D^3$ est un radical $CO_2D^6$, où $D^6$ est $C_{1-4}$-alcoyle ou aryle et $D^4$ est hydrogène ou $C_{1-4}$-alcoyle.

**3.** composé de formule (I) suivant l'une quelconque des revendications 1 et 2, où Q est un radical phényle, pyridyle, thiényle ou naphthyle, chacun facultativement substitué par :

soit a) $C_{1-6}$-hydrocarbyle, $C_{1-6}$-alcoxy ou méthylènedioxy, chacun facultativement substitué par 1 à 5 halo;

soit b) halo, cyano, nitro, formyle ou $C_{1-5}$-acyle;

soit c) $C_{2-3}$-alcynyle ou $C_{2-3}$-alcényle, chacun facultativement substitué par 1 à 5 halo;

soit d) un radical $S(O)_nR^7$, où n est 0, 1 ou 2 et $R^7$ est $C_{1-4}$-alcoyle facultativement substitué par un ou plusieurs halo, halo ou $NR^8R^9$, où $R^8$ et $R^9$ sont choisis indépendamment entre hydrogène et $C_{1-4}$-alcoyle;

soit e) un radical $NR^8R^9$, où $R^8$ et $R^9$ sont choisis indépendamment parmi hydrogène, $C_{1-4}$-alcoyle et un radical $COR^{10}$, où $R^{10}$ est $C_{1-6}$-alcoyle ou $C_{1-6}$-alcoxy.

**4.** Composé de formule (I) suivant l'une quelconque des revendications 1 à 3, où $R^X$ est hydrogène ou $C_{1-4}$-alcoyle ou un radical (B) où b est 0 et $D^2$ est phényle; ou un radical (A), où A est O et D est hydrogène ou $C_{1-4}$-alcoxy.

**5.** Composé de formule (I) suivant l'une quelconque des revendications 1 à 4, où b est 0 et a est 0.

**6.** Composé de formule (I) suivant l'une quelconque des revendications 1 à 5, où la position 3 du radical cyclopropyle $Q^1$ est non substituée et les positions 1 et 2 sont non substituées ou substituées par fluoro ou chloro.

**7.** Composé de formule (I) suivant l'une quelconque des revendications 1 à 6, où $R^1$ est phényle facultativement substitué par 1 à 3 radicaux choisis parmi alcoyle, $C_{1-4}$-alcoyle substitué par 1 à 5

halo, halo et $C_{1-4}$-alcoxy facultativement substitué par 1 à 5 halo.

**8.** Composé selon la revendication 1 et répondant à la formule (II) :

$$Q^aQ^{1a}CR^{2a}=CR^{3a}CR^{4a}=CR^{5a}C(=X^a)NHR^{1a} \quad (II)$$

ou un sel correspondant,
où

$Q^a$ est un radical phényle ou pyridyle facultativement substitué ou un système cyclique bicyclique condensé facultativement substitué dont au moins un cycle est aromatique et contenant 0 ou 1 atome d'azote ou 0 et 1 atome de soufre; $Q^{1a}$ est un cycle cyclopropyle 1,2-disubstitué facultativement substitué par un ou plusieurs radicaux choisis parmi $C_{1-3}$-alcoyle, halo et $C_{1-3}$-haloalcoyle;

$R^{2a}$, $R^{3a}$, $R^{4a}$ et $R^{5a}$ sont identiques ou différents, au moins l'un étant un atome d'hydrogène et les autres étant choisis indépendamment parmi hydrogène, halo, $C_{1-4}$-alcoyle et $C_{1-4}$-haloalcoyle;

$X^a$ est oxygène ou soufre;

$R^{1a}$ est choisi parmi phényle facultativement substitué par halo et $C_{1-4}$-alcoyle facultativement substitué.

**9.** Composé choisi parmi :

le (±)-(2E,4E)-N-phényl-5-[c-2-(3,4-dibromophényl)-r-1-fluorocyclopropyl]penta-2,4-diénamide,

le (±)-(2E,4E)-N-(2-chlorophényl)-5-[c-2-(3,4-dibromophényl)-r-1-fluorocyclopropyl]penta-2,4-diénamide,

le (±)-(2E/Z,4E)-N-(2-méthylphényl)-5-[c-2-(3,4-dibromophényl)-r-1-fluorocyclopropyl]-3-méthylpenta-2,4-diénamide (2E:2Z = 3:1),

le (±)-(2E,4E)-N-(2-méthylphényl)-5-[c-2-(3,4-dibromophényl)-r-1-fluorocyclopropyl]-3-méthylpenta-2,4-diénamide,

le (±)-(2E,4E)-N-(2-bromophényl)-5-[c-2-(3,4-dibromophényl)-r-1-fluorocyclopropyl]penta-2,4-diénamide,

le (±)-(2E,4E)-N-(2-méthylphényl)-5-[c-2-(3,4-dibromophényl)-r-1-fluorocyclopropyl]penta-2,4-diénamide,

le (±)-(2E,4E)-N-(4-fluoro-2-méthylphényl)-5-[c-2-(3,4-dibromophényl)-r-1-fluorocyclopropyl]penta-2,4-diénamide,

le (±)-(2E,4E)-N-(5-fluoro-2-méthylphényl)-5-[c-2-(3,4-dibromophényl)-r-1-fluorocyclopropyl]penta-2,4-diénamide,

le (±)-(2E,4E)-N-(2,4-difluorophényl)-5-[c-2-(3,4-dibromophényl)-r-1-fluorocyclopropyl]penta-2,4-diénamide,

le (±)-(2E,4E)-N-(2-chlorophényl)-5-[c-2-(3,4-dibromophényl)-r-1-fluorocyclopropyl]-3-méthylpenta-2,4-diénamide,

le (±)-(2E,4E)-N-(2-méthylphényl)-5-[c-2-(3,4-dichlorophényl)-r-1-fluorocyclopropyl]penta-2,4-diénamide,

le (±)-(2E,4E)-N-(2-méthylphényl)-5-[r-1-fluoro-c-2-(3,4,5-trichlorophényl)cyclopropyl]penta-2,4-diénamide,

le (±)-(2E/Z,4E)-N-(2-méthylphényl)-5-[c-2-(3,4-dichlorophényl)-r-1-fluorocyclopropyl]-3-méthylpenta-2,4-diénamide (2E:2Z = 1:1),

le (±)-(2E,4E)-N-(2-méthylphényl)-5-[c-2-(3-chloro-4-iodophényl)-r-1-fluorocyclopropyl]penta-2,4-diénamide,

le (±)-(2E,4E)-N-(2-méthylphényl)-5-[r-1-fluoro-c-2-(4-trifluorométhylphényl)cyclopropyl]penta-2,4-diénamide,

le (±)-(2E,4E)-N-(2-méthylphényl)-5-[r-1-fluoro-c-(4-iodophényl)cyclopropyl]-3-méthylpenta-2,4-diénamide,

le (±)-(2E,4E)-N-(5-fluoro-2-méthylphényl)-5-[c-2-(3,4-dichlorophényl)-r-1-fluorocyclopropyl]penta-2,4-diénamide,

le (±)-(2E,4E)-N-(4-fluoro-2-méthylphényl)-5-[r-1-fluoro-c-2-(3,4,5-trichlorophényl)cyclopropyl]penta-2,4-diénamide,

le (±)-(2E,4E)-N-(4-fluoro-2-méthylphényl)-5-[c-2-(3,4-dichlorophényl)-r-1-fluorocyclopropyl]penta-2,4-diénamide,

le (±)-(2E,4E)-N-(2-méthylphényl)-5-[c-2-(4-bromophényl)-r-1-fluorocyclopropyl]penta-2,4-diénamide,

le (±)-(2E,4E)-N-(2-fluorométhylphényl)-5-[c-2-(3,4-dibromophényl)-r-1-fluorocyclopropyl]penta-2,4-

diénamide,

le(-)-(2E,4E)-N-(2-méthylphényl)-5-[(1R,2S)-2-(3,4-dibromophényl)-1-fluorocyclopropyl]penta-2,4-diénamide,

le (±)-(2E,4E)-N-(2-méthylphényl)-5-[r-1-chloro-c-2-(3,4-dichlorophényl)cyclopropyl]-3-méthylpenta-2,4-diénamide,

le (±)-(2E,4E)-N-(2-méthylphényl)-5-[r-1-chloro-c-2-(3,4-dibromophényl)cyclopropyl]penta-2,4-diénamide,

le (±)-(2E,4E)-N-(2-méthylphényl)-5-[r-1-chloro-c-2-(3,4-dibromophényl)cyclopropyl]-3-méthylpenta-2,4-diénamide,

le (±)-(2Z,4E)-N-(2-méthylphényl)-5-[c-2-(3,4-dibromophényl)-r-1-fluorocyclopropyl]-2-fluoro-3-méthylpenta-2,4-diénamide,

le (±)-(2Z,4E)-N-(2-chlorophényl)-5-[c-2-(3,4-dibromophényl)-r-1-fluorocyclopropyl]-2-fluoro-3-méthylpenta-2,4-diénamide,

le (±)-(2Z,4E)-N-(2-méthylphényl)-5-[c-2-(3,4-dichlorophényl)-r-1-fluorocyclopropyl]-2-fluoropenta-2,4-diénamide,

le (±)-(2Z,4E)-N-(2-méthylphényl)-5-[c-2-(3,4-dibromophényl)-r-1-fluorocyclopropyl]-2-fluoropenta-2,4-diénamide,

le (-)-(2E,4E)-N-(2-méthylphényl)-5-[(1R,2S)-trans-2-(3,4-dibromophényl)cyclopropyl]-3-méthylpenta-2,4-diénamide,

le (±)-(2E,4E)-N-méthyl-N-phényl-5-[c-2-(3,4-dibromophényl)-r-1-fluorocyclopropyl]penta-2,4-diénamide,

le (±)-(2E,4E)-N-(2-chlorophényl)-N-méthyl-5-[c-2-(3,4-dibromophényl)-r-1-fluorocyclopropyl]penta-2,4-diénamide,

le (±)-(2E,4E)-N-(2-chlorophényl)-N-méthyl-5-[c-2-(3,4-dibromophényl)-r-1-fluorocyclopropyl]-3-méthylpenta-2,4-diénamide,

le (±)-(2E,4E)-N-(2-méthylphényl)-N-carboéthoxy-5-[c-2-(3,4-dibromophényl)-r-1-fluorocyclopropyl]-penta-2,4-diénamide.

**10.** Procédé de préparation d'un composé suivant l'une quelconque des revendications 1 à 9, qui comprend :

a) lorsque X est oxygène, la réaction de l'acide ou dérivé d'acide correspondant :

$$QQ^1CR^2=CR^3CR^4=CR^5C(=X)Z^1$$

avec une amine $H_2NR^1$, où Q, $Q^1$, $R^2$, $R^3$, $R^4$, $R^5$, X et $R^1$ sont tels que définis ci-dessus et $Z^1$ est hydroxyle, $C_{1-6}$-alcoxy, halo ou un ester phosphoroimidique [-P(O)(O-aryl)NH-aryle, où aryle est $C_{6-10}$-aryle)] et X est oxygène, ou

b) la formation de la partie :

$$CR^2=CR^3CR^4=CR^5C(=X)NR^XR^1$$

par une réaction de type Wittig, et ensuite, facultativement, la conversion d'un composé de formule (I) en un autre composé de formule (I) suivant des procédés connus de l'homme de métier.

**11.** Composition pesticide comprenant un composé de formule (I) tel que défini dans l'une quelconque des revendications 1 à 9 en mélange avec un excipient ou diluant.

**12.** Composition pesticide synergique, comprenant un composé de formule (I) tel que défini dans l'une quelconque des revendications 1 à 9, un agent de synergie pour le composé de formule (I) et un excipient au diluant.

**13.** Mélange d'un composé de formule (I) tel que défini dans l'une quelconque des revendications 1 à 9 et d'un autre composé pesticide.

**14.** Utilisation d'un composé de formule (I) tel que défini à l'une quelconque des revendications 1 à 9 dans la protection et le traitement des espèces végétales, notamment comme insecticide, acaricide, molluscide ou nématocide.

**15.** Composé tel que défini dans l'une quelconque des revendications 1 à 9 ou composition suivant l'une quelconque des revendications 11 à 13 à utiliser dans un procédé de chirurgie ou de thérapeutique pratiqué sur le corps de l'être humain ou d'un animal ou dans un procédé de diagnostic pratiqué sur le corps de l'être humain ou d'un animal.

**Revendications pour l'Etat contractant suivant : ES**

**1.** Procédé pour préparer un composé de formule (I) :

$Q(CH_2)_a(O)_bQ^1CR^2=CR^3CR^4=CR^5CXNR^1R^X$

ou un sel ou propesticide de celui-ci;
où

soit Q est un système cyclique aromatique monocyclique ou bicyclique condensé facultativement substitué dans lequel au moins un cycle est aromatique, soit Q est un radical dihalovinyle ou un radical $R^6$-C≡C, où $R^6$ est $C_{1-4}$-alcoyle, tri-$C_{1-4}$-alcoylsilyle, halo ou hydrogène;

$Q^1$ est un cycle 1,2-cyclopropyle facultativement substitue par un ou plusieurs radicaux choisis parmi $C_{1-3}$-alcoyle, halo, $C_{1-3}$-haloalcoyle, $C_{2-3}$-alcynyle et cyano;

a est 0 ou 1;

b est 0 ou 1;

$R^2$, $R^3$, $R^4$ et $R^5$ sont identiques ou différents, au moins l'un étant hydrogène et les autres étant choisis indépendamment parmi hydrogène, halo, $C_{1-4}$-alcoyle et $C_{1-4}$-haloalcoyle;

X est oxygène ou soufre;

$R^1$ est phényle facultativement substitué par 1 à 5 substituants choisis parmi :

a) $C_{1-4}$-alcoyle, $C_{1-4}$-alcoxy, phénoxy et méthylènedioxy, chacun facultativement substitué par 1 à 5 halo;

b) halo, cyano, nitro, formyle, $C_{1-5}$-acyle;

c) $C_{2-3}$-alcynyle, $C_{2-3}$alcényle, chacun facultativement substitué par 1 à 5 halo;

d) un radical $S(O)_nR^7$, où n est 0, 1 ou 2 et $R^7$ est $C_{1-4}$-alcoyle, halo ou $NR^8R^9$, où $R^8$ et $R^9$ sont choisis indépendamment parmi hydrogène, $C_{1-4}$-alcoyle et $C_{1-4}$-acyle, et

e) un radical $NR^8R^9$, où $R^8$ et $R^9$ sont tels que définis ci-dessus:

$R^X$ est hydrogène ou un radical $C_{1-8}$-alcoyle ou benzyle facultativement substitué, lequel procédé comprend :

a) lorsque X est oxygène, la réaction de l'acide ou dérivé d'acide correspondant :

$QQ^1CR^2=CR^3CR^4=CR^5C(=X)Z^1$

avec une amine $H_2NR^1$, où Q, $Q^1$, $R^2$, $R^3$, $R^4$, $R^5$, X et $R^1$ sont tels que définis ci-dessus et $Z^1$ est hydroxyle, $C_{1-6}$-alcoxy, halo ou un ester phosphoroimidique [-P(O)(O-aryl)NH-aryle, où aryle est $C_{6-10}$-aryle)] et X est oxygène, ou

b) la formation de la partie :

$CR^2=CR^3CR^4=CR^5C(=X)NR^xR^1$

par une réaction de type Wittig,
et ensuite, facultativement, la conversion d'un composé de formule (I) en un autre composé de formule (I) suivant des procédés connus de l'homme de métier.

**2.** Procédé pour préparer un composé propesticide de formule (I) suivant la revendication 1, et $R^X$ est remplacé par $R^{XI}$, et $R^{XI}$ est choisi parmi :

(A)

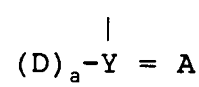

où

A est O ou S, Y est phosphore ou carbone, D est hydrogène, $C_{1-4}$-alcoyle, $C_{1-4}$-alcoxy, $C_{1-5}$-

acyle ou aryle ou bien $CO_2D^1$, où $D^1$ est $C_{1-4}$-alcoyle ou aryle et a est 1 ou 2;

(B) $-S(O)_bD^2$

où

b est 0, 1 ou 2 et soit (i) $D^2$ est $C_{1-4}$-alcoyle, aryle, aryloxy ou $C_{1-4}$-alcoxy, dans lequel un cycle aryle peut être substitué par un ou plusieurs radicaux halo, nitro, cyano, $C_{1-4}$-alcoyle et $C_{1-4}$-alcoxy dont chacun à son tour est facultativement substitué par un ou plusieurs halogènes, soit (ii) $D^2$ est un radical $ND^3D^4$, où soit $D^3$ est $-COD^5$, où $D^5$ est hydrogène, fluor ou $C_{1-4}$-alcoyle, soit $D^3$ est $C_{1-4}$-alcoyle substitué par $C_{1-5}$-acyle ou aryle, carboalcoxy ou cyano, soit $D^3$ est un radical $CO_2D^6$, où $D^6$ est $C_{1-4}$-alcoyle ou aryle et $D^4$ est hydrogène ou $C_{1-4}$-alcoyle.

**3.** Procédé pour préparer un composé de formule (I) suivant l'une quelconque des revendications 1 et 2, où Q est un radical phényle, pyridyle, thiényle ou naphtyle, chacun facultativement substitué par :
soit a) $C_{1-6}$-hydrocarbyle, $C_{1-6}$-alcoxy ou méthylènedioxy, chacun facultativement substitué par 1 à 5 halo;
soit b) halo, cyano, nitro, formyle ou $C_{1-5}$-acyle;
soit c) $C_{2-3}$-alcynyle ou $C_{2-3}$-alcényle, chacun facultativement substitué par 1 à 5 halo;
soit d) un radical $S(O)_nR^7$, où n est 0, 1 ou 2 et $R^7$ est $C_{1-4}$-alcoyle facultativement substitué par un ou plusieurs halo, halo ou $NR^8R^9$, où $R^8$ et $R^9$ sont choisis indépendamment entre hydrogène et $C_{1-4}$-alcoyle;
soit e) un radical $NR^8R^9$, où $R^8$ et $R^9$ sont choisis indépendamment parmi hydrogène, $C_{1-4}$-alcoyle et un radical $COR^{10}$, où $R^{10}$ est $C_{1-6}$-alcoyle ou $C_{1-6}$-alcoxy.

**4.** Procédé pour préparer un composé de formule (I) suivant l'une quelconque des revendications 1 à 3, où $R^X$ est hydrogène ou $C_{1-4}$-alcoyle ou un radical (B) où b est 0 et $D^2$ est phényle; ou un radical (A), où A est O et D est hydrogène ou $C_{1-4}$-alcoxy.

**5.** Procédé pour préparer un composé de formule (I) suivant l'une quelconque des revendications 1 à 4, où b est 0 et a est 0.

**6.** Procédé pour préparer un composé de formule (I) suivant l'une quelconque des revendications 1 à 5, où la position 3 du radical cyclopropyle $Q^1$ est non substituée et les positions 1 et 2 sont non substituées ou substituées par fluoro ou chloro.

**7.** Procédé pour préparer un composé de formule (I) suivant l'une quelconque des revendications 1 à 6, où $R^1$ est phényle facultativement substitué par 1 à 3 radicaux choisis parmi alcoyle, $C_{1-4}$-alcoyle substitué par 1 à 5 halo, halo et $C_{1-4}$-alcoxy facultativement substitué par 1 à 5 halo.

**8.** Procédé pour préparer un composé de formule (II) :

$$Q^aQ^{1a}CR^{2a}=CR^{3a}CR^{4a}=CR^{5a}C(=X^a)NHR^{1a} \qquad (II)$$

ou un sel correspondant,
où

$Q^a$ est un radical phényle ou pyridyle facultativement substitué ou un système cyclique bicyclique condensé facultativement substitué dont au moins un cycle est aromatique et contenant 0 ou 1 atome d'azote ou 0 et 1 atome de soufre;

$Q^{1a}$ est un cycle cyclopropyle 1,2-disubstitué facultativement substitué par un ou plusieurs radicaux choisis parmi $C_{1-3}$-alcoyle, halo et $C_{1-3}$-haloalcoyle;

$R^{2a}$, $R^{3a}$, $R^{4a}$ et $R^{5a}$ sont identiques ou différents, au moins l'un étant un atome d'hydrogène et les autres étant choisis indépendamment parmi hydrogène, halo, $C_{1-4}$-alcoyle et $C_{1-4}$-haloalcoyle;

$X^a$ est oxygène ou soufre;

$R^{1a}$ est choisi parmi phényle facultativement substitué par halo et $C_{1-4}$-alcoyle facultativement substitué.

**9.** Procédé pour préparer un composé choisi parmi :
le (±)-(2E,4E)-N-phényl-5-[c-2-(3,4-dibromophényl)-r-1-fluorocyclopropyl]penta-2,4-diénamide,

le (±)-(2E,4E)-N-(2-chlorophényl)-5-[c-2-(3,4-dibromophényl)-r-1-fluorocyclopropyl]penta-2,4-diénamide,

le (±)-(2E/Z,4E)-N-(2-méthylphényl)-5-[c-2-(3,4-dibromophényl)-r-1-fluorocyclopropyl]-3-méthylpenta-2,4-diénamide (2E:2Z = 3:1),

le (±)-(2E,4E)-N-(2-méthylphényl)-5-[c-2-(3,4-dibromophényl)-r-1-fluorocyclopropyl]-3-méthylpenta-2,4-diénamide,

le (±)-(2E,4E)-N-(2-bromophényl)-5-[c-2-(3,4-dibromophényl)-r-1-fluorocyclopropyl]penta-2,4-diénamide,

le (±)-(2E,4E)-N-(2-méthylphényl)-5-[c-2-(3,4-dibromophényl)-r-1-fluorocyclopropyl]penta-2,4-diénamide,

le (±)-(2E,4E)-N-(4-fluoro-2-méthylphényl)-5-[c-2-(3,4-dibromophényl)-r-1-fluorocyclopropyl]penta-2,4-diénamide,

le (±)-(2E,4E)-N-(5-fluoro-2-méthylphényl)-5-[c-2-(3,4-dibromophényl)-r-1-fluorocyclopropyl]penta-2,4-diénamide,

le (±)-(2E,4E)-N-(2,4-difluorophényl)-5-[c-2-(3,4-dibromophényl)-r-1-fluorocyclopropyl]penta-2,4-diénamide,

le (±)-(2E,4E)-N-(2-chlorophényl)-5-[c-2-(3,4-dibromophényl)-r-1-fluorocyclopropyl]-3-méthylpenta-2,4-diénamide,

le (±)-(2E,4E)-N-(2-méthylphényl)-5-[c-2-(3,4-dichlorophényl)-r-1-fluorocyclopropyl]penta-2,4-diénamide,

le (±)-(2E,4E)-N-(2-méthylphényl)-5-[r-1-fluoro-c-2- (3,4,5-trichlorophényl)cyclopropyl]penta-2,4-diénamide,

le (±)-(2E/Z,4E)-N-(2-méthylphényl)-5-[c-2-(3,4-dichlorophényl)-r-1-fluorocyclopropyl]-3-méthylpenta-2,4-diénamide (2E:2Z = 1:1),

le (±)-(2E,4E)-N-(2-méthylphényl)-5-[c-2-(3-chloro-4-iodophényl)-r-1-fluorocyclopropyl]penta-2,4-diénamide,

le (±)-(2E,4E)-N-(2-méthylphényl)-5-[r-1-fluoro-c-2-(4-trifluorométhylphényl)cyclopropyl]penta-2,4-diénamide,

le (±)-(2E,4E)-N-(2-méthylphényl)-5-[r-1-fluoro-c-(4-iodophényl)cyclopropyl]-3-méthylpenta-2,4-diénamide,

le (±)-(2E,4E)-N-(5-fluoro-2-méthylphényl)-5-[c-2-(3,4-dichlorophényl)-r-1-fluorocyclopropyl]penta-2,4-diénamide,

le (±)-(2E,4E)-N-(4-fluoro-2-méthylphényl)-5-[r-1-fluoro-c-2-(3,4,5-trichlorophényl)cyclopropyl]penta-2,4-diénamide,

le (±)-(2E,4E)-N-(4-fluoro-2-méthylphényl)-5-[c-2-(3,4-dichlorophényl)-r-1-fluorocyclopropyl]penta-2,4-diénamide,

le (±)-(2E,4E)-N-(2-méthylphényl)-5-[c-2-(4-bromophényl)-r-1-fluorocyclopropyl]penta-2,4-diénamide,

le (±)-(2E,4E)-N-(2-fluorométhylphényl)-5-[c-2-(3,4-dibromophényl)-r-1-fluorocyclopropyl]penta-2,4-diénamide,

le(-)-(2E,4E)-N-(2-méthylphényl)-5-[(1R,2S)-2-(3,4-dibromophényl)-1-fluorocyclopropyl]penta-2,4-diénamide,

le (±)-(2E,4E)-N-(2-méthylphényl)-5-[r-1-chloro-c-2-(3,4-dichlorophényl)cyclopropyl]-3-méthylpenta-2,4-diénamide,

le (±)-(2E,4E)-N-(2-méthylphényl)-5-[r-1-chloro-c-2-(3,4-dibromophényl)cyclopropyl]penta-2,4-diénamide,

le (±)-(2E,4E)-N-(2-méthylphényl)-5-[r-1-chloro-c-2-(3,4-dibromophényl)cyclopropyl]-3-méthylpenta-2,4-diénamide,

le (±)-(2Z,4E)-N-(2-méthylphényl)-5-[c-2-(3,4-dibromophényl)-r-1-fluorocyclopropyl]-2-fluoro-3-méthylpenta-2,4-diénamide,

le (±)-(2Z,4E)-N-(2-chlorophényl)-5-[c-2-(3,4-dibromophényl)-r-1-fluorocyclopropyl]-2-fluoro-3-méthylpenta-2,4-diénamide,

le (±)-(2Z,4E)-N-(2-méthylphényl)-5-[c-2-(3,4-dichlorophényl)-r-1-fluorocyclopropyl]-2-fluoropenta-2,4-diénamide,

le (±)-(2Z,4E)-N-(2-méthylphényl)-5-[c-2-(3,4-dibromophényl)-r-1-fluorocyclopropyl]-2-fluoropenta-2,4-diénamide,

le (-)-(2E,4E)-N-(2-méthylphényl)-5-[(1R,2S)-trans-2-(3,4-dibromophényl)cyclopropyl]-3-méthylpenta-2,4-diénamide,

le (±)-(2E,4E)-N-méthyl-N-phényl-5-[c-2-(3,4-dibromophényl)-r-1-fluorocyclopropyl]penta-2,4-diéni-

mide,

le (±)-(2E,4E)-N-(2-chlorophényl)-N-méthyl-5-[c-2-(3,4-dibromophényl)-r-1-fluorocyclopropyl)penta-2,4-diénamide,

le (±)-(2E,4E)-N-(2-chlorophényl)-N-méthyl-5-[c-2-(3,4-dibromophényl)-r-1-fluorocyclopropyl]-3-méthylpenta-2,4-diénamide,

le (±)-(2E,4E)-N-(2-méthylphényl)-N-carboéthoxy-5-[c-2-(3,4-dibromophényl)-r-1-fluorocyclopropyl)-penta-2,4-diénamide.

**10.** Procédé suivant la revendication 1(a), qui comprend l'exécution en ayant un radical aldéhyde ou cétone uni soit à l'extrémité amide/thioamide, soit au fragment $QQ^1$ de formule (I), puis en faisant réagir celui-ci avec l'ylure de phosphore approprié.

**11.** Composition pesticide comprenant un composé de formule (I) tel que défini dans l'une quelconque des revendications 1 à 9 en mélange avec un excipient ou diluant.

**12.** Composition pesticide synergique, comprenant un composé de formule (I) tel que défini dans l'une quelconque des revendications 1 à 9, un agent de synergie pour le composé de formule (I) et un excipient ou diluant.

**13.** Mélange d'un composé de formule (I) tel que défini dans l'une quelconque des revendications 1 à 9 et d'un autre composé pesticide.

**14.** Utilisation d'un composé de formule (I) tel que défini à l'une quelconque des revendications 1 à 9 dans la protection et le traitement des espèces végétales, notamment comme insecticide, acaricide, molluscide ou nématocide.

**15.** Composé tel que défini dans l'une quelconque des revendications 1 à 9 ou composition suivant l'une quelconque des revendications 11 à 13 à utiliser dans un procédé de chirurgie au de thérapeutique pratiqué sur le corps de l'être humain ou d'un animal ou dans un procédé de diagnostic pratiqué sur le corps de l'être humain ou d'un animal.

**Claims**

**Claims for the following Contracting States : AT, BE, CH, DE, DK, FR, GB, IT, GR, LI, LU, NL, PT, SE**

**1.** A compound of formula (I):

$$Q(CH_2)_a(O)_bQ^1CR^2=CR^3CR^4=CR^5CXNR^1R^X$$

or a salt or propesticide of this;

where

either Q is an optionally substituted condensed monocyclic or bicyclic aromatic cyclic system in which at least one ring is aromatic, or Q is a dihalovinyl radical or a $R^6$-C≡C- radical, where $R^6$ is a $C_{1-4}$-alkyl, tri-$C_{1-4}$-alkylsilyl, halo or hydrogen;

$Q^1$ is a 1,2-cyclopropyl ring optionally substituted by one or more radicals chosen from $C_{1-3}$-alkyl, halo, $C_{1-3}$-haloalkyl, $C_{2-3}$-alkynyl and cyano;

a is 0 or 1;

b is 0 or 1;

$R^2$, $R^3$, $R^4$ and $R^5$ are identical or different, at least one being hydrogen and the others being chosen independently from hydrogen, halo, $C_{1-4}$-alkyl and $C_{1-4}$-haloalkyl;

X is oxygen or sulphur; $R^1$ is phenyl optionally substituted by 1 to 5 substituents chosen from:

a) $C_{1-4}$-alkyl, $C_{1-4}$-alkoxy, phenoxy and methylenedioxy, each optionally substituted by 1 to 5 halo;

b) halo, cyano, nitro, formyl, $C_{1-5}$-acyl;

c) $C_{2-3}$-alkynyl, $C_{2-3}$-alkenyl, each optionally substituted by 1 to 5 halo;

d) an $S(O)_nR^7$ radical, where n is 0, 1 or 2 and $R^7$ is $C_{1-4}$-alkyl, halo or $NR^8R^9$, where $R^8$ and $R^9$ are chosen independently from hydrogen, $C_{1-4}$-alkyl and $C_{1-4}$ acyl, and

e) an $NR^8R^9$ radical, where $R^8$ and $R^9$ are as defined above:

$R^X$ is hydrogen or an optionally substituted $C_{1-8}$-alkyl or benzyl radical.

**2.** A propesticide compound of formula (I) according to claim 1, where $R^X$ is replaced by $R^{XI}$, and $R^{XI}$ is chosen from:

(A) $$(D)_a-\overset{|}{Y}=A$$

where

A is O or S, Y is phosphorus or carbon, D is hydrogen, $C_{1-4}$-alkyl, $C_{1-4}$-alkoxy, $C_{1-5}$-acyl or aryl or $CO_2D^1$, where $D^1$ is $C_{1-4}$-alkyl or aryl and a is 1 or 2;

(B) $-S(O)_bD^2$

where

b is 0, 1 or 2 and either (i) $D^2$ is $C_{1-4}$-alkyl, aryl, aryloxy or $C_{1-4}$-alkoxy, in which an aryl ring can be substituted by one or more halo, nitro, cyano, $C_{1-4}$-alkyl and $C_{1-4}$-alkoxy radicals each of which in its turn is optionally substituted by one or more halogens, or (ii) $D^2$ is an $ND^3D^4$ radical, where either $D^3$ is $-COD^5$, where $D^5$ is hydrogen, fluorine or $C_{1-4}$-alkyl, or $D^3$ is $C_{1-4}$-alkyl substituted by $C_{1-5}$-acyl or aryl, carboalkoxy or cyano, or $D^3$ is a $CO_2D^6$ radical, where $D^6$ is $C_{1-4}$-alkyl or aryl and $D^4$ is hydrogen or $C_{1-4}$-alkyl.

**3.** A compound of formula (I) according to any one of claims 1 and 2, where Q is a phenyl, pyridyl, thienyl or naphthyl radical, each optionally substituted by:

either a) $C_{1-6}$-hydrocarbyl, $C_{1-6}$-alkoxy or methylnedioxy, each optionally substituted by 1 to 5 halo;
or b) halo, cyano, nitro, formyl or $C_{1-5}$-acyl;
or c) $C_{2-3}$-alkynyl or $C_{2-3}$-alkenyl, each optionally substituted by 1 to 5 halo;
or d) an $S(O)_nR^7$ radical, where n is 0, 1 or 2 and $R^7$ is $C_{1-4}$-alkyl optionally substituted by one or more halo, halo or $NR^8R^9$ radicals, where $R^8$ and $R^9$ are chosen independently from hydrogen and $C_{1-4}$-alkyl;
or e) an $NR^8R^9$ radical, where $R^8$ and $R^9$ are chosen independently from hydrogen, $C_{1-4}$-alkyl and a $COR^{10}$ radical, where $R^{10}$ is $C_{1-6}$-alkyl or $C_{1-6}$-alkoxy.

**4.** A compound of formula (I) according to any one of claims 1 to 3, where $R^X$ is hydrogen or $C_{1-4}$-alkyl or a (B) radical where b is 0 and $D^2$ is phenyl; or an (A) radical, where A is O and D is hydrogen or $C_{1-4}$-alkoxy.

**5.** A compound of formula (I) according to any one of claims 1 to 4, where b is 0 and a is 0.

**6.** A compound of formula (I) according to any one of claims 1 to 5, where position 3 of the cyclopropyl radical $Q^1$ is non substituted and positions 1 and 2 are non substituted or substituted by fluoro or chloro.

**7.** A compound of formula (I) according to any one of claims 1 to 6, where $R^1$ is phenyl optionally substituted by 1 to 3 radicals chosen from alkyl, $C_{1-4}$-alkyl substituted by 1 to 5 halo, halo and $C_{1-4}$-alkoxy optionally substituted by 1 to 5 halo.

**8.** A compound according to claim 1 and corresponding to formula (II):

$$Q^aQ^{1a}CR^{2a}=CR^{3a}CR^{4a}=CR^{5a}C(=X^a)NHR^{1a} \quad (II)$$

or a corresponding salt,
where

$Q^a$ is an optionally substituted phenyl or pyridyl radical or an optionally substituted condensed bicyclic cyclic system at least one ring of which is aromatic and containing 0 or 1 nitrogen atoms or 0 and 1 sulphur atoms;

$Q^{1a}$ is a 1,2-disubstituted cyclopropyl ring optionally substituted by one or more radicals chosen from $C_{1-3}$-alkyl, halo and $C_{1-3}$-haloalkyl;

$R^{2a}$, $R^{3a}$, $R^{4a}$ and $R^{5a}$ are identical or different, at least one being a hydrogen atom and the others being chosen independently from hydrogen, halo, $C_{1-4}$-alkyl and $C_{1-4}$-haloalkyl;
$X^a$ is oxygen or sulphur;
$R^{1a}$ is chosen from phenyl optionally substituted by halo and optionally substituted $C_{1-4}$-alkyl.

**9.** A compound chosen from:
(±)-(2E,4E)-N-phenyl 5-[c-2-(3,4-dibromophenyl)-r-1-fluorocyclopropyl]penta-2,4-dienamide,
(±)-(2E,4E)-N-(2-chlorophenyl)-5-[c-2-(3,4-dibromophenyl)-r-1-fluorocyclopropyl]penta-2,4-dienamide,
(±)-(2E/Z,4E)-N-(2-methylphenyl)-5-[c-2-(3,4-dibromophenyl)-r-1-fluorocyclopropyl]-3-methylpenta-2,4-dienamide (2E:2Z = 3:1),
(±)-(2E,4E)-N-(2-methylphenyl)-5-[c-2-(3,4-dibromophenyl)-r-1-fluorocyclopropyl]-3-methylpenta-2,4-dienamide,
(±)-(2E,4E)-N-(2-bromophenyl)-5-[c-2-(3,4-dibromophenyl)-r-1-fluorocyclopropyl]penta-2,4-dienamide,
(±)-(2E,4E)-N-(2-methylphenyl)-5-[c-2-(3,4-dibromophenyl)-r-1-fluorocyclopropyl]penta-2,4-dienamide,
(±)-(2E,4E)-N-(4-fluoro-2-methylphenyl)-5-[c-2-(3,4-dibromophenyl)-r-1-fluorocyclopropyl]penta-2,4-dienamide,
(±)-(2E,4E)-N-(5-fluoro-2-methylphenyl)-5-[c-2-(3,4-dibromophenyl)-r-1-fluorocyclopropyl]penta-2,4-dienamide,
(±)-(2E,4E)-N-(2,4-difluorophenyl)-5-[c-2-(3,4-dibromophenyl)-r-1-fluorocyclopropyl]penta-2,4-dienamide,
(±)-(2E,4E)-N-(2-chlorophenyl)-5-[c-2-(3,4-dibromophenyl)-r-1-fluorocyclopropyl]-3-methylpenta-2,4-dienamide,
(±)-(2E,4E)-N-(2-methylphenyl)-5-[c-2-(3,4-dichlorophenyl)-r-1-fluorocyclopropyl]penta-2,4-dienamide,
(±)-(2E,4E)-N-(2-methylphenyl)-5-[r-1-fluoro-c-2-(3,4,5-trichlorophenyl)cyclopropyl]penta-2,4-dienamide,
(±)-(2E/Z,4E)-N-(2-methylphenyl)-5-[c-2-(3,4-dichlorophenyl)-r-1-fluorocyclopropyl]-3-methylpenta-2,4-dienamide (2E:2Z = 1:1),
(±)-(2E,4E)-N-(2-methylphenyl)-5-[c-2-(3-chloro-4-iodophenyl)-r-1-fluorocyclopropyl]penta-2,4-dienamide,
(±)-(2E,4E)-N-(2-methylphenyl)-5-[r-1-fluoro-c-2-(4-trifluoromethylphenyl)cyclopropyl]penta-2,4-dienamide,
(±)-(2E,4E)-N-(2-methylphenyl)-5-[r-1-fluoro-c-(4-iodophenyl)cyclopropyl]-3-methylpenta-2,4-dienamide,
(±)-(2E,4E)-N-(5-fluoro-2-methylphenyl)-5-[c-2-(3,4-dichlorophenyl)-r-1-fluorocyclopropyl]penta-2,4-dienamide,
(±)-(2E,4E)-N-(4-fluoro-2-methylphenyl)-5-[r-1-fluoro-c-2-(3,4,5-trichlorophenyl)cyclopropyl]penta-2,4-dienamide,
(±)-(2E,4E)-N-(4-fluoro-2-methylphenyl)-5-[c-2-(3,4-dichlorophenyl)-r-1-fluorocyclopropyl]penta-2,4-dienamide,
(±)-(2E,4E)-N-(2-methylphenyl)-5-[c-2-(4-bromophenyl)-r-1-fluorocyclopropyl]penta-2,4-dienamide,
(±)-(2E,4E)-N-(2-fluoromethylphenyl)-5-[c-2-(3,4-dibromophenyl)-r-1-fluorocyclopropyl]penta-2,4-dienamide,
(-)-(2E,4E)-N-(2-methylphenyl)-5-[(1R,2S)-2-(3,4-dibromophenyl)-1-fluorocyclopropyl]penta-2,4-dienamide,
(±)-(2E,4E)-N-(2-methylphenyl)-5-[r-1-chloro-c-2-(3,4-dichlorophenyl)cyclopropyl]-3-methylpenta-2,4-dienamide,
(±)-(2E,4E)-N-(2-methylphenyl)-5-[r-1-chloro-c-2-(3,4-dibromophenyl)cyclopropyl]penta-2,4-dienamide,
(±)-(2E,4E)-N-(2-methylphenyl)-5-[r-1-chloro-c-2-(3,4-dibromophenyl)cyclopropyl]-3-methylpenta-2,4-dienamide,
(±)-(2Z,4E)-N-(2-methylphenyl)-5-[c-2-(3,4-dibromophenyl)-r-1-fluorocyclopropyl]-2-fluoro-3-methylpenta-2,4-dienamide,
(±)-(2Z,4E)-N-(2-chlorophenyl)-5-[c-2-(3,4-dibromophenyl)-r-1-fluorocyclopropyl]-2-fluoro-3-methylpenta-2,4-dienamide,

(±)-(2Z,4E)-N-(2-methylphenyl)-5-[c-2-(3,4-dichlorophenyl)-r-1-fluorocyclopropyl]-2-fluoropenta-2,4-dienamide,

(±)-(2Z,4E)-N-(2-methylphenyl)-5-[c-2-(3,4-dibromophenyl)-r-1-fluorocyclopropyl]-2-fluoropenta-2,4-dienamide,

(-)-(2E,4E)-N-(2-methylphenyl)-5-[(1R,2S)-trans-2-(3,4-dibromophenyl)cyclopropyl]-3-methylpenta-2,4-dienamide,

(±)-(2E,4E)-N-methyl-N-phenyl-5-[c-2-(3,4-dibromophenyl)-r-1-fluorocyclopropyl]penta-2,4-dienamide,

(±)-(2E,4E)-N-(2-chlorophenyl)-N-methyl-5-[c-2-(3,4-dibromophenyl)-r-1-fluorocyclopropyl]penta-2,4-dienamide,

(±)-(2E,4E)-N-(2-chlorophenyl)-N-methyl-5-[c-2-(3,4-dibromophenyl)-r-1-fluorocyclopropyl]-3-methylpenta-2,4-dienamide,

(±)-(2E,4E)-N-(2-methylphenyl)-N-carboethoxy-5-[c-2-(3,4-dibromophenyl)-r-1-fluorocyclopropyl]-penta-2,4-dienamide.

**10.** Preparation process for a compound according to any one of claims 1 to 9, which includes:

a) when X is oxygen, reaction of the acid or corresponding acid derivative:

$$QQ^1CR^2=CR^3CR^4=CR^5C(=X)Z^1$$

with an amine $H_2NR^1$, where Q, $Q^1$, $R^2$, $R^3$, $R^4$, $R^5$, X and $R^1$ are as defined above and $Z^1$ is hydroxyl, $C_{1-6}$-alkoxy, halo or a phosphoroimidic ester [-P(O)(O-aryl)NH-aryl, where aryl is $C_{6-10}$-aryl)] and X is oxygen, or

b) formation of the part:

$$CR^2=CR^3CR^4=CR^5C(=X)NR^XR^1$$

by a Wittig-type reaction, and then, optionally, the conversion of a compound of formula (I) into another compound of formula (I) according to processes known to a man skilled in the art.

**11.** Pesticide composition containing a compound of formula (I) as defined in any one of claims 1 to 9 in a mixture with an excipient or diluting agent.

**12.** Synergic pesticide composition, containing a compound of formula (I) as defined in any one of claims 1 to 9, a synergist for the compound of formula (I) and an excipient or diluting agent.

**13.** Mixture of a compound of formula (I) as defined in any one of claims 1 to 9 and another pesticide compound.

**14.** Use of a compound of formula (I) as defined in any one of claims 1 to 9 for the protection and treatment of vegetable species, in particular as an insecticide, acaricide, molluscide or nematocide.

**15.** A compound as defined in any one of claims 1 to 9 or a composition according to any one of claims 11 to 13 for use in a surgical or therapeutic process carried out on a human or animal body or in a diagnostic process carried out on a human or animal body.

**Claims for the following Contracting State : ES**

**1.** Process for the preparation of a compound of formula (I):

$$Q(CH_2)_a(O)_bQ^1CR^2=CR^3CR^4=CR^5CXNR^1R^x$$

or a salt or propesticide of this;

where

either Q is an optionally substituted condensed monocyclic or bicyclic aromatic cyclic system in which at least one ring is aromatic, or Q is a dihalovinyl radical or a $R^6$-C≡C- radical, where $R^6$ is a $C_{1-4}$-alkyl, tri-$C_{1-4}$-alkylsilyl, halo or hydrogen;

$Q^1$ is a 1,2-cyclopropyl ring optionally substituted by one or more radicals chosen from $C_{1-3}$-alkyl,

halo, $C_{1-3}$-haloalkyl, $C_{2-3}$-alkynyl and cyano;

a is 0 or 1;

b is 0 or 1;

$R^2$, $R^3$, $R^4$ and $R^5$ are identical or different, at least one being hydrogen and the others being chosen independently from hydrogen, halo, $C_{1-4}$-alkyl and $C_{1-4}$-haloalkyl;

X is oxygen or sulphur;

R1 is phenyl optionally substituted by 1 to 5 substituents chosen from:

a) $C_{1-4}$-alkyl, $C_{1-4}$-alkoxy, phenoxy and methylenedioxy, each optionally substituted by 1 to 5 halo;

b) halo, cyano, nitro, formyl, $C_{1-5}$-acyl;

c) $C_{2-3}$-alkynyl, $C_{2-3}$-alkenyl, each optionally substituted by 1 to 5 halo;

d) an $S(O)_nR^7$ radical, where n is 0, 1 or 2 and $R^7$ is $C_{1-4}$-alkyl, halo or $NR^8R^9$, where $R^8$ and $R^9$ are chosen independently from hydrogen, $C_{1-4}$-alkyl and $c_{1-4}$ acyl, and

e) an $NR^8R^9$ radical, where $R^8$ and $R^9$ are as defined above:

$R^X$ is hydrogen or an optionally substituted $C_{1-8}$-alkyl or benzyl radical.

which process includes:

a) when X is oxygen, reaction of the acid or corresponding acid derivative:

$QQ^1CR^2=CR^3CR^4=CR^5C(=X)Z^1$

with an amine $H_2NR^1$, where Q, $Q^1$, $R^2$, $R^3$, $R^4$, $R^5$, X and $R^1$ are as defined above and $Z^1$ is hydroxyl, $C_{1-6}$-alkoxy, halo or a phosphoroimidic ester [-P(O)(O-aryl)NH-aryl, where aryl is $C_{6-10}$-aryl)] and X is oxygen, or

b) formation of the part:

$CR^2=CR^3CR^4=CR^5C(=X)NR^xR^1$

by a Wittig-type reaction,

and then, optionally, the conversion of a compound of formula (I) into another compound of formula (I) according to processes known to a man skilled in the art.

**2.** Process for the preparation of a propesticide compound of formula (I) according to claim 1, and $R^X$ is replaced by $R^{XI}$, and $R^{XI}$ is chosen from:

(A) 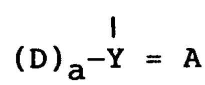

where

A is O or S, Y is phosphorus or carbon, D is hydrogen, $C_{1-4}$-alkyl, $C_{1-4}$-alkoxy, $C_{1-5}$-acyl or aryl or $CO_2D^1$, where $D^1$ is $C_{1-4}$-alkyl or aryl and a is 1 or 2;

(B) $-S(O)_bD^2$

where

b is 0, 1 or 2 and either (i) $D^2$ is $C_{1-4}$-alkyl, aryl, aryloxy or $C_{1-4}$-alkoxy, in which an aryl ring can be substituted by one or more halo, nitro, cyano, $C_{1-4}$-alkyl and $C_{1-4}$-alkoxy radicals each of which in its turn is optionally substituted by one or more halogens, or (ii) $D^2$ is an $ND^3D^4$ radical, where either $D^3$ is $-COD^5$, where $D^5$ is hydrogen, fluorine or $C_{1-4}$-alkyl, or $D^3$ is $C_{1-4}$-alkyl substituted by $C_{1-5}$-acyl or aryl, carboalkoxy or cyano, or $D^3$ is a $CO_2D^6$ radical, where $D^6$ is $C_{1-4}$-alkyl or aryl and $D^4$ is hydrogen or $C_{1-4}$-alkyl.

**3.** Process for the preparation of a compound of formula (I) according to any one of claims 1 and 2, where Q is a phenyl, pyridyl, thienyl or naphthyl radical, each optionally substituted by:

either a) $C_{1-6}$-hydrocarbyl, $C_{1-6}$-alkoxy or methylenedioxy, each optionally substituted by 1 to 5 halo;

or b) halo, cyano, nitro, formyl or $C_{1-5}$-acyl;

or c) $C_{2-3}$-alkynyl or $C_{2-3}$-alkenyl, each optionally substituted by 1 to 5 halo;

or d) an $S(O)_nR^7$ radical, where n is 0, 1 or 2 and $R^7$ is $C_{1-4}$-alkyl optionally substituted by one or more halo, halo or $NR^8R^9$ radicals, where $R^8$ and $R^9$ are chosen independently from hydrogen and $C_{1-4}$-alkyl;
or e) an $NR^8R^9$ radical, where $R^8$ and $R^9$ are chosen independently from hydrogen, $C_{1-4}$-alkyl and a $COR^{10}$ radical, where $R^{10}$ is $C_{1-6}$-alkyl or $C_{1-6}$-alkoxy.

**4.** Process for the preparation of a compound of formula (I) according to any one of claims 1 to 3, where $R^X$ is hydrogen or $C_{1-4}$-alkyl or a (B) radical where b is 0 and $D^2$ is phenyl; or an (A) radical, where A is O and D is hydrogen or $C_{1-4}$-alkoxy.

**5.** Process for the preparation of a compound of formula (I) according to any one of claims 1 to 4, where b is 0 and a is 0.

**6.** Process for the preparation of a compound of formula (I) according to any one of claims 1 to 5, where position 3 of the cyclopropyl radical $Q^1$ is non substituted and positions 1 and 2 are non substituted or substituted by fluoro or chloro.

**7.** Process for the preparation of a compound of formula (I) according to any one of claims 1 to 6, where $R^1$ is phenyl optionally substituted by 1 to 3 radicals chosen from alkyl, $C_{1-4}$-alkyl substituted by 1 to 5 halo, halo and $C_{1-4}$-alkoxy optionally substituted by 1 to 5 halo.

**8.** Process for the preparation of a compound of formula (II):

$$Q^aQ^{1a}CR^{2a}=CR^{3a}CR^{4a}=CR^{5a}C(=X^a)NHR^{1a} \qquad (II)$$

or a corresponding salt,
where

$Q^a$ is an optionally substituted phenyl or pyridyl radical or an optionally substituted condensed bicyclic cyclic system at least one ring of which is aromatic and containing 0 or 1 nitrogen atoms or 0 and 1 sulphur atoms;

$Q^{1a}$ is a 1,2-disubstituted cyclopropyl ring optionally substituted by one or more radicals chosen from $C_{1-3}$-alkyl, halo and $C_{1-3}$-haloalkyl;

$R^{2a}$, $R^{3a}$, $R^{4a}$ and $R^{5a}$ are identical or different, at least one being a hydrogen atom and the others being chosen independently from hydrogen, halo, $C_{1-4}$-alkyl and $C_{1-4}$-haloalkyl;

$X^a$ is oxygen or sulphur;

$R^{1a}$ is chosen from phenyl optionally substituted by halo and optionally substituted $C_{1-4}$-alkyl.

**9.** Process for the preparation of a compound chosen from:

(±)-(2E,4E)-N-phenyl 5-[c-2-(3,4-dibromophenyl)-r-1-fluorocyclopropyl]penta-2,4-dienamide,

(±)-(2E,4E)-N-(2-chlorophenyl)-5-[c-2-(3,4-dibromophenyl)-r-1-fluorocyclopropyl]penta-2,4-dienamide,

(±)-(2E/Z,4E)-N-(2-methylphenyl)-5-[c-2-(3,4-dibromophenyl)-r-1-fluorocyclopropyl]-3-methylpenta-2,4-dienamide (2E:2Z = 3:1),

(±)-(2E,4E)-N-(2-methylphenyl)-5-[c-2-(3,4-dibromophenyl)-r-1-fluorocyclopropyl]-3-methylpenta-2,4-dienamide,

(±)-(2E,4E)-N-(2-bromophenyl)-5-[c-2-(3,4-dibromophenyl)-r-1-fluorocyclopropyl]penta-2,4-dienamide,

(±)-(2E,4E)-N-(2-methylphenyl)-5-[c-2-(3,4-dibromophenyl)-r-1-fluorocyclopropyl]penta-2,4-dienamide,

(±)-(2E,4E)-N-(4-fluoro-2-methylphenyl)-5-[c-2-(3,4-dibromophenyl)-r-1-fluorocyclopropyl]penta-2,4-dienamide,

(±)-(2E,4E)-N-(5-fluoro-2-methylphenyl)-5-[c-2-(3,4-dibromophenyl)-r-1-fluorocyclopropyl]penta-2,4-dienamide,

(±)-(2E,4E)-N-(2,4-difluorophenyl)-5-[c-2-(3,4-dibromophenyl)-r-1-fluorocyclopropyl]penta-2,4-dienamide,

(±)-(2E,4E)-N-(2-chlorophenyl)-5-[c-2-(3,4-dibromophenyl)-r-1-fluorocyclopropyl]-3-methylpenta-2,4-dienamide,

(±)-(2E,4E)-N-(2-methylphenyl)-5-[c-2-(3,4-dichlorophenyl)-r-1-fluorocyclopropyl]penta-2,4-

dienamide,
(±)-(2E,4E)-N-(2-methylphenyl)-5-[r-1-fluoro-c-2-(3,4,5-trichlorophenyl)cyclopropyl]penta-2,4-dienamide,
(±)-(2E/Z,4E)-N-(2-methylphenyl)-5-[c-2-(3,4-dichlorophenyl)-r-1-fluorocyclopropyl]-3-methylpenta-2,4-dienamide (2E:2Z = 1:1),
(±)-(2E,4E)-N-(2-methylphenyl)-5-[c-2-(3-chloro-4-iodophenyl)-r-1-fluorocyclopropyl]penta-2,4-dienamide,
(±)-(2E,4E)-N-(2-methylphenyl)-5-[r-1-fluoro-c-2-(4-trifluoromethylphenyl)cyclopropyl]penta-2,4-dienamide,
(±)-(2E,4E)-N-(2-methylphenyl)-5-[r-1-fluoro-c-(4-iodophenyl)cyclopropyl]-3-methylpenta-2,4-dienamide,
(±)-(2E,4E)-N-(5-fluoro-2-methylphenyl)-5-[c-2-(3,4-dichlorophenyl)-r-1-fluorocyclopropyl]penta-2,4-dienamide,
(±)-(2E,4E)-N-(4-fluoro-2-methylphenyl)-5-[r-1-fluoro-c-2-(3,4,5-trichlorophenyl)cyclopropyl]penta-2,4-dienamide,
(±)-(2E,4E)-N-(4-fluoro-2-methylphenyl)-5-[c-2-(3,4-dichlorophenyl)-r-1-fluorocyclopropyl]penta-2,4-dienamide,
(±)-(2E,4E)-N-(2-methylphenyl)-5-[c-2-(4-bromophenyl)-r-1-fluorocyclopropyl]penta-2,4-dienamide,
(±)-(2E,4E)-N-(2-fluoromethylphenyl)-5-[c-2-(3,4-dibromophenyl)-r-1-fluorocyclopropyl]penta-2,4-dienamide,
(-)-(2E,4E)-N-(2-methylphenyl)-5-[(1R,2S)-2-(3,4-dibromophenyl)-1-fluorocyclopropyl]penta-2,4-dienamide,
(±)-(2E,4E)-N-(2-methylphenyl)-5-[r-1-chloro-c-2-(3,4-dichlorophenyl)cyclopropyl]-3-methylpenta-2,4-dienamide,
(±)-(2E,4E)-N-(2-methylphenyl)-5-[r-1-chloro-c-2-(3,4-dibromophenyl)cyclopropyl]penta-2,4-dienamide,
(±)-(2E,4E)-N-(2-methylphenyl)-5-[r-1-chloro-c-2-(3,4-dibromophenyl)cyclopropyl]-3-methylpenta-2,4-dienamide,
(±)-(2Z,4E)-N-(2-methylphenyl)-5-[c-2-(3,4-dibromophenyl)-r-1-fluorocyclopropyl]-2-fluoro-3-methylpenta-2,4-dienamide,
(±)-(2Z,4E)-N-(2-chlorophenyl)-5-[c-2-(3,4-dibromophenyl)-r-1-fluorocyclopropyl]-2-fluoro-3-methylpenta-2,4-dienamide,
(±)-(2Z,4E)-N-(2-methylphenyl)-5-[c-2-(3,4-dichlorophenyl)-r-1-fluorocyclopropyl]-2-fluoropenta-2,4-dienamide,
(±)-(2Z,4E)-N-(2-methylphenyl)-5-[c-2-(3,4-dibromophenyl)-r-1-fluorocyclopropyl]-2-fluoropenta-2,4-dienamide,
(-)-(2E,4E)-N-(2-methylphenyl)-5-[(1R,2S)-trans-2-(3,4-dibromophenyl)cyclopropyl]-3-methylpenta-2,4-dienamide,
(±)-(2E,4E)-N-methyl-N-phenyl-5-[c-2-(3,4-dibromophenyl)-r-1-fluorocyclopropyl]penta-2,4-dienamide,
(±)-(2E,4E)-N-(2-chlorophenyl)-N-methyl-5-[c-2-(3,4-dibromophenyl)-r-1-fluorocyclopropyl]penta-2,4-dienamide,
(±)-(2E,4E)-N-(2-chlorophenyl)-N-methyl-5-[c-2-(3,4-dibromophenyl)-r-1-fluorocyclopropyl]-3-methylpenta-2,4-dienamide,
(±)-(2E,4E)-N-(2-methylphenyl)-N-carboethoxy-5-[c-2-(3,4-dibromophenyl)-r-1-fluorocyclopropyl]-penta-2,4-dienamide.

**10.** Process according to claim 1(a), which comprises the implementation having an aldehyde or ketone radical linked either to the amide/thioamide end, or to the $QQ^1$ fragment of formula (I), then reacting this with the appropriate phosphorus ylide.

**11.** Pesticide composition containing a compound of formula (I) as defined in any one of claims 1 to 9 in a mixture with an excipient or diluting agent.

**12.** Synergic pesticide composition, containing a compound of formula (I) as defined in any one of claims 1 to 9, a synergist for the compound of formula (I) and an excipient or diluting agent.

**13.** Mixture of a compound of formula (I) as defined in any one of claims 1 to 9 and another pesticide compound.

**14.** Use of a compound of formula (I) as defined in any one of claims 1 to 9 for the protection and treatment of vegetable species, in particular as an insecticide, acaricide, molluscide or nematocide.

**15.** A compound as defined in any one of claims 1 to 9 or a composition according to any one of claims 11 to 13 for use in a surgical or therapeutic process carried out on a human or animal body or in a diagnostic process carried out on a human or animal body.

**Patentansprüche**
**Patentansprüche für folgende Vertragsstaaten : AT, BE, CH, DE, DK, FR, GB, IT, GR, LI, LU, NL, PT, SE**

**1.** Verbindung der Formel (I)

$Q(CH_2)_a(O)_bQ^1CR^2=CR^3CR^4=CR^5CXNR^1R^x$

oder ein Salz oder Propestizid derselben,
worin

entweder Q ein gegebenenfalls substituiertes, monocyclisches oder kondensiertes, bicyclisches, aromatisches Ringsystem ist, worin zumindest ein Ring aromatisch ist, oder Q ein Dihalovinylrest oder ein Rest $R^6$-C≡C-ist, worin $R^6$ für $C_{1-4}$-Alkyl, Tri-$C_{1-4}$-alkylsilyl, Halo oder Wasserstoff steht;

$Q^1$ einen 1,2-Cyclopropylring bedeutet, der gegebenenfalls durch einen oder mehrere Reste, ausgewählt unter $C_{1-3}$-Alkyl, Halo, $C_{1-3}$-Haloalkyl, $C_{2-3}$-Alkinyl und Cyano, substituiert ist;

a für 0 oder 1 steht;

b für 0 oder 1 steht;

$R^2$, $R^3$, $R^4$ und $R^5$ gleich oder voneinander verschieden sind, wobei zumindest eines Wasserstoff ist und die anderen unabhängig unter Wasserstoff, Halo, $C_{1-4}$-Alkyl und $C_{1-4}$-Haloalkyl ausgewählt sind;

X für Sauerstoff oder Schwefel steht;

$R^1$ Phenyl bedeutet, das gegebenenfalls durch 1 bis 5 Substituenten substituiert ist, ausgewählt unter

a) $C_{1-4}$-Alkyl, $C_{1-4}$-Alkoxy, Phenoxy und Methylendioxy, ein jedes gegebenenfalls substituiert durch 1 bis 5 Halo;

b) Halo, Cyano, Nitro, Formyl, $C_{1-5}$-Acyl;

c) $C_{2-3}$-Alkinyl, $C_{2-3}$-Alkenyl, ein jedes gegebenenfalls substituiert durch 1 bis 5 Halo;

d) einem Rest $S(O)_nR^7$, worin n für 0, 1 oder 2 steht und $R^7$ $C_{1-4}$-Alkyl, Halo oder $NR^8R^9$ bedeutet, worin $R^8$ und $R^9$ unabhängig unter Wasserstoff, $C_{1-4}$-Alkyl und $C_{1-4}$-Acyl ausgewählt sind; und

e) einem Rest $NR^8R^9$, worin $R^8$ und $R^9$ wie vorstehend definiert sind;

$R^x$ Wasserstoff oder einen $C_{1-8}$-Alkylrest oder gegebenenfalls substituiertes Benzyl bedeutet,

**2.** Propestizide Verbindung der Formel (I) gemäß Anspruch 1, worin $R^x$ durch $R^{x1}$ ersetzt ist und $R^{x1}$ ausgewählt ist unter

(A) $(D)_a-\overset{|}{Y}=A$

worin

A für O oder S steht, Y Phosphor oder Kohlenstoff bedeutet, D Wasserstoff, $C_{1-4}$-Alkyl, $C_{1-4}$-Alkoxy, $C_{1-5}$-Acyl oder Aryl oder auch $CO_2D^1$, worin $D^1$ $C_{1-4}$-Alkyl oder Aryl ist, bedeutet und a für 1 oder 2 steht;

(B) $-S(O)_bD^2$

worin

b für 0, 1 oder 2 steht und entweder (i) $D^2$ $C_{1-4}$-Alkyl, Aryl, Aryloxy oder $C_{1-4}$-Alkoxy ist, worin ein

Arylring substituiert sein kann durch einen oder mehrere Reste Halo, Nitro, Cyano, $C_{1-4}$-Alkyl und $C_{1-4}$-Alkoxy, von denen seinerseits ein jedes gegebenenfalls durch ein oder mehrere Halogene substituiert ist, oder (ii) $D^2$ ein Rest $ND^3D^4$ ist, worin entweder $D^3$ für $-COD^5$ steht, worin $D^5$ Wasserstoff, Fluor oder $C_{1-4}$-Alkyl bedeutet, oder $D^3$ $C_{1-4}$-Alkyl, substituiert durch $C_{1-5}$-Acyl oder Aryl, Carbalkoxy oder Cyano ist oder $D^3$ ein Rest $CO_2D^6$, worin $D^6$ $C_{1-4}$-Alkyl oder Aryl bedeutet, und $D^4$ Wasserstoff oder $C_{1-4}$-Alkyl bedeutet.

**3.** Verbindung der Formel (I) gemäß einem der Ansprüche 1 und 2, worin Q für einen Phenyl-, Pyridyl-, Thienyl- oder Naphthylrest steht, von denen ein jeder gegebenenfalls substituiert ist durch

entweder a) $C_{1-6}$-Hydrocarbyl, $C_{1-6}$-Alkoxy oder Methylendioxy, ein jedes gegebenenfalls substituiert durch 1 bis 5 Halo;

oder b) Halo, Cyano, Nitro, Formyl oder $C_{1-5}$-Acyl;

oder c) $C_{2-3}$-Alkinyl oder $C_{2-3}$-Alkenyl, ein jedes gegebenenfalls substituiert durch 1 bis 5 Halo;

oder d) einen Rest $S(O)_nR^7$, worin n für 0, 1 oder 2 steht und $R^7$ $C_{1-4}$-Alkyl, gegebenenfalls substituiert durch ein oder mehrere Halo, Halo oder $NR^8R^9$ bedeutet, worin $R^8$ und $R^9$ unabhängig unter Wasserstoff und $C_{1-4}$-Alkyl ausgewählt sind;

oder e) einen Rest $NR^8R^9$, worin $R^8$ und $R^9$ unabhängig unter Wasserstoff, $C_{1-4}$-Alkyl und einem Rest $COR^{10}$, worin $R^{10}$ für $C_{1-6}$-Alkyl oder $C_{1-6}$-Alkoxy steht, ausgewählt sind.

**4.** Verbindung der Formel (I) gemäß einem der Ansprüche 1 bis 3, worin $R^x$ Wasserstoff oder $C_{1-4}$-Alkyl oder einen Rest (B), worin b für 0 steht und $D^2$ Phenyl ist, oder einen Rest (A), worin A für O steht und D Wasserstoff oder $C_{1-4}$-Alkoxy darstellt, bedeutet.

**5.** Verbindung der Formel (I) gemäß einem der Ansprüche 1 bis 4, worin b für 0 steht und a für 0 steht.

**6.** Verbindung der Formel (I) gemäß einem der Ansprüche 1 bis 5, worin die Stellung 3 des Cyclopropylrestes $Q^1$ unsubstituiert ist und die Stellungen 1 und 2 unsubstituiert oder durch Fluor oder Chlor substituiert sind.

**7.** Verbindung der Formel (I) gemäß einem der Ansprüche 1 bis 6, worin $R^1$ Phenyl ist, welches gegebenenfalls substituiert ist durch 1 bis 3 Reste, ausgewählt unter Alkyl, $C_{1-4}$-Alkyl, substituiert durch 1 bis 5 Halo, Halo und $C_{1-4}$-Alkoxy, gegebenenfalls substituiert durch 1 bis 5 Halo.

**8.** Verbindung gemäß Anspruch 1 entsprechend der Formel (II)

$$Q^aQ^{1a}CR^{2a}=CR^{3a}CR^{4a}=CR^{5a}C(=X^a)NHR^{1a} \qquad (II)$$

oder ein entsprechendes Salz, worin

$Q^a$ einen gegebenenfalls substituierten Phenyl- oder Pyridylrest oder ein gegebenenfalls substituiertes, kondensiertes, bicyclisches Ringsystem, von dem zumindest ein Ring aromatisch ist und 0 oder 1 Stickstoffatom oder 0 und 1 Sauerstoffatom enthält, bedeutet;

$Q^{1a}$ ein 1,2-disubstituierter Cyclopropylring, gegebenenfalls substituiert durch einen oder mehrere Reste, ausgewählt unter $C_{1-3}$-Alkyl, Halo und $C_{1-3}$-Haloalkyl, ist;

$R^{2a}$, $R^{3a}$, $R^{4a}$ und $R^{5a}$ identisch oder verschieden sind, worin zumindest eines ein Wasserstoffatom ist und die anderen unabhängig unter Wasserstoff, Halo, $C_{1-4}$-Alkyl und $C_{1-4}$-Haloalkyl ausgewählt sind;

$X^a$ für Sauerstoff oder Schwefel steht;

$R^{1a}$ ausgewählt ist unter Phenyl, gegebenenfalls substituiert durch Halo, und gegebenenfalls substituiertem $C_{1-4}$-Alkyl.

**9.** Verbindung, ausgewählt unter

(±)-(2E,4E)-N-Phenyl-5-[c-2-(3,4-dibromphenyl)-r-1-fluorcyclopropyl)-penta-2,4-dienamid,

(±)-(2E,4E)-N-(2-Chlorphenyl)-5-[c-2-(3,4-dibromphenyl)-r-1-fluorcyclopropyl)-penta-2,4-dienamid,

(±)-(2E/Z,4E)-N-(2-Methylphenyl)-5-[c-2-(3,4-dibromphenyl)-r-1-fluorcyclopropyl]-3-methylpenta-2,4-dienamid (2E:2Z = 3:1),

(±)-(2E,4E)-N-(2-Methylphenyl)-5-[c-2-(3,4-dibromphenyl)-r-1-fluorcyclopropyl]-3-methylpenta-2,4-dienamid,

(±)-(2E,4E)-N-(2-Bromphenyl)-5-[c-2-(3,4-dibromphenyl)-r-1-fluorcyclopropyl]-penta-2,4-dienamid,

(±)-(2E,4E)-N-(2-Methylphenyl)-5-[c-2-(3,4-dibromphenyl)-r-1-fluorcyclopropyl]-penta-2,4-dienamid,
(±)-(2E,4E)-N-(4-Fluor-2-methylphenyl)-5-[c-2-(3,4-dibromphenyl)-r-1-fluorcyclopropyl]-penta-2,4-dienamid,
(±)-(2E,4E)-N-(5-Fluor-2-methylphenyl)-5-[c-2-(3,4-dibromphenyl)-r-1-fluorcyclopropyl]-penta-2,4-dienamid,
(±)-(2E,4E)-N-(2,4-Difluorphenyl)-5-[c-2-(3,4-dibromphenyl)-r-1-fluorcyclopropyl]-penta-2,4-dienamid,
(±)-(2E,4E)-N-(2-Chlorphenyl)-5-[c-2-(3,4-dibromphenyl)-r-1-fluorcyclopropyl]-3-methylpenta-2,4-dienamid,
(±)-(2E,4E)-N-(2-Methylphenyl)-5-[c-2-(3,4-dichlorphenyl)-r-1-fluorcyclopropyl]-penta-2,4-dienamid,
(±)-(2E,4E)-N-(2-Methylphenyl)-5-[r-1-fluor-c-2-(3,4,5-trichlorphenyl)-cyclopropyl]-penta-2,4-dienamid,
(±)-(2E/Z,4E)-N-(2-Methylphenyl)-5-[c-2-(3,4-dichlorphenyl)-r-1-fluorcyclopropyl]-3-methylpenta-2,4-dienamid (2E:2Z = 1:1),
(±)-(2E,4E)-N-(2-Methylphenyl)-5-[c-2-(3-chlor-4-jodphenyl)-r-1-fluorcyclopropyl]-penta-2,4-dienamid,
(±)-(2E,4E)-N-(2-Methylphenyl)-5-[r-1-fluor-c-2-(4-trifluormethylphenyl)-cyclopropyl]-penta-2,4-dienamid,
(±)-(2E,4E)-N-(2-Methylphenyl)-5-[r-1-fluor-c(4-jodphenyl)-cyclopropyl]-3-methylpenta-2,4-dienamid,
(±)-(2E,4E)-N-(5-Fluor-2-methylphenyl)-5-[c-2-(3,4-dichlorphenyl)-r-1-fluorcyclopropyl]-penta-2,4-dienamid,
(±)-(2E,4E)-N-(4-Fluor-2-methylphenyl)-5-[r-1-fluor-c-2-(3,4,5-trichlorphenyl)-cyclopropyl]-penta-2,4-dienamid,
(±)-(2E,4E)-N-(4-Fluor-2-methylphenyl)-5-[c-2-(3,4-dichlorphenyl)-r-1-fluorcyclopropyl]-penta-2,4-dienamid,
(±)-(2E,4E)-N-(2-Methylphenyl)-5-[c-2-(4-bromphenyl)-r-1-fluorcyclopropyl]-penta-2,4-dienamid,
(±)-(2E,4E)-N-(2-Fluormethylphenyl)-5-[c-2-(3,4-dibromphenyl)-r-1-fluorcyclopropyl]-penta-2,4-dienamid,
(-)-(2E,4E)-N-(2-Methylphenyl)-5-[(1R,2S)-2-(3,4-dibromphenyl)-1-fluorcyclopropyl]-penta-2,4-dienamid,
(±)-(2E,4E)-N-(2-Methylphenyl)-5-[r-1-chlor-c-2-(3,4-dichlorphenyl)-cyclopropyl]-3-methylpenta-2,4-dienamid,
(±)-(2E,4E)-N-(2-Methylphenyl)-5-[r-1-chlor-c-2-(3,4-dibromphenyl)-cyclopropyl]-penta-2,4-dienamid,
(±)-(2E,4E)-N-(2-Methylphenyl)-5-[r-1-chlor-c-2-(3,4-dibromphenyl)-cyclopropyl]-3-methylpenta-2,4-dienamid,
(±)-(2Z,4E)-N-(2-Methylphenyl)-5-[c-2-(3,4-dibromphenyl)-r-1-fluorcyclopropyl]-2-fluor-3-methylpenta-2,4-dienamid,
(±)-(2Z,4E)-N-(2-Chlorphenyl)-5-[c-2-(3,4-dibromphenyl)-r-1-fluorcyclopropyl]-2-fluor-3-methylpenta-2,4-dienamid,
(±)-(2Z,4E)-N-(2-Methylphenyl)-5-[c-2-(3,4-dichlorphenyl)-r-1-fluorcyclopropyl]-2-fluorpenta-2,4-dienamid,
(±)-(2Z,4E)-N-(2-Methylphenyl)-5-[c-2-(3,4-dibromphenyl)-r-1-fluorcyclopropyl]-2-fluorpenta-2,4-dienamid,
(-)-(2E,4E)-N-(2-Methylphenyl)-5-[(1R,2S)-trans-2-(3,4-dibromphenyl)-cyclopropyl]-3-methylpenta-2,4-dienamid,
(±)-(2E,4E)-N-Methyl-N-phenyl-5-[c-2-(3,4-dibromphenyl)-r-1-fluorcyclopropyl]-penta-2,4-dienamid,
(±)-(2E,4E)-N-(2-Chlorphenyl)-N-methyl-5-[c-2-(3,4-dibromphenyl)-r-1-fluorcyclopropyl]-penta-2,4-dienamid,
(±)-(2E,4E)-N-(2-Chlorphenyl)-N-methyl-5-[c-2-(3,4-dibromphenyl)-r-1-fluorcyclopropyl]-3-methylpenta-2,4-dienamid,
(±)-(2E,4E)-N-(2-Methylphenyl)-N-carboethoxy-5-[c-2-(3,4-dibromphenyl)-r-1-fluorcyclopropyl]-penta-2,4-dienamid.

**10.** Verfahren zur Herstellung einer Verbindung gemäß einem der Ansprüche 1 bis 9, welches umfaßt
a) wenn X für Sauerstoff steht, die Umsetzung der Säure oder des Säurederivats entsprechend

$$QQ^1CR^2=CR^3CR^4=CR^5C(=X)Z^1$$

mit einem Amin $H_2NR^1$, worin Q, $Q^1$, $R^2$, $R^3$, $R^4$, $R^5$, X und $R^1$ wie vorstehend definiert sind und $Z^1$

Hydroxyl, $C_{1-6}$-Alkoxy, Halo oder einen Phosphoroimidester [-P(O)(O-Aryl)NH-Aryl, worin Aryl für $C_{6-10}$-Aryl steht] bedeutet und X für Sauerstoff steht, oder

b) die Bildung des Teils

$CR^2=CR^3CR^4=CR^5C(=X)NR^xR^1$

durch eine Umsetzung vom Wittig-Typ und hierauf gegebenenfalls die Umwandlung einer Verbindung der Formel (I) in eine andere Verbindung der Formel (I) nach dem Fachmann bekannten Verfahren.

**11.** Pestizide Zusammensetzung, enthaltend eine Verbindung der Formel (I) gemäß einem der Ansprüche 1 bis 9 im Gemisch mit einem Exzipienten oder Verdünnungsmittel.

**12.** Synergistische, pestizide Zusammensetzung, enthaltend eine Verbindung der Formel (I) gemäß einem der Ansprüche 1 bis 9, ein synergistisches Mittel für die Verbindung der Formel (I) und einen Exzipienten oder Verdünnungsmittel.

**13.** Gemisch einer Verbindung der Formel (I) gemäß einem der Ansprüche 1 bis 9 und einer weiteren pestiziden Verbindung.

**14.** Verwendung einer Verbindung der Formel (I) gemäß einem der Ansprüche 1 bis 9 beim Schutz und der Behandlung von Pflanzengattungen, insbesondere als Insektizid, Akarizid, Molluszid oder Nematozid.

**15.** Verbindung gemäß einem der Ansprüche 1 bis 9 oder Zusammensetzung gemäß einem der Ansprüche 11 bis 13 für die Verwendung bei einem chirurgischen oder therapeutischen Verfahren, das am menschlichen oder tierischen Körper ausgeübt wird, oder bei einem diagnostischen Verfahren, das am menschlichen oder tierischen Körper ausgeübt wird.

**Patentansprüche für folgenden Vertragsstaat : ES**

**1.** Verfahren zur Herstellung einer Verbindung der Formel (I)

$Q(CH_2)_a(O)_bQ^1CR^2=CR^3CR^4=CR^5CXNR^1R^x$

oder eines Salzes oder Propestizids derselben,
worin

entweder Q ein gegebenenfalls substituiertes, monocyclisches oder kondensiertes, bicyclisches, aromatisches Ringsystem ist, worin zumindest ein Ring aromatisch ist, oder Q ein Dihalovinylrest oder ein Rest $R^6$-C≡C ist, worin $R^6$ für $C_{1-4}$-Alkyl, Tri-$C_{1-4}$-alkylsilyl, Halo oder Wasserstoff steht;

$Q^1$ einen 1,2-Cyclopropylring bedeutet, der gegebenenfalls durch einen oder mehrere Reste, ausgewählt unter $C_{1-3}$-Alkyl, Halo, $C_{1-3}$-Haloalkyl, $C_{2-3}$-Alkinyl und Cyano, substituiert ist;

a für 0 oder 1 steht;

b für 0 oder 1 steht;

$R^2$, $R^3$, $R^4$ und $R^5$ gleich oder voneinander verschieden sind, wobei zumindest eines Wasserstoff ist und die anderen unabhängig unter Wasserstoff, Halo, $C_{1-4}$-Alkyl und $C_{1-4}$-Haloalkyl ausgewählt sind;

X für Sauerstoff oder Schwefel steht;

$R^1$ Phenyl bedeutet, das gegebenenfalls durch 1 bis 5 Substituenten substituiert ist, ausgewählt unter

a) $C_{1-4}$-Alkyl, $C_{1-4}$-Alkoxy, Phenoxy und Methylendioxy, ein jedes gegebenenfalls substituiert durch 1 bis 5 Halo;

b) Halo, Cyano, Nitro, Formyl, $C_{1-5}$-Acyl;

c) $C_{2-3}$-Alkinyl, $C_{2-3}$-Alkenyl, ein jedes gegebenenfalls substituiert durch 1 bis 5 Halo;

d) einem Rest $S(O)_nR^7$, worin n für 0, 1 oder 2 steht und $R^7$ $C_{1-4}$-Alkyl, Halo oder $NR^8R^9$ bedeutet, worin $R^8$ und $R^9$ unabhängig unter Wasserstoff, $C_{1-4}$-Alkyl und $C_{1-4}$-Acyl ausgewählt sind; und

e) einem Rest $NR^8R^9$, worin $R^8$ und $R^9$ wie vorstehend definiert sind;

$R^x$ Wasserstoff oder einen $C_{1-8}$-Alkylrest oder gegebenenfalls substituiertes Benzyl bedeutet, welches Verfahren umfaßt:

a) wenn X für Sauerstoff steht, die Umsetzung der Säure oder des Säurederivats entsprechend

$QQ^1CR^2=CR^3CR^4=CR^5C(=X)Z^1$

mit einem Amin $H_2NR^1$, worin Q, $Q^1$, $R^2$, $R^3$, $R^4$, $R^5$, X und $R^1$ wie vorstehend definiert sind und $Z^1$ Hydroxyl, $C_{1-6}$-Alkoxy, Halo oder einen Phosphoroimidester [-P(O)(O-Aryl)NH-Aryl, worin Aryl für $C_{6-10}$-Aryl steht] bedeutet und X für Sauerstoff steht, oder
b) die Bildung des Teils

$CR^2=CR^3CR^4=CR^5C(=X)NR^xR^1$

durch eine Umsetzung vom Wittig-Typ und hierauf gegebenenfalls die Umwandlung einer Verbindung der Formel (I) in eine andere Verbindung der Formel (I) nach dem Fachmann bekannten Verfahren.

2. Verfahren zur Herstellung einer propestiziden Verbindung der Formel (I) gemäß Anspruch 1, worin $R^x$ durch $R^{x1}$ ersetzt ist und $R^{x1}$ ausgewählt ist unter

(A) $$(D)_a\text{-}\dot{Y} = A$$

worin
A für O oder S steht, Y Phosphor oder Kohlenstoff bedeutet, D Wasserstoff, $C_{1-4}$-Alkyl, $C_{1-4}$-Alkoxy, $C_{1-5}$-Acyl oder Aryl oder auch $CO_2D^1$, worin $D^1$ $C_{1-4}$-Alkyl oder Aryl ist, bedeutet und a für 1 oder 2 steht;

(B) $-S(O)_bD^2$

worin
b für 0, 1 oder 2 steht und entweder (i) $D^2$ $C_{1-4}$-Alkyl, Aryl, Aryloxy oder $C_{1-4}$-Alkoxy ist, worin ein Arylring substituiert sein kann durch einen oder mehrere Reste Halo, Nitro, Cyano, $C_{1-4}$-Alkyl und $C_{1-4}$-Alkoxy, von denen seinerseits ein jedes gegebenenfalls durch ein oder mehrere Halogene substituiert ist, oder (ii) $D^2$ ein Rest $ND^3D^4$ ist, worin entweder $D^3$ für $-COD^5$ steht, worin $D^5$ Wasserstoff, Fluor oder $C_{1-4}$-Alkyl bedeutet, oder $D^3$ $C_{1-4}$-Alkyl, substituiert durch $C_{1-5}$-Acyl oder Aryl, Carbalkoxy oder Cyano ist oder $D^3$ ein Rest $CO_2D^6$ ist, worin $D^6$ $C_{1-4}$-Alkyl oder Aryl bedeutet, und $D^4$ Wasserstoff oder $C_{1-4}$-Alkyl bedeutet.

3. Verfahren zur Herstellung einer Verbindung der Formel (I) gemäß einem der Ansprüche 1 und 2, worin Q für einen Phenyl-, Pyridyl-, Thienyl- oder Naphthylrest steht, von denen ein jeder gegebenenfalls substituiert ist durch
entweder a) $C_{1-6}$-Hydrocarbyl, $C_{1-6}$-Alkoxy oder Methylendioxy, ein jedes gegebenenfalls substituiert durch 1 bis 5 Halo;
oder b) Halo, Cyano, Nitro, Formyl oder $C_{1-5}$-Acyl;
oder c) $C_{2-3}$-Alkinyl oder $C_{2-3}$-Alkenyl, ein jedes gegebenenfalls substituiert durch 1 bis 5 Halo;
oder d) einen Rest $S(O)_nR^7$, worin n für 0, 1 oder 2 steht und $R^7$ $C_{1-4}$-Alkyl, gegebenenfalls substituiert durch ein oder mehrere Halo, Halo oder $NR^8R^9$ bedeutet, worin $R^8$ und $R^9$ unabhängig unter Wasserstoff und $C_{1-4}$-Alkyl ausgewählt sind;
oder e) einen Rest $NR^8R^9$, worin $R^8$ und $R^9$ unabhängig unter Wasserstoff, $C_{1-4}$-Alkyl und einem Rest $COR^{10}$, worin $R^{10}$ für $C_{1-6}$-Alkyl oder $C_{1-6}$-Alkoxy steht, ausgewählt sind.

4. Verfahren zur Herstellung einer Verbindung der Formel (I) gemäß einem der Ansprüche 1 bis 3, worin $R^x$ Wasserstoff oder $C_{1-4}$-Alkyl oder einen Rest (B), worin b für 0 steht und $D^2$ Phenyl ist, oder einen Rest (A), worin A für O steht und D Wasserstoff oder $C_{1-4}$-Alkoxy darstellt, bedeutet.

5. Verfahren zur Herstellung einer Verbindung der Formel (I) gemäß einem der Ansprüche 1 bis 4, worin b für 0 steht und a für 0 steht.

6. Verfahren zur Herstellung einer Verbindung der Formel (I) gemäß einem der Ansprüche 1 bis 5, worin die 3-Stellung des Cyclopropylrestes $Q^1$ unsubstituiert ist und die 1- und 2-Stellungen unsubstituiert oder durch Fluor oder Chlor substituiert sind.

7. Verfahren zur Herstellung einer Verbindung der Formel (I) gemäß einem der Ansprüche 1 bis 6, worin $R^1$ Phenyl ist, welches gegebenenfalls substituiert ist durch 1 bis 3 Reste, ausgewählt unter Alkyl, $C_{1-4}$-Alkyl, substituiert durch 1 bis 5 Halo, Halo und $C_{1-4}$-Alkoxy, gegebenenfalls substituiert durch 1 bis 5 Halo.

8. Verfahren zur Herstellung einer Verbindung der Formel (II)

$Q^aQ^{1a}CR^{2a}=CR^{3a}CR^{4a}=CR^{5a}C(=X^a)NHR^{1a}$ (II)

oder eines entsprechenden Salzes,
worin

$Q^a$ einen gegebenenfalls substituierten Phenyl- oder Pyridylrest oder ein gegebenenfalls substituiertes, kondensiertes, bicyclisches Ringsystem, von dem zumindest ein Ring aromatisch ist und 0 oder 1 Stickstoffatom oder 0 und 1 Sauerstoffatom enthält, bedeutet;

$Q^{1a}$ ein 1,2-disubstituierter Cyclopropylring, gegebenenfalls substituiert durch einen oder mehrere Reste, ausgewählt unter $C_{1-3}$-Alkyl, Halo und $C_{1-3}$-Haloalkyl, ist;

$R^{2a}$, $R^{3a}$, $R^{4a}$ und $R^{5a}$ identisch oder verschieden sind, worin zumindest eines ein Wasserstoffatom ist und die anderen unabhängig unter Wasserstoff, Halo, $C_{1-4}$-Alkyl und $C_{1-4}$-Haloalkyl ausgewählt sind;

$X^a$ für Sauerstoff oder Schwefel steht;

$R^{1a}$ ausgewählt ist unter Phenyl, gegebenenfalls substituiert durch Halo, und gegebenenfalls substituiertem $C_{1-4}$-Alkyl.

9. Verfahren zur Herstellung einer Verbindung, ausgewählt unter

(±)-(2E,4E)-N-Phenyl-5-[c-2-(3,4-dibromphenyl)-r-1-fluorcyclopropyl)-penta-2,4-dienamid,

(±)-(2E,4E)-N-(2-Chlorphenyl)-5-[c-2-(3,4-dibromphenyl)-r-1-fluorcyclopropyl)-penta-2,4-dienamid,

(±)-(2E/Z,4E)-N-(2-Methylphenyl)-5-[c-2-(3,4-dibromphenyl)-r-1-fluorcyclopropyl]-3-methylpenta-2,4-dienamid (2E:2Z = 3:1),

(±)-(2E,4E)-N-(2-Methylphenyl)-5-[c-2-(3,4-dibromphenyl)-r-1-fluorcyclopropyl]-3-methylpenta-2,4-dienamid,

(±)-(2E,4E)-N-(2-Bromphenyl)-5-[c-2-(3,4-dibromphenyl)-r-1-fluorcyclopropyl]-penta-2,4-dienamid,

(±)-(2E,4E)-N-(2-Methylphenyl)-5-[c-2-(3,4-dibromphenyl)-r-1-fluorcyclopropyl]-penta-2,4-dienamid,

(±)-(2E,4E)-N-(4-Fluor-2-methylphenyl)-5-[c-2-(3,4-dibromphenyl)-r-1-fluorcyclopropyl]-penta-2,4-dienamid,

(±)-(2E,4E)-N-(5-Fluor-2-methylphenyl)-5-[c-2-(3,4-dibromphenyl)-r-1-fluorcyclopropyl]-penta-2,4-dienamid,

(±)-(2E,4E)-N-(2,4-Difluorphenyl)-5-[c-2-(3,4-dibromphenyl)-r-1-fluorcyclopropyl]-penta-2,4-dienamid,

(±)-(2E,4E)-N-(2-Chlorphenyl)-5-[c-2-(3,4-dibromphenyl)-r-1-fluorcyclopropyl]-3-methylpenta-2,4-dienamid,

(±)-(2E,4E)-N-(2-Methylphenyl)-5-[c-2-(3,4-dichlorphenyl)-r-1-fluorcyclopropyl]-penta-2,4-dienamid,

(±)-(2E,4E)-N-(2-Methylphenyl)-5-[r-1-fluor-c-2-(3,4,5-trichlorphenyl)-cyclopropyl]-penta-2,4-dienamid,

(±)-(2E/Z,4E)-N-(2-Methylphenyl)-5-[c-2-(3,4-dichlorphenyl)-r-1-fluorcyclopropyl]-3-methylpenta-2,4-dienamid (2E:2Z = 1:1),

(±)-(2E,4E)-N-(2-Methylphenyl)-5-[c-2-(3-chlor-4-jodphenyl)-r-1-fluorcyclopropyl]-penta-2,4-dienamid,

(±)-(2E,4E)-N-(2-Methylphenyl)-5-[r-1-fluor-c-2-(4-trifluormethylphenyl)-cyclopropyl]-penta-2,4-dienamid,

(±)-(2E,4E)-N-(2-Methylphenyl)-5-[r-1-fluor-c-(4-jodphenyl)-cyclopropyl]-3-methylpenta-2,4-dienamid,

(±)-(2E,4E)-N-(5-Fluor-2-methylphenyl)-5-[c-2-(3,4-dichlorphenyl)-r-1-fluorcyclopropyl]-penta-2,4-dienamid,

(±)-(2E,4E)-N-(4-Fluor-2-methylphenyl)-5-[r-1-fluor-c-2-(3,4,5-trichlorphenyl)-cyclopropyl]-penta-2,4-dienamid,

(±)-(2E,4E)-N-(4-Fluor-2-methylphenyl)-5-[c-2-(3,4-dichlorphenyl)-r-1-fluorcyclopropyl]-penta-2,4-

dienamid,
(±)-(2E,4E)-N-(2-Methylphenyl)-5-[c-2-(4-bromphenyl)-r-1-fluorcyclopropyl]-penta-2,4-dienamid,
(±)-(2E,4E)-N-(2-Fluormethylphenyl)-5-[c-2-(3,4-dibromphenyl)-r-1-fluorcyclopropyl]-penta-2,4-dienamid,
(-)-(2E,4E)-N-(2-Methylphenyl)-5-[(1R,2S)-2-(3,4-dibromphenyl)-1-fluorcyclopropyl]-penta-2,4-dienamid,
(±)-(2E,4E)-N-(2-Methylphenyl)-5-[r-1-chlor-c-2-(3,4-dichlorphenyl)-cyclopropyl]-3-methylpenta-2,4-dienamid,
(±)-(2E,4E)-N-(2-Methylphenyl)-5-[r-1-chlor-c-2-(3,4-dibromphenyl)-cyclopropyl]-penta-2,4-dienamid,
(±)-(2E,4E)-N-(2-Methylphenyl)-5-[r-1-chlor-c-2-(3,4-dibromphenyl)-cyclopropyl]-3-methylpenta-2,4-dienamid,
(±)-(2Z,4E)-N-(2-Methylphenyl)-5-[c-2-(3,4-dibromphenyl)-r-1-fluorcyclopropyl]-2-fluor-3-methylpenta-2,4-dienamid,
(±)-(2Z,4E)-N-(2-Chlorphenyl)-5-[c-2-(3,4-dibromphenyl)-r-1-fluorcyclopropyl]-2-fluor-3-methylpenta-2,4-dienamid,
(±)-(2Z,4E)-N-(2-Methylphenyl)-5-[c-2-(3,4-dichlorphenyl)-r-1-fluorcyclopropyl]-2-fluorpenta-2,4-dienamid,
(±)-(2Z,4E)-N-(2-Methylphenyl)-5-[c-2-(3,4-dibromphenyl)-r-1-fluorcyclopropyl]-2-fluorpenta-2,4-dienamid,
(-)-(2E,4E)-N-(2-Methylphenyl)-5-[(1R,2S)-trans-2-(3,4-dibromphenyl)-cyclopropyl]-3-methylpenta-2,4-dienamid,
(±)-(2E,4E)-N-Methyl-N-phenyl-5-[c-2-(3,4-dibromphenyl)-r-1-fluorcyclopropyl]-penta-2,4-dienamid,
(±)-(2E,4E)-N-(2-Chlorphenyl)-N-methyl-5-[c-2-(3,4-dibromphenyl)-r-1-fluorcyclopropyl]-penta-2,4-dienamid,
(±)-(2E,4E)-N-(2-Chlorphenyl)-N-methyl-5-[c-2-(3,4-dibromphenyl)-r-1-fluorcyclopropyl]-3-methylpenta-2,4-dienamid,
(±)-(2E,4E)-N-(2-Methylphenyl)-N-carboethoxy-5-[c-2-(3,4-dibromphenyl)-r-1-fluorcyclopropyl]-penta-2,4-dienamid.

**10.** Verfahren gemäß Anspruch 1(a), umfassend die Ausführungsform, bei der man einen Aldehyd- oder Ketonrest entweder mit dem Amid/Thioamid-Ende oder mit dem Fragment $QQ^1$ der Formel (I) verbunden hat, wonach man diesen mit dem geeigneten Phosphorylid umsetzt.

**11.** Pestizide Zusammensetzung, umfassend eine Verbindung der Formel (I) gemäß einem der Ansprüche 1 bis 9 im Gemisch mit einem Exzipienten oder Verdünnungsmittel.

**12.** Synergistische, pestizide Zusammensetzung, umfassend eine Verbindung der Formel (I) gemäß einem der Ansprüche 1 bis 9, ein synergistisches Mittel für die Verbindung der Formel (I) und einen Exzipienten oder Verdünnungsmittel.

**13.** Gemisch einer Verbindung der Formel (I) gemäß einem der Ansprüche 1 bis 9 und einer weiteren pestiziden Verbindung.

**14.** Verwendung einer Verbindung der Formel (I) gemäß einem der Ansprüche 1 bis 9 beim Schutz und der Behandlung von Pflanzengattungen, insbesondere als Insektizid, Akarizid, Molluszid oder Nematozid.

**15.** Verbindung gemäß einem der Ansprüche 1 bis 9 oder Zusammensetzung gemäß einem der Ansprüche 11 bis 13 für die Verwendung bei einem chirurgischen oder therapeutischen Verfahren, praktiziert am menschlichen oder tierischen Körper, oder bei einem diagnostischen Verfahren, praktiziert am menschlichen oder tierischen Körper.